# EUROPEAN PATENT SPECIFICATION

(11) **EP 2 432 890 B1**
(45) Date of publication and mention of the grant of the patent: **02.09.2015**
(21) Application number: 10778451.4
(22) Date of filing: 21.05.2010
(51) Int. Cl.: C12P 7/64

(54) **ENGINEERED BIOSYNTHESIS OF FATTY ALCOHOLS**
INDUSTRIELLE BIOSYNTHESE VON FETTALKOHOLEN
BIOSYNTHÈSE MODIFIÉE D'ALCOOLS GRAS

(30) Priority: 22.05.2009 US 180534 P
(43) Date of publication of application: 28.03.2012
(73) Proprietor: Codexis, Inc., Redwood City, CA 94063 (US)
(72) Inventor: BEHROUZIAN, Behnaz, Redwood City, California 94063 (US); MCDANIEL, Robert, Redwood City, California 94063 (US); ZHANG, Xiyun, Redwood City, California 94063 (US); CLARK, Louis, Redwood City, California 94063 (US)
(74) Representative: Chapman, Desmond Mark
(86) International application number: PCT/US2010/035737
(87) International publication number: WO 2010/135624

(56) References cited:
- US-A1- 2006 199 254
- US-A1- 2007 042 383
- US-A1- 2010 105 963
- US-B2- 7 192 735
- VENKITASUBRAMANIAN ET AL: "Aldehyde oxidoreductase as a biocatalyst: Reductions of vanillic acid", ENZYME AND MICROBIAL TECHNOLOGY, STONEHAM, MA, US, vol. 42, no. 2, 12 December 2007 (2007-12-12), pages 130-137, XP022387256, ISSN: 0141-0229
- VENKITASUBRAMANIAN PADMESH ET AL: "Reduction of carboxylic acids by Nocardia aldehyde oxidoreductase requires a phosphopantetheinylated enzyme.", THE JOURNAL OF BIOLOGICAL CHEMISTRY 5 JAN 2007 LNKD- PUBMED:17102130, vol. 282, no. 1, 5 January 2007 (2007-01-05), pages 478-485, XP002684496, ISSN: 0021-9258
- ESBEN H HANSEN ET AL: "De Novo Biosynthesis of Vanillin in Fission Yeast (Schizosaccharomyces pombe) and Baker's Yeast (Saccharomyces cerevisiae)", APPLIED AND ENVIRONMENTAL MICROBIOLOGY, AMERICAN SOCIETY FOR MICROBIOLOGY, US, vol. 75, no. 9, 1 May 2009 (2009-05-01), pages 2765-2774, XP002666446, ISSN: 0099-2240, DOI: 10.1128/AEM.02681-08 [retrieved on 2009-03-01]

## Description

### 1. FIELD OF THE INVENTION

This invention relates to recombinant microorganisms which include polynucleotides encoding heterologous carboxylic acid reductases and the production of fatty alcohols having between C8 and C24 carbons in length as well as methods of their use.

### 2. BACKGROUND

The non-renewable nature and cost of fossil fuels have sparked interest in alternative energy sources including nuclear power, solar energy, wind power, as well as biological processes for production of fuels ("biofuels"). The latter biological approaches are particularly valuable in that they represent a renewable source of combustible materials which are not derived from petroleum sources. One option for producing biofuels includes the use of biomass to provide sugars for microbial (e.g., yeast) fermentations with the ultimate production of short chain alcohols such as ethanol and butanol. However, another alternative which includes the use of renewable carbon substrates includes the production of fatty acid derivatives such as fatty acid esters or fatty alcohols which may be used as a biofuel. The physical properties of fatty acids make them very suitable for fuel applications. Therefore fatty acid derived molecules such as fatty alcohols could be highly desirable products for biodiesel and/or jet fuel targets.

Venkitasubramanian et al. (Enzyme and Microbial Technology 2008 Jan;42(2):130-7) and Venkitasubramanian et al. (J Biol Chem. 2007 Jan 5;282(1):478-85) disclose aldehyde oxidoreductase as a biocatalyst for the reduction of vanillic acid and that the reduction of carboxylic acids by aldehyde oxidoreductase requires a phosphopantetheinylated enzyme. US 2006/0199254 discloses the nucleotide and amino acid sequence for carboxylic acid reductase, isolated from bacteria.

US 7,192,735 discloses phosphopantetheinyl transferases.

US 2010/0105963 discloses methods and compositions, including nucleotide sequences, amino acid sequences and host cells, for producing fatty alcohols.

### 3. SUMMARY

The invention provides a process for the biologically-derived production of fatty alcohols which comprise at least 8 carbon atoms comprising: (a) culturing a recombinant microorganism comprising a nucleic acid sequence encoding a heterologous carboxylic acid reductase and a gene encoding a phosphopantetheinyl transferase polypeptide (PPTase) capable of attaching a phosphopantetheine moiety to the carboxylic acid reductase, in an aqueous nutrient medium comprising an assimilable source of carbon under suitable culture conditions for a sufficient period of time to allow the production the fatty alcohols, wherein the recombinant microorganism is capable of producing at least 2 mg/L of fatty alcohols having C8 to C24 carbons in length, and wherein the PPTase is: (i) heterologous to the recombinant microorganism; or (ii) derived from the recombinant microorganism and over expressed by genetic manipulation of the PPTase, and (b) isolating the produced fatty alcohols.

The present disclosure has multiple aspects.

In one aspect, the disclosure relates to a recombinant microorganism comprising a nucleic acid sequence encoding a heterologous carboxylic acid reductase (CAR), wherein the recombinant microorganism is capable of producing fatty alcohols having C8 to C24 carbons in length. In one embodiment, the recombinant microorganism is capable of producing fatty alcohols having C10 to C20 carbons in length. In other embodiments, the carboxylic acid reductase is selected from the group consisting of a *Mycobacterium* carboxylic acid reductase, a *Nocardia* carboxylic acid reductase, and a *Streptomyces griseus* carboxylic acid reductase. In another embodiment, the carboxylic acid reductase has at least 90% sequence identity to SEQ ID NO: 2, at least 90% sequence identity to SEQ ID NO: 4, or at least 90% sequence identity to SEQ ID NO: 6. In some embodiments, the sequence identity is at least 95% to SEQ ID NO: 2, at least 95% to SEQ ID NO: 4, or at least 95% to SEQ ID NO: 6. In some embodiments, the sequence identity is at least 95% to SEQ ID NO: 2, at least 95% to SEQ ID NO: 4, or at least 95% to SEQ ID NO: 6. In other embodiments, the polynucleotide sequence encoding a CAR has at least 90% sequence identity to SEQ ID NO: 1, at least 90% sequence identity to SEQ ID NO: 3, or at least 90% sequence identity to SEQ ID NO: 5. In further embodiments, the recombinant microorganism is a bacterial strain, a filamentous fungal strain, a yeast strain or an algal strain. In other embodiments, the recombinant microorganism comprises a gene encoding a phosphopantetheinyl transferase polypeptide capable of attaching a pantetheine moiety to the carboxylic acid reductase.

In a second aspect, the disclosure relates to an isolated CAR variant, the variant comprising at least 90% sequence identity to SEQ ID NO: 4 and an amino acid substitution at one or more of the following positions R270, A271, K274, A275, P467, Q584, E626, and/or D701 in SEQ ID NO: 4. In some embodiments, the variant is R270W, A271W, K274(G/ N/V/I/W/L/M/Q/S), A275F, P467S, Q584R, E626G, and/or D701G. In some embodiments, the variant comprises at least 90% sequence identity to SEQ ID NO: 4 and a combination of substitutions selected from K274L/A369T/L380Y, K274L/V358H/E845A, K274M/T282K, K274Q/T282Y, K274S/A715T, K274W/L380G/ A477T, K274W/T282E/L380V, K274W/T282Q, K274W/V358R and/or R43C/K274I when aligned with SEQ ID NO: 4.

In a third aspect, the disclosure relates to a process for the biologically-derived production of fatty alcohols comprising a) culturing a recombinant microorganism encompassed by the disclosure in an aqueous nutrient medium comprising an assimilable source of carbon under suitable culture conditions for a sufficient period of time to allow the production the fatty alcohols and b) recovering the fatty alcohols produced by the recombinant microorganism. In one embodiment, the culturing step is carried out a temperature within the range of from about 10°C to about 80°C. In another embodiment, the culturing step is carried out for a period of from about 8 hours to about 240 hours. In a further embodiment, the amount of biologically produced fatty alcohol is in the range of 2 mg/L to 200g/L of fermentation broth. In a further embodiment, the amount of biologically produced C14 to C18 fatty alcohols is in the range of 2 mg/L to 200g/L. In yet other embodiments, the production of fatty alcohols having C10 to C20 carbons in length comprise at least 80% of the total isolated fatty alcohols. In another embodiment, the process further comprises reducing the fatty alcohols to corresponding alkanes.

In a fourth aspect, the disclosure relates to a method of catalytically reducing a fatty acid substrate to a corresponding C8 to C24 carbon containing fatty aldehyde comprising a) mixing an effective amount of a carboxylic acid reductase with a fatty acid substrate and co-substrates selected from ATP, NAD(H) and/or NADP(H) and b) incubating the mixture for a period of time and under conditions suitable to achieve reduction of the fatty acid substrate to the corresponding fatty aldehyde. In one embodiment, the fatty aldehyde is further reduced to a fatty alcohol.

In a fifth aspect, the disclosure relates to a process for the biologically-derived production of fatty alcohols in yeast which comprises culturing a recombinant yeast cell, which comprises a polynucleotide encoding a heterologous carboxylic acid reductase (CAR), said CAR comprising an amino acid sequence having at least 85% sequence identity (that is at least 85%, at least 88%, at least 90%, 91%, 92%, 93%, 94%, 95%, 96%, 97%, 98%, 99% and even 100% sequence identity) to SEQ ID NOs: 2, 4, 6, 9 or 10 under suitable culture conditions to allow expression of said CAR and production of the fatty alcohols and recovering the produced fatty alcohol. In some embodiments, the yeast is a *Yarrowia* strain or *Saccharomyces,* strain. In some embodiments, the amount of fatty alcohol produced is at least 2.0 mg/L. In further embodiments, the yeast are capable of producing fatty alcohols comprising C10 to C20 carbons in length. In other embodiments, the recombinant yeast further comprises a gene encoding a phosphopantetheinyl transferase. The phosphopantetheinyl transferase (PPTase) may be a heterologous PPTase, such as but not limited to a PPTase derived from a *Nocardia* strain or *Mycobacterium,* strain. In other embodiments, the recombinant yeast cells comprise a polynucleotide that encodes a heterologous alcohol dehydrogenase. In yet other embodiments of this aspect, the disclosure relates to a biologically-derived fatty alcohol composition comprising the fatty alcohols or derivative thereof produced according to the process.

In yet a further aspect, the disclosure relates to a process for the biologically-derived production of fatty alcohols comprising culturing a recombinant microorganism, which comprises a gene coding for a heterologous carboxylic acid reductase (CAR) comprising an amino acid sequence having at least 90% sequence identity to SEQ ID NOs: 2, 4 , 6, 9 or 10 and a polynucleotide coding for a heterologous phosphopantetheinyl transferase (PPTase) having at least 80% sequence identity to SEQ ID NOs: 8 or 11, wherein said PPTase is capable of attaching a phosphopantetheine moiety to the CAR and culturing under suitable culture conditions to allow to expression of the CAR and PPTase and production of the fatty alcohols, and recovering the fatty alcohols produced by the recombinant microorganism. In some embodiments, the recombinant microorganism is a bacterial, yeast, filamentous fungal or algal strain. In other embodiments, the CAR and PPTase are derived from the same organism.

### 4. BRIEF DESCRIPTION OF THE FIGURES

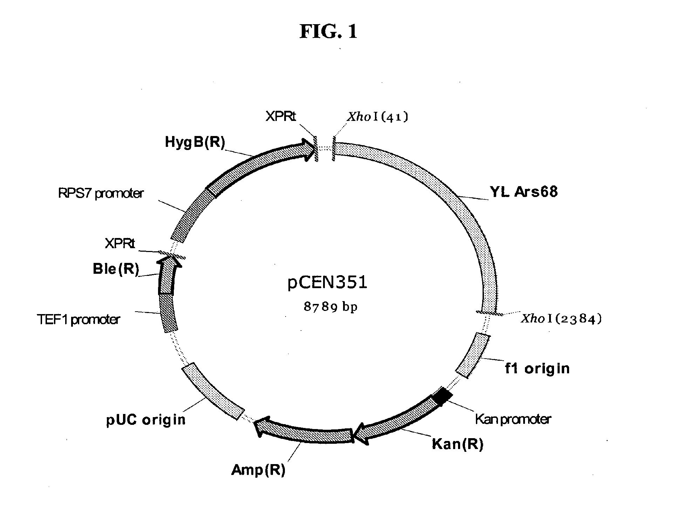

FIG. 1 depicts the replicative *Y. lipolytica* vector pCEN351 (8789bp) containing cassettes encoding phleomycin (Ble) and hygromycin (HygB) resistance. Ars68 is an autonomous replicating sequence isolated from *Y. lipolytica* chromosomal DNA.

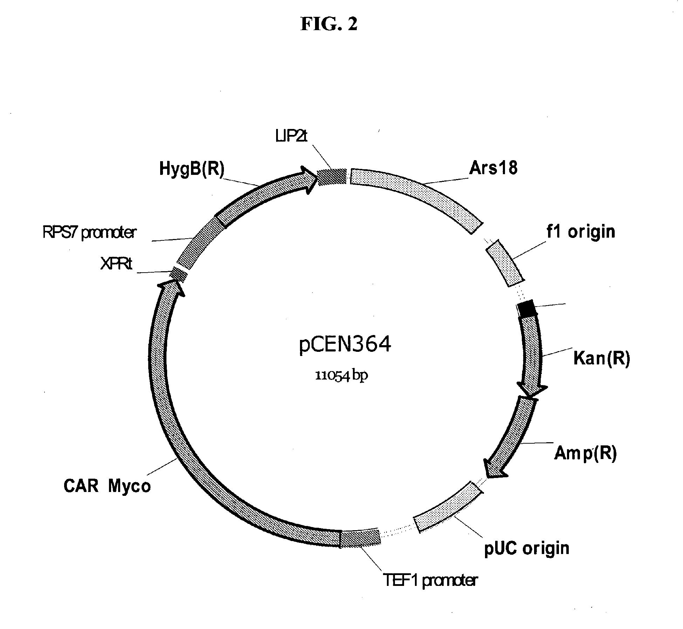

FIG. 2 depicts the expression vector pCEN364 comprising the *Mycobacterium sp JLS* gene encoding carboxylic acid reductase (CAR).

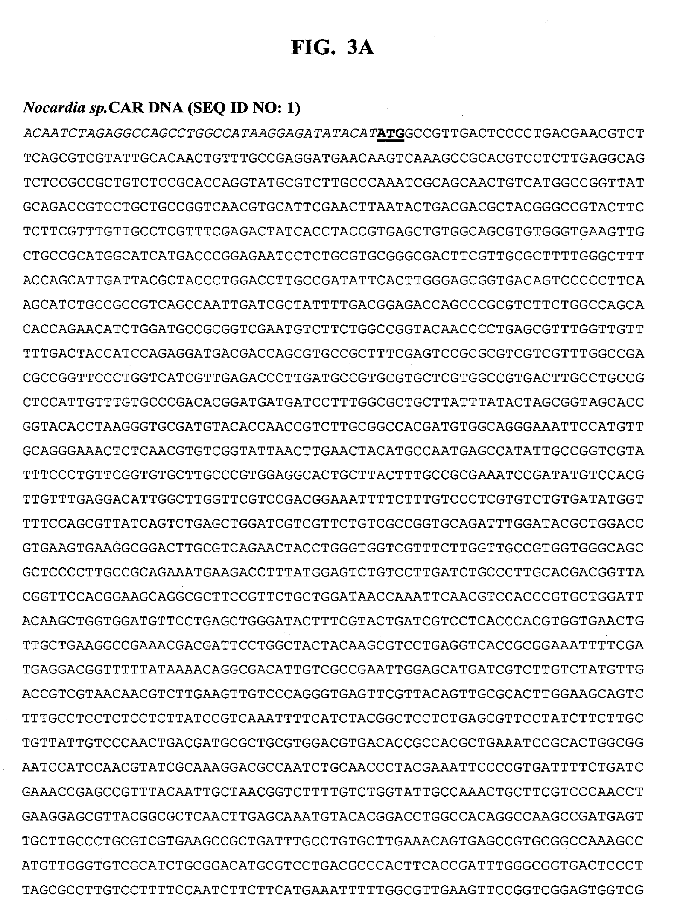

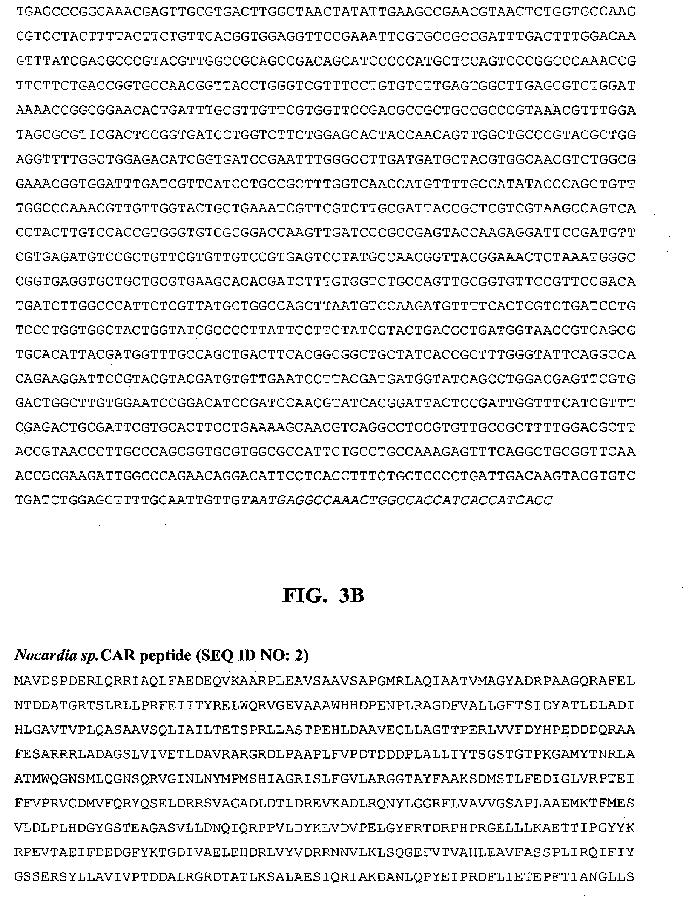

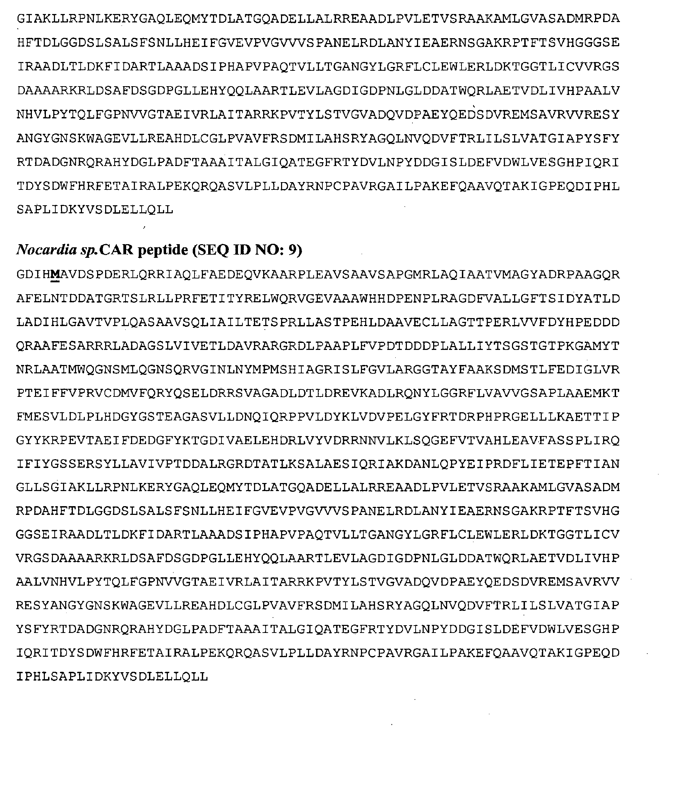

FIGS. 3A and 3B illustrate the codon optimized polynucleotide sequence (SEQ ID NO: 1) encoding a CAR (SEQ ID NO: 2) of *Nocardia* NRRL5646. The 5' and 3' polynucleotide flanking regions are in italics and the first ATG coding for methionine in the expressed protein is underlined and in bold. Stop codons are identified by "*". The flanking regions upstream and downstream of the stop codon are not expressed. Conceptual translation of the longest open reading frame (ORF) in SEQ ID NO: 1 resulted in SEQ ID NO: 9. The initiator methionine (underlined and in bold) of the CAR protein is residue 5 of SEQ ID NO: 9.

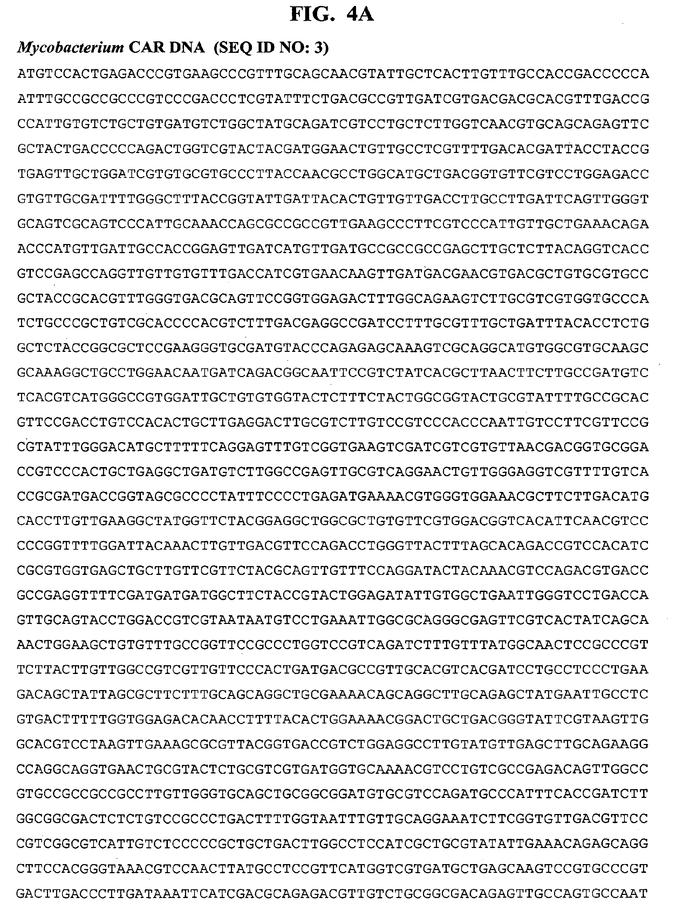

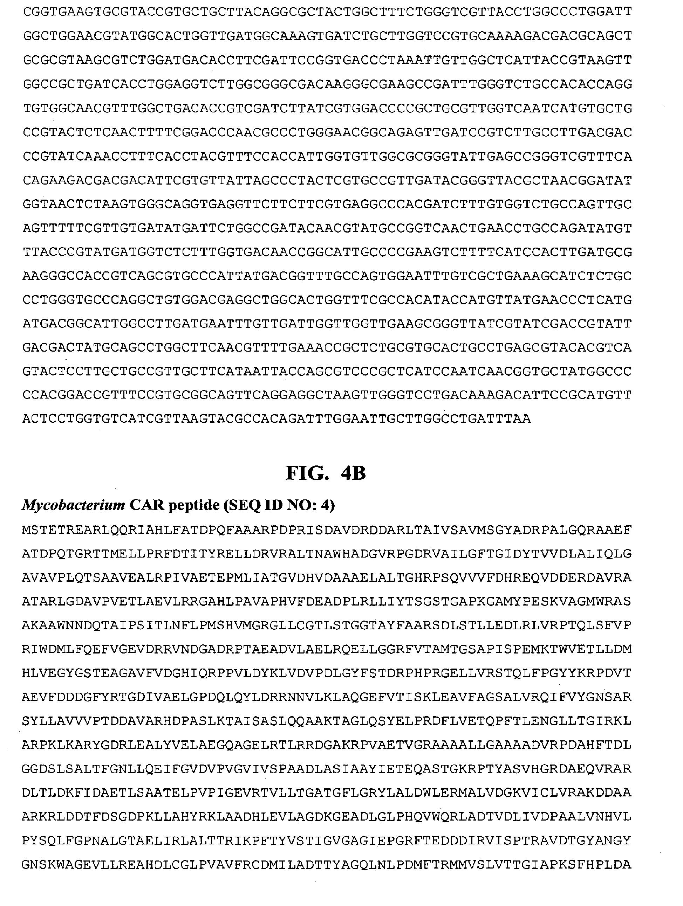

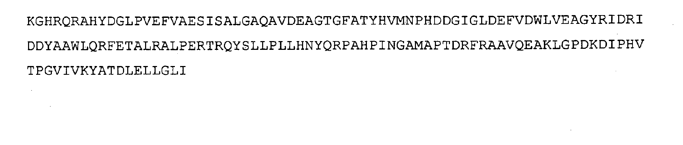

FIGS. 4A and 4B illustrate the codon optimized polynucleotide sequence (SEQ ID NO: 3) encoding a CAR (SEQ ID NO: 4) of *Mycobacterium* sp.(strain JLS).

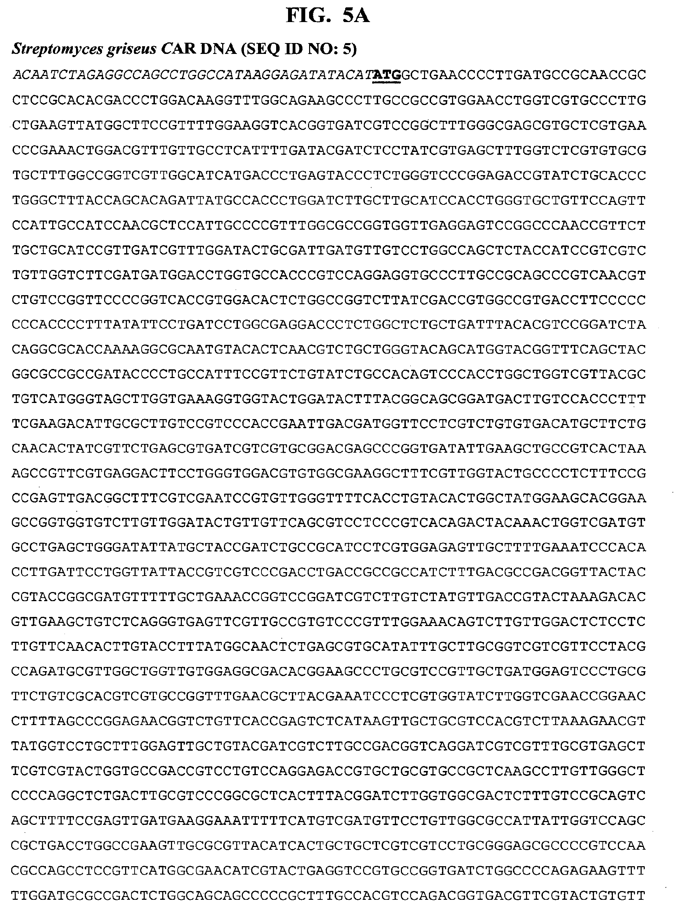

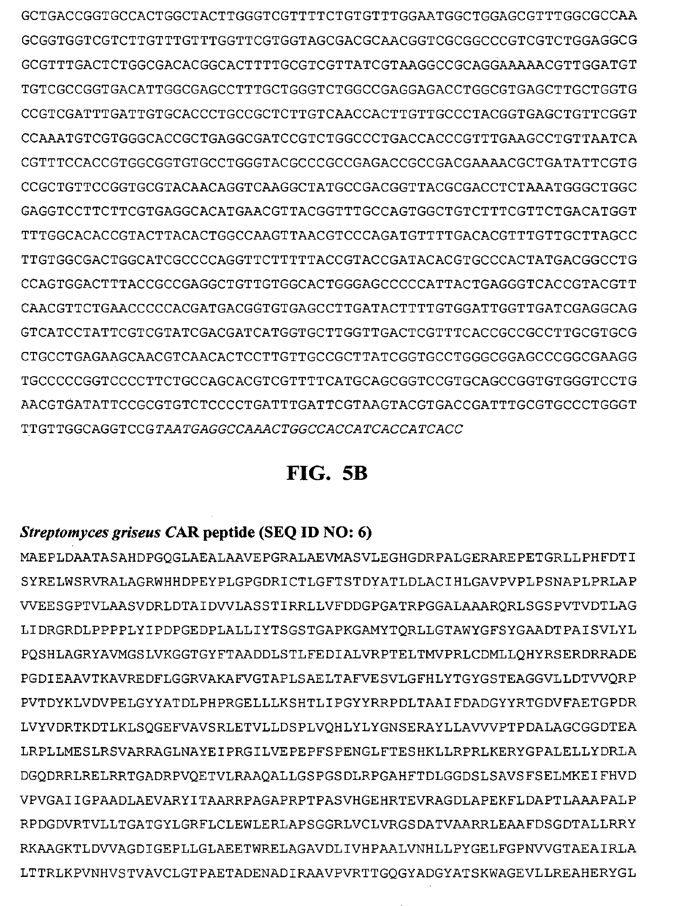

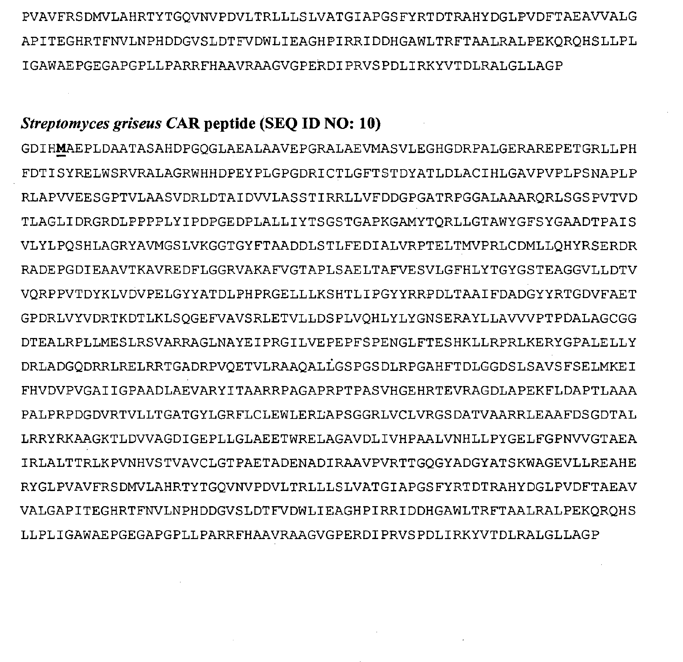

FIGS. 5A and 5B illustrate the codon optimized polynucleotide sequence (SEQ ID NO: 5) encoding a CAR (SEQ ID NO: 6) of *Streptomyces griseus.* The 5' and 3' polynucleotide flanking regions are in italics and the first ATG coding for methionine in the expressed protein is underlined and in bold. Stop codons are identified by "*". The flanking regions upstream and downstream of the stop codon are not expressed. Conceptual translation of the longest open reading frame (ORF) in SEQ ID NO: 5 resulted in SEQ ID NO: 10. The initiator methionine (underlined and in bold) of the CAR protein is residue 5 of SEQ ID NO: 10.

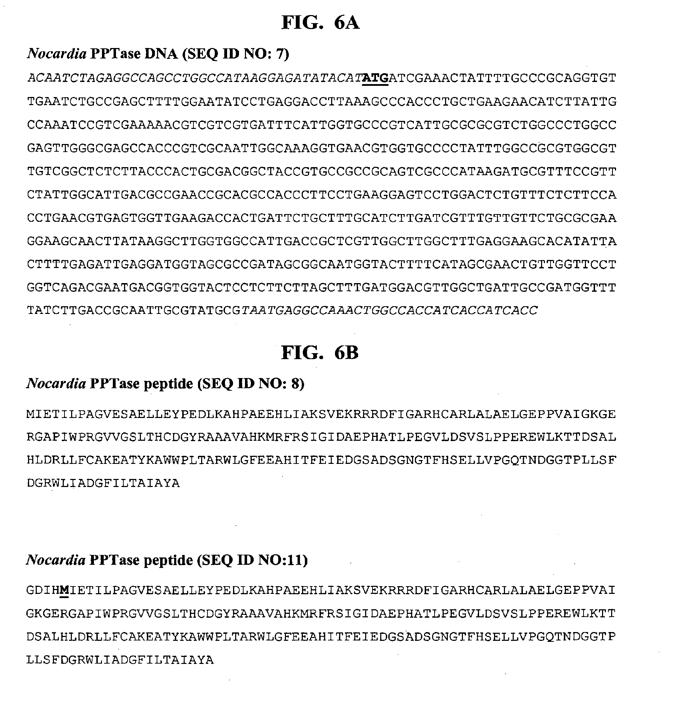

FIGS. 6a and 6B illustrate the codon optimized polynucleotide sequence (SEQ ID NO: 7) encoding a *Nocardia* NRRL 5646 phosphopantetheinyl transferase (PPTase) (SEQ ID NO: 8). The 5' and 3' polynucleotide flanking regions are in italics and the first ATG coding for methionine in the expressed protein is underlined and in bold. Stop codons are identified by "*". The flanking regions upstream and downstream of the stop codon are not expressed. Conceptual translation of the longest open reading frame (ORF) in SEQ ID NO: 10 resulted in SEQ ID NO: 11. The initiator methionine of the PPTase is residue 5 of SEQ ID NO: 11.

### 5. SUMMARY OF DISCLOSURE

### 6.1 Definitions

Unless defined otherwise, all technical and scientific terms used herein generally have the same meaning as commonly understood by one of ordinary skill in the art to which this disclosure pertains. Generally, the nomenclature used herein and the laboratory procedures of cell culture, molecular genetics, organic chemistry, analytical chemistry and nucleic acid chemistry described below are those well known and commonly employed in the art. As used herein, the following terms are intended to have the following meanings:

The following abbreviations are used herein:

"CoA" for coenzyme A; "TE" for thioesterase; "CAR" for carboxylic acid reductase; "ADH" for alcohol dehydrogenase; "ACP" for acyl carrier protein; "EC" means Enzyme Classification Number; CX:0 fatty acid, wherein X = 8 - 24 means a saturated fatty acid having X carbons (e.g., for illustrative purposes, C16:0 means hexadecanoic acid and C18:0 means octadecanoic acid); CX:1 means a monounsaturated fatty acid, wherein X = 8 - 24; CX:0-OH, means a saturated fatty alcohol, wherein X = 8 - 24 (e.g., for illustrative purposes, C14:0-OH means 1-tetradecanol and C16:0-OH means 1-hexadecanol; and C18:1-0H means 1-octadecenol); and "PPTase" is phosphopantetheinyl transferase.

"Fatty acids" are aliphatic mono carboxylic acids which may be saturated or unsaturated. As used herein a fatty acid comprises at least 8 carbon atoms. For example a saturated fatty acid has the formula CH₃(CH₂)ₓCOOH, wherein x is ≥ 6. Unsaturated fatty acids are of the same formula and contain one or more double bonds in the aliphatic chain.

"Fatty alcohol" as used herein refers to a long chain saturated or unsaturated hydrocarbon chain wherein the OH group attaches to the terminal carbon. As used herein a fatty alcohol comprises at least 8 carbon atoms. For example, a saturated fatty alcohol has the formula CH₃(CH₂)ₓCH₂OH, wherein x is ≥ 6. Unsaturated fatty alcohols are of the same formula and contain one or more double bonds in the hydrocarbon chain.

"Fatty aldehyde" as used herein refers to a saturated or unsaturated aliphatic aldehyde comprising at least 8 carbon atoms. For example, a saturated fatty aldehyde has the formula CH₃(CH₂)ₓCHO, wherein x is ≥ 6. Unsaturated fatty aldehydes are of the same formula and contain one or more double bonds in the aliphatic chain.

"Acyl-ACP thioesterase" (EC 3.1.2.14) used herein refers to a polypeptide having an enzymatic capability of carrying out the reaction depicted for TE in Scheme 1. Acyl-ACP thioesterases as used herein include naturally occurring (wild type) acyl-ACP thioesterases as well as non-naturally occurring engineered polypeptides generated by human manipulation.

"Alcohol dehydrogenase (ADH)" (EC 1.1.1.1) is used herein to refer to a polypeptide having an enzymatic capability of carrying out the reaction depicted for ADH in Scheme 1. Alcohol dehydrogenases as used herein include naturally occurring (wild type) alcohol dehydrogenases as well as non-naturally occurring engineered polypeptides generated by human manipulation.

"Carboxylic acid reductase (CAR)" (EC 1.2.1.30 or EC 1.2.1.3) sometimes referred to in the literature as aryl-aldehyde oxidoreductase as used herein refers to a polypeptide having an enzymatic capability of carrying out the reaction depicted for CAR in Scheme 1. Carboxylic acid reductases as used herein include naturally occurring (wild type) carboxylic acid reductases as well as non-naturally occurring engineered polypeptides generated by human manipulation. Preferred CARs of the present disclosure are those that require NADP/NADPH as a co-substrate.

The term "variant CAR" refers to a CAR of the present disclosure that is derived by manipulation from a reference CAR. Variant CARs may be constructed by modifying a DNA sequence that encodes for a wild-type CAR (*e*.*g*. a wild-type CAR depicted by SEQ ID NO: 2, SEQ ID NO: 4 or SEQ ID NO: 6).

The term "pantetheine", IUPAC 2,4-dihydroxy-3,3-dimethyl-N-[3-oxo-3-(2-sulfanylyethylamino,propyl]butanamide and having the molecular formula of C₁₁H₂₂N₂O₄S refers to an intermediate in the pathway of coenzyme A.

A "phosphopantetheinyl transferase" (PPTase) refers to an enzyme that activates an acyl carrier protein (ACP). The phospho-pantetheine coenzyme is linked to the ACP by a phospho ester linkage. The PPTase converts the inactive apoprotein to an active holoprotein.

The terms "culturing" and "cultivation" refer to growing a population of microbial cells under suitable conditions in a liquid or solid medium. In some embodiments, culturing refers to fermentative bioconversion of a substrate to an end-product.

"Coding sequence" or "coding region" refers to that portion of a nucleic acid (*e*.*g*., a gene) that encodes an amino acid sequence of a protein.

"Naturally-occurring" or "wild-type" refers to the form found in nature. For example, a naturally occurring or wild-type polypeptide or polynucleotide sequence is a sequence present in an organism that can be isolated from a source in nature and which has not been intentionally modified by human manipulation.

"Recombinant" when used with reference to, *e.g.,* a cell, nucleic acid, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant cells expressing genes that are not found within the native (non-recombinant) form of the cell (i.e. "heterologous" genes) or express native genes that are otherwise expressed at a different level.

"Recombinant host cell" or "recombinant microorganism" refers to a cell or microorganism into which has been introduced a heterologous polynucleotide or vector.

"Host cell" refers to a suitable host for an expression vector comprising DNA encoding a CAR encompassed by the disclosure and the progeny thereof. Host cells useful in the present disclosure are generally prokaryotic or eukaryotic hosts, including any transformable microorganism in which expression can be achieved.

The term "transformed" or "transformation" used in reference to a cell means a cell has a non-native nucleic acid sequence integrated into its genome or as an episomal plasmid that is maintained through multiple generations.

"Fermentable sugar" means simple sugars (monosaccharides, disaccharides and short oligosaccharides) such as but not limited to glucose, xylose, galactose, arabinose, mannose and sucrose. The term "fermentable sugar" is sometimes used interchangeably with the term "assimilable carbon source".

"Percentage of sequence identity" is used herein to refer to comparisons among polynucleotides and polypeptides, and are determined by comparing two optimally aligned sequences over a comparison window, wherein the portion of the polynucleotide or polypeptide sequence in the comparison window may comprise additions or deletions (*i*.*e*., gaps) as compared to the reference sequence (which does not comprise additions or deletions) for optimal alignment of the two sequences. The percentage may be calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Alternatively, the percentage may be calculated by determining the number of positions at which either the identical nucleic acid base or amino acid residue occurs in both sequences or a nucleic acid base or amino acid residue is aligned with a gap to yield the number of matched positions, dividing the number of matched positions by the total number of positions in the window of comparison and multiplying the result by 100 to yield the percentage of sequence identity. Those of skill in the art appreciate that there are many established algorithms available to align two sequences. Optimal alignment of sequences for comparison can be conducted, e.g., by the local homology algorithm of Smith and Waterman, 1981, Adv. Appl. Math. 2:482, by the homology alignment algorithm ofNeedleman and Wunsch, 1970, J. Mol. Biol. 48:443, by the search for similarity method of Pearson and Lipman, 1988, Proc. Natl. Acad. Sci. USA 85:2444, by computerized implementations of these algorithms (GAP, BESTFIT, FASTA, and TFASTA in the GCG Wisconsin Software Package), or by visual inspection (see generally, Current Protocols in Molecular Biology, F. M. Ausubel et al., eds., Current Protocols, a joint venture between Greene Publishing Associates, Inc. and John Wiley & Sons, Inc., (1995 Supplement) (Ausubel)). Examples of algorithms that are suitable for determining percent sequence identity and sequence similarity are the BLAST and BLAST 2.0 algorithms, which are described in Altschul et al., 1990, J. Mol. Biol. 215: 403-410 and Altschul et al., 1977, Nucleic Acids Res. 3389-3402, respectively. Software for performing BLAST analyses is publicly available through the National Center for Biotechnology Information website. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix (see Henikoff and Henikoff, 1989, Proc Natl Acad Sci USA 89:10915). Exemplary determination of sequence alignment and % sequence identity can employ the BESTFIT or GAP programs in the GCG Wisconsin Software package (Accelrys, Madison WI), using default parameters provided.

"Corresponding to", "reference to", or "relative to", when used in the context of the numbering of a given amino acid or polynucleotide sequence refers to the numbering of the residues of a specified reference sequence when the given amino acid or polynucleotide sequence is compared to the reference sequence.

"Conversion" refers to the enzymatic conversion of the substrate to the corresponding product. "Percent conversion" refers to the percent of the substrate that is reduced to the product within a period of time under specified conditions. Thus, the "enzymatic activity" or "activity" of a polypeptide can be expressed as "percent conversion" of the substrate to the product.

"Hydrophilic Amino Acid or Residue" refers to an amino acid or residue having a side chain exhibiting a hydrophobicity of less than zero according to the normalized consensus hydrophobicity scale of Eisenberg et al., 1984, J. Mol. Biol. 179:125-142. Genetically encoded hydrophilic amino acids include L-Thr (T), L Ser (S), L His (H), L Glu (E), L Asn (N), L Gln (Q), L Asp (D), L Lys (K) and L Arg (R).

"Acidic Amino Acid or Residue" refers to a hydrophilic amino acid or residue having a side chain exhibiting a pK value of less than about 6 when the amino acid is included in a peptide or polypeptide. Acidic amino acids typically have negatively charged side chains at physiological pH due to loss of a hydrogen ion. Genetically encoded acidic amino acids include L Glu (E) and L Asp (D).

"Basic Amino Acid or Residue" refers to a hydrophilic amino acid or residue having a side chain exhibiting a pK value of greater than about 6 when the amino acid is included in a peptide or polypeptide. Basic amino acids typically have positively charged side chains at physiological pH due to association with hydronium ion. Genetically encoded basic amino acids include L Arg (R) and L Lys (K).

"Polar Amino Acid or Residue" refers to a hydrophilic amino acid or residue having a side chain that is uncharged at physiological pH, but which has at least one bond in which the pair of electrons shared in common by two atoms is held more closely by one of the atoms. Genetically encoded polar amino acids include L Asn (N), L Gln (Q), L Ser (S) and L Thr (T).

"Hydrophobic Amino Acid or Residue" refers to an amino acid or residue having a side chain exhibiting a hydrophobicity of greater than zero according to the normalized consensus hydrophobicity scale of Eisenberg et al., 1984, J. Mol. Biol. 179:125-142. Genetically encoded hydrophobic amino acids include L Pro (P), L Ile (I), L Phe (F), L Val (V), L Leu (L), L Trp (W), L Met (M), L Ala (A) and L Tyr (Y).

"Aromatic Amino Acid or Residue" refers to a hydrophilic or hydrophobic amino acid or residue having a side chain that includes at least one aromatic or heteroaromatic ring. Genetically encoded aromatic amino acids include L Phe (F), L Tyr (Y) and L Trp (W). Although owing to the pKa of its heteroaromatic nitrogen atom L His (H) it is sometimes classified as a basic residue, or as an aromatic residue as its side chain includes a heteroaromatic ring, herein histidine is classified as a hydrophilic residue.

"Non-polar Amino Acid or Residue" refers to a hydrophobic amino acid or residue having a side chain that is uncharged at physiological pH and which has bonds in which the pair of electrons shared in common by two atoms is generally held equally by each of the two atoms (*i*.*e*., the side chain is not polar). Genetically encoded non-polar amino acids include L Gly (G), L Leu (L), L Val (V), L Ile (I), L Met (M) and L Ala (A).

"Aliphatic Amino Acid or Residue" refers to a hydrophobic amino acid or residue having an aliphatic hydrocarbon side chain. Genetically encoded aliphatic amino acids include L Ala (A), L Val (V), L Leu (L) and L Ile (I).

"Small Amino Acid or Residue" refers to an amino acid or residue having a side chain that is composed of a total three or fewer carbon and/or heteroatom (excluding the α carbon and hydrogens). The small amino acids or residues may be further categorized as aliphatic, non-polar, polar or acidic small amino acids or residues, in accordance with the above definitions. Genetically-encoded small amino acids include L Ala (A), L Val (V), L Cys (C), L Asn (N), L Ser (S), L Thr (T) and L Asp (D).

"Hydroxyl-containing Amino Acid or Residue" refers to an amino acid containing a hydroxyl (-OH) moiety. Genetically-encoded hydroxyl-containing amino acids include L Ser (S) L Thr (T) and L-Tyr (Y).

"Conservative" amino acid substitutions or mutations refer to the interchangeability of residues having similar side chains, and thus typically involves substitution of the amino acid in the polypeptide with amino acids within the same or similar defined class of amino acids. However, as used herein, conservative mutations do not include substitutions from a hydrophilic to hydrophilic, hydrophobic to hydrophobic, hydroxyl-containing to hydroxyl-containing, or small to small residue, if the conservative mutation can instead be a substitution from an aliphatic to an aliphatic, non-polar to non-polar, polar to polar, acidic to acidic, basic to basic, aromatic to aromatic, or constrained to constrained residue. Further, as used herein, A, V, L, or I can be conservatively mutated to either another aliphatic residue or to another non-polar residue. Table 1 below shows exemplary conservative substitutions.

**Table 1: Conservative Substitutions**

| **Residue** | **P Possible Conservative Mutations** |
|---|---|
| A, L, V, I | Other aliphatic (A, L, V, I) |
| | Other non-polar (A, L, V, I, G, M) |
| G, M | Other non-polar (A, L, V, I, G, M) |
| D, E | Other acidic (D, E) |
| K, R | Other basic (K, R) |
| P, H | Other constrained (P, H) |
| N, Q, S, T | Other polar (N, Q, S, T) |
| Y, W, F | Other aromatic (Y, W, F) |
| C | None |

"Non-conservative substitution" refers to substitution or mutation of an amino acid in the polypeptide with an amino acid with significantly differing side chain properties. Non-conservative substitutions may use amino acids between, rather than within, the defined groups listed above. In one embodiment, a non-conservative mutation affects (a) the structure of the peptide backbone in the area of the substitution (e.g., proline for glycine) (b) the charge or hydrophobicity, or (c) the bulk of the side chain.

"Deletion" refers to modification to the polypeptide by removal of one or more amino acids from the reference polypeptide. Deletions can comprise removal of 1 or more amino acids, 2 or more amino acids, 5 or more amino acids, 10 or more amino acids, 15 or more amino acids, or 20 or more amino acids, up to 10% of the total number of amino acids, or up to 20% of the total number of amino acids making up the reference enzyme while retaining enzymatic activity and/or retaining the improved properties of an engineered enzyme. Deletions can be directed to the internal portions and/or terminal portions of the polypeptide. In various embodiments, the deletion can comprise a continuous segment or can be discontinuous. The term "deletion" is also used to refer to a DNA modification in which one or more nucleotides or nucleotide base-pairs have been removed, as compared to the corresponding reference, parental or "wild type" DNA.

"Insertion" refers to modification to the polypeptide by addition of one or more amino acids from the reference polypeptide. In some embodiments, the improved engineered comprise insertions of one or more amino acids to the naturally occurring polypeptide as well as insertions of one or more amino acids to other improved polypeptides. Insertions can be in the internal portions of the polypeptide, or to the carboxy or amino terminus. Insertions as used herein include fusion proteins as is known in the art. The insertion can be a contiguous segment of amino acids or separated by one or more of the amino acids in the naturally occurring polypeptide. The term "insertion" is also used to refer to a DNA modification in which or more nucleotides or nucleotide base-pairs have been inserted, as compared to the corresponding reference, parental or "wild type" DNA.

"Different from" or "differs from" with respect to a designated reference sequence refers to difference of a given amino acid or polynucleotide sequence when aligned to the reference sequence. Generally, the differences can be determined when the two sequences are optimally aligned. Differences include insertions, deletions, or substitutions of amino acid residues in comparison to the reference sequence.

"Isolated polypeptide or polynucleotide" refers to a polypeptide or polynucleotide which is substantially separated from other contaminants that naturally accompany it, e.g., protein, lipids, and polynucleotides. The term embraces polypeptides and polynucleotides which have been removed or purified from their naturally-occurring environment or expression system (*e*.*g*., host cell or in vitro synthesis). Improved enzymes may be present within a cell, present in the cellular medium, or prepared in various forms, such as lysates or isolated preparations. As such, in some embodiments, the improved enzyme can be an isolated polypeptide.

"Heterologous" polynucleotide, gene, promoter, or polypeptide refers to any polynucleotide, gene, promoter, or polypeptide that is introduced into a host cell by laboratory techniques, and includes a polynucleotide, gene, promoter, or polypeptide that is removed from a host cell, subjected to laboratory manipulation, and then reintroduced into a host cell.

"Endogenous" polynucleotide, gene, promoter or polypeptide refers to any polynucleotide, gene, promoter or polypeptide that is in the cell and was not introduced into the cell using laboratory or recombinant techniques.

"Improved enzyme property" refers to a polypeptide that exhibits an improvement in any enzyme property as compared to a reference polypeptide. For the engineered polypeptides described herein, the comparison is generally made to the wild-type enzyme. Enzyme properties for which improvement is desirable include, but are not limited to, enzymatic activity, thermal stability, pH activity profile, refractoriness to inhibitors, e.g., feedback inhibition, product inhibition, and substrate inhibition, as well as increased stability and/or activity in the presence of additional components present in, added to, or formed within the aqueous nutrient medium or within the recombinant host cell.

"Codon optimized" refers to changes in the codons of the polynucleotide encoding a protein to those preferentially used in a particular organism such that the encoded protein is efficiently expressed in the organism of interest. Although the genetic code is degenerate in that most amino acids are represented by several codons, called "synonyms" or "synonymous" codons, it is well known that codon usage by particular organisms is nonrandom and biased towards particular codon triplets. This codon usage bias may be higher in reference to a given gene, genes of common function or ancestral origin, highly expressed proteins versus low copy number proteins, and the aggregate protein coding regions of an organism's genome. In some embodiments, the polynucleotides encoding enzymes may be codon optimized for optimal production from the host organism selected for expression.

"Preferred, optimal, high codon usage bias codons" refers interchangeably to codons that are used at higher frequency in the protein coding regions than other codons that code for the same amino acid. The preferred codons may be determined in relation to codon usage in a single gene, a set of genes of common function or origin, highly expressed genes, the codon frequency in the aggregate protein coding regions of the whole organism, codon frequency in the aggregate protein coding regions of related organisms, or combinations thereof. Codons whose frequency increases with the level of gene expression are typically optimal codons for expression. A variety of methods are known for determining the codon frequency (*e.g*., codon usage, relative synonymous codon usage) and codon preference in specific organisms, including multivariate analysis, for example, using cluster analysis or correspondence analysis, and the effective number of codons used in a gene (see GCG Codon Preference, Genetics Computer Group Wisconsin Package; CodonW, John Peden, University ofNottingham; McInerney, J. O, 1998, Bioinformatics 14:372-73; Stenico et al., 1994, Nucleic Acids Res. 222437-46; Wright, F., 1990, Gene 87:23-29). Codon usage tables are available for a growing list of organisms (see for example, Wada et al., 1992, Nucleic Acids Res. 20:2111-2118; Nakamura et al., 2000, Nucl. Acids Res. 28:292; Duret, *et al*., supra; Henaut and Danchin, "Escherichia coli and Salmonella," 1996, Neidhardt, et al. Eds., ASM Press, Washington D.C., p. 2047-2066. The data source for obtaining codon usage may rely on any available nucleotide sequence capable of coding for a protein. These data sets include nucleic acid sequences actually known to encode expressed proteins (*e.g*., complete protein coding sequences-CDS), expressed sequence tags (ESTs), or predicted coding regions of genomic sequences (see for example, Mount, D., Bioinformatics: Sequence and Genome Analysis, Chapter 8, Cold Spring Harbor Laboratory Press, Cold Spring Harbor, N.Y., 2001; Uberbacher, E. C., 1996, Methods Enzymol. 266:259-281; Tiwari et al., 1997, Comput. Appl. Biosci. 13:263-270).

"Hybridization stringency" relates to such washing conditions of nucleic acids. Generally, hybridization reactions are performed under conditions of lower stringency, followed by washes of varying but higher stringency. The term "moderately stringent hybridization" refers to conditions that permit target-DNA to bind a complementary nucleic acid that has about 60% identity, preferably about 75% identity, about 85% identity to the target DNA; with greater than about 90% identity to target-polynucleotide. Exemplary moderately stringent conditions are conditions equivalent to hybridization in 50% formamide, 5× Denhart's solution, 5×SSPE, 0.2% SDS at 42°C., followed by washing in 0.2×SSPE, 0.2% SDS, at 42°C. "High stringency hybridization" refers generally to conditions that are about 10°C or less from the thermal melting temperature Tm as determined under the solution condition for a defined polynucleotide sequence. In some embodiments, a high stringency condition refers to conditions that permit hybridization of only those nucleic acid sequences that form stable hybrids in 0.018M NaCl at 65°C. (*i*.*e*., if a hybrid is not stable in 0.018M NaCl at 65°C, it will not be stable under high stringency conditions, as contemplated herein). High stringency conditions can be provided, for example, by hybridization in conditions equivalent to 50% formamide, 5× Denhart's solution, 5×SSPE, 0.2% SDS at 42°C, followed by washing in 0.1×SSPE, and 0.1% SDS at 65°C. Other high stringency hybridization conditions, as well as moderately stringent conditions, are described in the references cited above.

"Control sequence" is defined herein to include all components, which are necessary or advantageous for the expression of a polypeptide of the present disclosure. Each control sequence may be native or foreign to the nucleic acid sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide.

"Operably linked" and "operably associated" are defined herein as a configuration in which a control sequence is appropriately placed at a position relative to the coding sequence of the DNA sequence such that the control sequence directs the expression of a polynucleotide and/or polypeptide.

"Promoter sequence" is a nucleic acid sequence that is recognized by a host cell for expression of the coding region. The control sequence may comprise an appropriate promoter sequence. The promoter sequence contains transcriptional control sequences, which mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

As used herein "a", "an", and "the" include plural references unless the context clearly dictates otherwise.

The term "comprising" and its cognates are used in their inclusive sense; that is, equivalent to the term "including" and its corresponding cognates.

### 6.2 Host Cells Useful In The Disclosed Process

In some embodiments, the host cell is a eukaryotic cell. Suitable eukaryotic host cells include, but are not limited to, fungal cells and algal cells. Some preferred fungal host cells are yeast cells and filamentous fungal cells.

The filamentous fungal host cell may be a cell of a species of, but not limited to Achlya, Acremonium, Aspergillus, Aureobasidium, Bjerkandera, Ceriporiopsis, Cephalosporium, Chrysosporium, Cochliobolus, Corynascus, Cryphonectria, Cryptococcus, Coprinus, Coriolus, Diplodia, Endothis, Fusarium, Gibberella, Gliocladium, Humicola, Hypocrea, Myceliophthora, Mucor, Neurospora, Penicillium, Podospora, Phlebia, Piromyces, Pyricularia, Rhizomucor, Rhizopus, Schizophyllum, Scytalidium, Sporotrichum, Talaromyces, Thermoascus, Thielavia, Trametes, Tolypocladium, Trichoderma, Verticillium, Volvariella, or teleomorphs, synonyms or taxonomic equivalents thereof.

In some embodiments of the invention, the filamentous fungal host cell is an *Aspergillus* species, a *Chrysosporium* species, a *Corynascus* species, a *Fusarium* species, a *Humicola* species, a *Myceliophthora* species, a *Neurospora* species, a *Penicillum* species, a *Tolypocladium* species, a *Tramates* species, or *Trichoderma* species. In some embodiments of the invention, the *Trichoderma* species is *T. longibrachiatum*, T. *viride, Hypocrea jecorina* or *T. reesei*; the *Aspergillus* species is *A. awamori*, *A. funigatus*, *A. japonicus, A. nidulans, A. niger*, *A. aculeatus*, *A. foetidus*, *A. oryzae*, *A. sojae,* and *A. kawachi*; the *Chrysosporium* species is *C*. *lucknowense*; the *Fusarium* species is *F.graminum*, *F. oxysporum* and *F*. *venenatum;* the *Neurospora* species is *N. crassa*; the *Humicola* species is *H. insolens, H. grisea*, and *H. lanuginosa*; the *Myceliophthora* species is *M. thermophilic*; the *Penicillum* species is *P. purpurogenum*, *P. chrysogenum,* and *P. verruculosum*; the *Thielavia* species is *T. terrestris*; and the *Trametes* species is *T. villosa* and *T. versicolor.*

In the present invention, a yeast host cell may be a cell of a species of, but not limited to Candida, Hansenula, Saccharomyces, Schizosaccharomyces, Pichia, Kluyveromyces, and Yarrowia. In some embodiments, the yeast host cell may be a cell of a species of Saccharomyces, Pichia, Candida or Yarrowia. In some embodiments of the invention, the yeast cell is *Hansenula polymorpha, Saccharomyces cerevisiae, Saccaromyces carlsbergensis, Saccharomyces*, *diastaticus*, *Saccharomyces norbensis*, *Saccharomyces kluyveri*, *Schizosaccharomyces pombe, Pichia pastoris*, *Pichia finlandica, Pichia trehalophila*, *Pichia kodamae*, *Pichia membranaefaciens*, *Pichia opuntiae, Pichia thermotolerans, Pichia salictaria, Pichia quercuum, Pichia pijperi*, *Pichia stipitis*, *Pichia methanolica, Pichia angusta, Kluyveromyces lactis, Candida albicans, Candida lipolytica, Candida krusei*, and *Yarrowia lipolytica.* Particularly useful *Yarrowia lipolytica* strains include but are not limited to DSMZ 1345, DSMZ 3286, DSMZ 8218, DSMZ 70561, DSMZ 70562, and DSMZ 21175.

In some embodiments of the invention, the host cell is an algal cell such as, Chlamydomonas (e.g., *C. Reinhardtii*) and Phormidium (P. sp. ATCC29409).

In other embodiments, the host cell is a prokaryotic cell. Suitable prokaryotic cells include gram positive, gram negative and gram-variable bacterial cells. The host cell may be a species of, but not limited to Agrobacterium, Alicyclobacillus, Anabaena, Anacystis, Acinetobacter, Acidothermus, Arthrobacter, Azobacter, Bacillus, Bifidobacterium, Brevibacterium, Butyrivibrio, Buchnera, Campestris, Camplyobacter, Clostridium, Corynebacterium, Chromatium, Coprococcus, Escherichia, Enterococcus, Enterobacter, Erwinia, Fusobacterium, Faecalibacterium, Francisella, Flavobacterium, Geobacillus, Haemophilus, Helicobacter, Klebsiella, Lactobacillus, Lactococcus, Ilyobacter, Micrococcus, Microbacterium, Mesorhizobium, Methylobacterium, Methylobacterium, Mycobacterium, Neisseria, Pantoea, Pseudomonas, Prochlorococcus, Rhodobacter, Rhodopseudomonas, Rhodopseudomonas, Roseburia, Rhodospirillum, Rhodococcus, Scenedesmun, Streptomyces, Streptococcus, Synnecoccus, Saccharomonospora, Staphylococcus, Serratia, Salmonella, Shigella, Thermoanaerobacterium, Tropheryma, Tularensis, Temecula, Thermosynechococcus, Thermococcus, Ureaplasma, Xanthomonas, Xylella, Yersinia and Zymomonas. In some preferred embodiments, the host cell may be a species of, but not limited to Agrobacterium, Arthrobacter, Bacillus, Clostridium, Corynebacterium, Escherichia, Erwinia, Geobacillus, Klebsiella, Lactobacillus, Mycobacterium, Pantoea, Streptomyces and Zymomonas.

In some embodiments, the bacterial host strain is an industrial strain. Numerous bacterial industrial strains are known and suitable in the present invention. In some embodiments of the invention, the bacterial host cell is of the *Bacillus* species, e.g., *B. thuringiensis*, *B. anthracis, B. megaterium*, *B. subtilis*, *B. lentus*, *B. circulans*, *B. pumilus*, *B. lautus*, *B.coagulans*, *B. brevis, B. firmus*, *B. alkaophius, B. licheniformis*, *B. clausii, B. stearothermophilus*, *B. halodurans* and *B. amyloliquefaciens*. In some embodiments the host cell will be an industrial Bacillus strain including but not limited to *B. subtilis*, *B. pumilus*, *B. licheniformis*, *B. clausii, B. stearothermophilus* and *B. amyloliquefaciens.*

In some embodiments, the bacterial host cell is of the *Escherichia* species, e.g., *E. coli.*

In some embodiments, the bacterial host cell is of the *Erwinia* species, e.g., *E*. *uredovora*, *E. carotovora*, *E. ananas*, *E. herbicola*, *E. punctata*, and *E. terreus.*

In some embodiments, the bacterial host cell is of the *Pantoea* species, e.g., *P. citrea*, and *P. agglomerans.*

In some embodiments, the bacterial host cell is of the Streptomyces species, e.g., *S. ambofaciens, S. achromogenes, S. avermitilis*, *S. coelicolor, S. aureofaciens, S. aureus*, *S. fungicidicus*, *S. griseus*, and *S. lividans*.

In some embodiments, the bacterial host cell is of the *Zymomonas.* species, e.g., Z. *mobilis*, and Z. *lipolytica.*

Strains which may be used in the practice of the invention including both prokaryotic and eukaryotic strains, and these are readily accessible to the public from a number of culture collections such as American Type Culture Collection (ATCC), Deutsche Sammlung von Mikroorganismen und Zellkulturen GmbH (DSMZ) (German Collection of Microorganisms and Cell Culture), Centraalbureau Voor Schimmelcultures (CBS), and Agricultural Research Service Patent Culture Collection, Northern Regional Research Center (NRRL).

In some particular embodiments, recombinant microorganisms encompassed by the inventions are derived from strains of *Escherichia coli*, *Bacillus, Saccharomyces*, *Streptomyces* and *Yarrowia.*

The recombinant microorganisms that are capable of producing fatty alcohols as encompassed by the invention will include heterologous genes encoding a carboxylic acid reductase. In some embodiments of the disclosure, the recombinant microorganisms will additionally comprise one or more heterologous genes selected from the group of acyl-ACP thioesterases, alcohol dehydrogenases and/or PPTases as further described below.

The present disclosure provides a process for conversion of carbon sources assimilable by recombinant microorganisms to fatty alcohols. Microorganisms have evolved efficient processes for the conversion of carbon sources to fatty acids. The presently disclosed process exploits that efficiency by diverting the fatty acids so produced to long chain fatty alcohols by metabolic engineering of the host microorganism. In one aspect, this is accomplished by developing a pathway within a recombinant host cell, which pathway is depicted in Scheme 1 below:

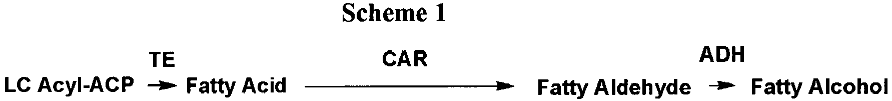

In this scheme LC Acyl-ACP refers to a long chain fatty acid (e.g., C8 - C24) bound to an acyl carrier protein by a thioester bond. The enzymes of the pathway depicted in Scheme 1 include an acyl ACP thioesterase (TE), a carboxylic acid reductase (CAR), and a ketoreductase/alcohol dehydrogenase (ADH). In a preferred embodiment of the disclosure, the CAR will be heterologous to the host cell. In some embodiments of the disclosure, the recombinant microorganism will include at least one additional heterologous gene encoding a polypeptide selected from the set of enzymes comprising: acyl-ACP thioesterase (TE) and dehydrogenase/ketoreductase (ADH). In some embodiments, the pathway of scheme 1 is the preferred pathway in bacterial host cells and particularly *E. coli* host cells.

In another scheme of the disclosure, the fatty acid may be derived from a source other than a LC Acyl-ACP; for example, the hydrolysis of triglycerides and/or phospholipids.

In a particular aspect, the recombinant microorganism further comprises a gene expressing a phosphopantetheinyl transferase polypeptide capable of attaching a pantetheine moiety to the carboxylic acid reductase polypeptide (CAR) depicted in Scheme 1 above.

### 6.3 Enzymes Useful In The Disclosed Process

### Carboxylic acid reductase (CAR)

As disclosed herein, it has been discovered that carboxylic acid reductases are capable of reducing a fatty acid to the corresponding aldehyde, as depicted below in Scheme 2:

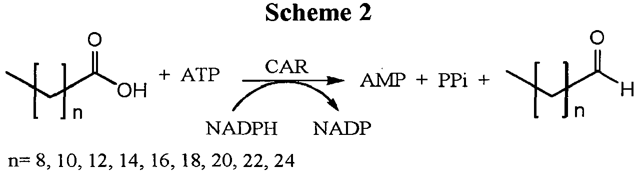

Carboxylic acid reductases (CAR) are unique ATP- and NADPH-dependent enzymes that reduce carboxylic acids, such as fatty acids to the corresponding aldehyde. CARs are multi-component enzymes comprising a reductase domain; an adenylation domain and a phosphopantetheine attachment site. As disclosed herein, fatty acids, such as those fatty acids comprising 8 to 24 carbon atoms and particularly those fatty acids comprising 12 carbon atoms (dodecanoic acid) to 18 carbon atoms (stearic acid)) may be reduced by a carboxylic acid reductase of the disclosure such as those having at least 85%, at least 90%, at least 93%, at least 95%, at least 96%, at least 97%, at least 98%, at least 99%, or even 100% sequence identity to the CAR of *Mycobacterium sp. JLS,* as illustrated in SEQ ID NO: 4; a carboxylic acid reductase having at least 85%, at least 90%, at least 93%, at least 95%, at least 96%, at least 97%, at least 98% , at least 99%, or even 100% sequence identity to the CAR of *Nocardia sp.* NRRL5646 as illustrated in SEQ ID NO: 2 or SEQ ID NO: 9; a carboxylic acid reductase having at least 85%, at least 90%, at least 93%, at least 95%, at least 96%, at least 97%, at least 98%, at least 99%, or even 100% sequence identity to the CAR of *Streptomyces griseus* as illustrated in SEQ ID NO: 6 or SEQ ID NO: 10.

In some embodiments, the carboxylic acid reductase has at least 85%, at least 90%, at least 93%, at least 95%, at least 96%, at least 97%, at least 98%, at least 99% or even 100% sequence identity to a CAR protein comprising the 4-mer GDIH appended at the amino terminus (SEQ ID NOs: 9 and 10). Reference is also made to the *Nocardia sp.* CAR disclosed in USP 6,261,814.

The present disclosure also encompasses variant CARs. The variant may comprise at least 90% (e.g., at least 93%, at least 95%, at least 96%, at least 97%, at least 98%, or at least 99%) sequence identity to SEQ ID NO: 2, SEQ ID NO: 4 or SEQ ID NO: 6. In some embodiments, the variant CAR comprises at least 90% (e.g., at least 93%, at least 95%, at least 96%, at least 97%, at least 98% and at least 99%) sequence identity with SEQ ID NO: 4 and a substitution of an amino acid at a position corresponding to position R270, A271, K274, A275, P467, Q584, E626, and/or D701when aligned with SEQ ID NO: 4. In some embodiments, the variant CAR may include an amino acid sequence that is at least 85%, (e.g., at least 90%, at least 93%, at least 95%, at least 96%, at least 97%, at least 98% and at least 99%) identical to SEQ ID NO: 4 and an amino acid substitution corresponding to R270W, A271W, K274(G/N/V/I/W/L/M/Q/S), A275F, P467S, Q584R, E626G, D701G, K274L/A369T/L380Y, K274L/V358H/E845A, K274M/T282K, K274Q/T282Y, K274S/A715T, K274W/L380G/A477T, K274W/T282E/L380V, K274W/T282Q, K274W/V358R and/or R43C/K274I in SEQ ID NO: 4. In some embodiments, the variant CAR will comprise an amino acid substitution at position K274 and one or more (e.g., 1, 2 or 3) further amino acid substitutions when the variant is aligned with SEQ ID NO: 4. In some embodiments, the CAR activity of the variant will be greater than the CAR activity of the reference or parent sequence. CAR activity can be determined for example by the assays described in examples below.

In some embodiments, a variant may encompass additional amino acid substitutions at positions other than those listed above including, for example, variants with one or more conservative substitutions. Examples of conservative substitutions are disclosed herein above. In some embodiments conservatively substituted variations of a CAR will include substitutions of a small percentage, typically less than 5%, more typically less than 2%, and often less than 1% of the amino acids of the polypeptide sequence.

As noted below, intracellular expression of a carboxylic acid reductase of the disclosure, will lead to production not only of the fatty aldehyde but also the corresponding fatty alcohol. This is the result of alcohol dehydrogenase activity within the recombinant host cell. Reference is made to Scheme 3 below. In some embodiments, the process will result in the production of fatty alcohols comprising C8, C10, C12, C14, C16, C18, C20, C22 and C24 carbons in length.

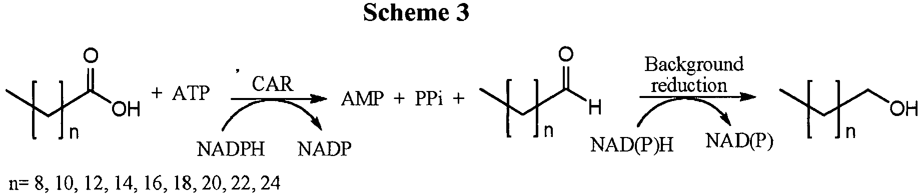

In certain embodiments of the present disclosure, the recombinant host cell expresses a carboxylic acid reductase that, as compared to its parent or the wild-type enzyme has a lower *Kₘ* for each of its carboxylic acid and ATP substrates, has an increased *Vₘₐₓ* and/or *k_{cat}* or a different carbon chain length profile with respect to the fatty aldehyde products it catalyzes for the reaction depicted in Schemes 2 and 3 or is more resistant to inhibition by increased concentrations of its carboxylic acid and ATP substrates or by increased concentrations of the fatty aldehyde, AMP, and pyrophosphate products of the reaction depicted in Schemes 2 and/or 3.

### Phosphopantetheinyl transferase (PPTase)

In some embodiments, the recombinant microorganism of the disclosure will express a phosphopantetheinyl transferase (PPTase) polypeptide which is capable of attaching a pantetheine moiety to the CAR. In some embodiments, the PPTase will be a transferase from a bacterial organism. In some embodiments, the transferase will be a *Nocardia* PPTase, (such as, but not limited to a PPTase derived from *N. iowensis* or *N. farcinica*); a Mycobacterium PPTase (such as, but not limited to a PPTase derived from *M. abscessus* (ATCC 19977), *M. sp.,* MCS, *M. vanbaalenii, M. avium, M. bovis, M. marinum* or *M. smegmatis*); a Rhodococcus PPTase (such as, but not limited to PPTases derived from *R*. *jostii*, *R. opacus*, or *R. erythropolis*) a Streptomyces PPTase (such as, but not limited to *S*. *verticillus*) or a Gordonia PPTase (such as, but not limited to a PPTase derived from *G*. *bronchialis*). PPTases derived from these organisms are known in the art and reference is made to Venkitasubramanian, P. et al 2007, J. Biological Chemistry Vol. 282 pp 478 -485 and Sanchez, C. et al., 2001 Chem. Biol. Vol. 8 pp725 - 738. In some embodiments, the PPTase will have at least 50%, at least 60%, at least 70%, at least 80%, at least 85%, at least 90%, at least 91%, at least 92%, at least 93%, at least 95%, at least 96%, at least 97%, at least 98%, at least 99% and even 100% sequence identity to SEQ ID NO: 8 or SEQ ID NO: 11.

Some host microorganisms have more than one PPTase. For example *E.coli* is believed to have three classes of PPTases and these PPTases have been classified depending on their sequence identity and substrate spectrum. In addition, two PPTases have been identified in *Bacillus subtilis.* Preferred PPTases encompassed by this disclosure are those that are involved in the modification of fatty acid ACP. In some embodiments the PPTase will be heterologous to the host microorganism, and in other embodiments, the PPTase may be derived from the host microorganism but will be over expressed by genetic manipulation in the host.

### Acyl ACP thioesterase

Acyl ACP thioesterases catalyze the hydrolysis of acyl-ACP (*i*.*e*. acylated Acyl Carrier Protein) that are intermediates in the biosynthesis of fatty acids, as depicted in Scheme 4 below, wherein n preferably is 10 - 18:

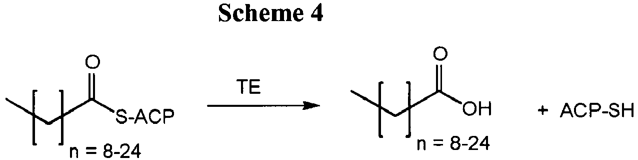

The disclosed methods preferably utilize an endogenous TE in the process of producing a fatty alcohol. However, host cells may be manipulated to include a heterologous TE. For example, host cells may over-express an acyl ACP thioesterase that has been manipulated and then introduced into the host cell. In some embodiments, the acyl-ACP thioesterase is obtained from *Escherichia coli*, *Cuphea hookeriana, Umbellularia california, Cinnamonum camphorum, Arabidipsis thaliana, Brassica junicea* and *Bradyrhiizobium japonicum* acyl-ACP thioesterases. Genes (such as tesA, tesB, fatB, fatA, fatA1 and the like) coding for TEs are known in the art and available from public database such as NCBI and GenBank. Examples include but are not limited to Accession numbers AAC73596; Q41635; AAC72881; AAC72883; AAC73555; P0ADA1; and Q39473.

In certain embodiments of the present disclosure, the recombinant host cell expresses a heterologous acyl-ACP thioesterase that, as compared to its parent or the wild-type enzyme has a lower *Kₘ* for its thioester substrate, has an increased *Vₘₐₓ* and/or *k_{cat},* or a different carbon chain length profile with respect to the fatty acid products it catalyzes for the reaction depicted in Scheme 4 above, or is more resistant to inhibition by increased concentrations of its thioester substrate or by increased concentrations of the carboxylic acid and ACP-SH products of the reaction depicted in Scheme 4.

### Alcohol dehydrogenase /ketoreductase (ADH)

Alcohol dehydrogenases (ketoreductases) catalyze the conversion of an aldehyde to the corresponding alcohol for example, as depicted in Scheme 5:

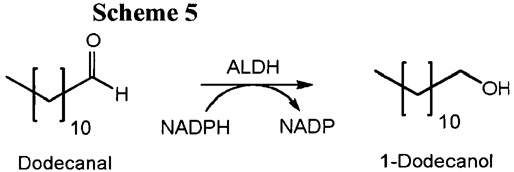

In this reaction the aldehyde, dodecanal and the corresponding alcohol, 1-dodecanol are representative of a species of a genus of various aldehyde substrates. Other preferred aldehyde reactions include the conversion of a C8, C12, C14, C16, C18, C20, C22 and C24 aldehyde to the corresponding saturated or unsaturated fatty alcohol. This reaction is energetically favorable and occurs without activation of the substrate. The method disclosed herein preferably utilizes an endogenous ADH in the process of producing a fatty alcohol. However, host cells may be manipulated to include a heterologous ADH. For example, host cells may over-express an ADH that has been manipulated and then introduced into the host cell. In some embodiments, the alcohol dehydrogenase is an *E. coli* ADH (genes coding for *E.coli* ADHs include but are not limited to dkgA and B; adhP, and yhdH). In some embodiments the ADH is a *Yarrowia. lipolytica* ADH such as but not limited to ADH1 - 4 and also NCIB accession numbers Q9UW08 (AAD51737.1); Q9UW06 (AAD51739.1); Q9UW07 (AAD51738.1) and CAG79261.

In certain embodiments of the present disclosure, the recombinant host cell expresses a heterologous alcohol dehydrogenase that, as compared to its parent or the wild-type enzyme has a lower *Kₘ* for each of its long chain aldehyde substrate, has an increased *Vₘₐₓ* and/or *k_{cat}* or a different carbon chain length profile with respect to the fatty alcohol products it catalyzes for the reaction depicted in Scheme 5 above, or is more resistant to inhibition by increased concentrations of its fatty aldehyde substrate or by increased concentrations of the fatty alcohol product.

The recombinant host cells of the present disclosure may also comprise mutations that lead to an increase in the levels of fatty acid produced by the host cell as well as mutations resulting in a decreased rate of utilization of fatty acids in competing pathways, *e.g.,* lipid synthesis, fatty acid β-oxidation, sphingolipid biosynthesis, and protein acylation. Additional mutations that may be introduced into the recombinant host cells of the disclosure include those enhancing processes that result in the extracellular accumulation of the fatty alcohols synthesized in the recombinant host cells of the disclosure. In certain embodiments, the recombinant host cells comprise combinations of mutations that, collectively, *e*.*g*., provide increased levels of fatty acid production coupled with decreased rates of utilization of those fatty acids in competing pathways, as well as increased extracellular accumulation of long chain fatty alcohols.

In certain embodiments, the recombinant host cells of the disclosure comprise mutations eliminating or selectively repressing metabolic regulatory pathways, e.g., feedback inhibition by long chain fatty acids, whereby the biosynthesis of fatty acids is repressed while the production of enzymes for fatty acid catabolism are induced, or glucose repression of pathways for the transport and use of alternative carbon sources, such as galactose.

### 6.4 Nucleic Acids, Genes and Vectors Useful In The Disclosed Process

In another embodiment, the present disclosure provides DNA constructs, vectors and polynucleotides encoding the enzymes (e.g., CARs) of the disclosure. Polynucleotides may be operably linked to one or more heterologous regulatory sequences that control gene expression to create a recombinant polynucleotide capable of expressing the polypeptide. Expression constructs containing a heterologous polynucleotide encoding the polypeptides of the disclosure can be introduced into appropriate host cells to express the corresponding polypeptide. Because of the knowledge of the codons corresponding to the various amino acids, availability of a protein sequence provides a description of all the polynucleotides capable of encoding the subject polypeptide. The degeneracy of the genetic code, where the same amino acids are encoded by alternative or synonymous codons allows an extremely large number of nucleic acids to be made, all of which encode the enzymes encompassed by the disclosure. Thus, having identified a particular amino acid sequence, those skilled in the art could make any number of different nucleic acids. In some embodiments, the codons are selected to fit the host cell in which the protein is being produced. For example, preferred codons used in bacteria are used to express the gene in bacteria and preferred codons used in yeast are used for expression in yeast. In some embodiments, the polynucleotide comprises a nucleotide sequence encoding a CAR polypeptide with an amino acid sequence that has at least about 80%, 85%, 86%, 87%, 88%, 89%, 90%, 91%, 92%, 93%, 94%, 95%, 96%, 97%, 98%, or 99% or more sequence identity to SEQ ID NOs: 2, 4, 6, 9 or 10. In some embodiments, the polynucleotide will be SEQ ID NO: 1, SEQ ID NO: 3 or SEQ ID NO: 5. In some embodiments, the polynucleotide sequence will have at least 90%, at least 93%, at least 95%, at least 96%, at least 97%, at least 98% and at least 99% sequence identity to SEQ ID NOs: 1, 3 or 5. In some embodiments, the polynucleotide will be a sequence that hybridizes to SEQ ID NO: 1, SEQ ID NO: 3, or SEQ ID NO: 5 under high stringency conditions.

In some embodiments the polynucleotide encoding a PPTase useful in a process of producing fatty alcohols according to the disclosure will have at least 50%, at least 60%, at least 70%, at least 80%, at least 85%, at least 90%, at least 93%, at least 95%, at least 96%, at least 97%, at least 98%, at least 99% and even 100% sequence identity to SEQ ID NO: 7.

An isolated polynucleotide encoding a polypeptide encompassed by the disclosure may be manipulated in a variety of ways to provide for expression of the polypeptide. Manipulation of the isolated polynucleotide prior to its insertion into a vector may be desirable or necessary depending on the expression vector. The techniques for modifying polynucleotides and nucleic acid sequences utilizing recombinant DNA methods are well known in the art. Guidance is provided in Sambrook et al., 2001, Molecular Cloning: A Laboratory Manual, 3rd Ed., Cold Spring Harbor Laboratory Press; and Current Protocols in Molecular Biology, Ausubel. F. ed., Greene Pub. Associates, 1998, updates to 2006.

One skilled in the art is well aware of techniques which may be used to generate polynucleotides which code for variant CARs and these techniques include but are not limited to classical and/or synthetic DNA shuffling techniques. Classical DNA shuffling generates variant DNA molecules by in vitro homologous recombination from random fragmentation of a parent DNA followed by reassembly using ligation and/or PCR, which results in randomly introduced point mutations. A resulting library can in turn be screened and further shuffled. Synthetic DNA shuffling may also be used wherein a plurality of oligonucleotides are synthesized which collectively encode a plurality of mutations to be combined. Recombination-based evolution may further be complemented by protein sequence activity relationships (e.g., ProSAR), which incorporates statistical analysis in targeting amino acid residues for mutational analysis. See e.g., Fox et al., Nature Biotechnology 25: 338 - 344 92007).

The polynucleotides encoding polypeptides encompassed by the disclosure are operably linked to a promoter and optionally other regulatory sequences. Suitable promoters include constitutive promoters, regulated promoters and inducible promoters.

For bacterial host cells, suitable promoters for directing transcription of the nucleic acid constructs of the present disclosure include the promoters obtained from *E. coli.* Other suitable promoter may be the *E. coli* lac operon, *Streptomyces coelicolor* agarase gene (dagA), *Bacillus subtilis* levansucrase gene (sacB), *Bacillus licheniformis* alpha-amylase gene (amyL), *Bacillus stearothermophilus* maltogenic amylase gene (amyM), *Bacillus arnyloliquefaciens* alpha-amylase gene (amyQ), *Bacillus licheniformis* penicillinase gene (penP), *Bacillus subtilis* xylA and xylB genes, and prokaryotic beta-lactamase gene (Villa-Kamaroff et al., 1978, Proc. Natl Acad. Sci. USA 75: 3727-3731), as well as the *tac* promoter (DeBoer et al., 1983, Proc. Natl Acad. Sci. USA 80: 21-25). Further promoters are described in "Useful proteins from recombinant bacteria" in Scientific American, 1980, 242:74-94; and in Sambrook et al., *supra*.

For filamentous fungal host cells, suitable promoters for directing the transcription of the nucleic acid constructs of the present disclosure include but are not limited to promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (glaA), *Rhizomucor miehei* lipase, *Aspergillus oryzae* alkaline protease, *Aspergillus oryzae* triose phosphate isomerase, *Aspergillus nidulans* acetamidase, and *Fusarium oxysporum* trypsin-like protease (WO 96/00787).

In a yeast host, useful promoters can be from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH2/GAP), and *Saccharomyces cerevisiae* 3-phosphoglycerate kinase; (TEF1) promoter. Other useful promoters for yeast host cells are described by Romanos et al., 1992, Yeast 8:423-488.

The control sequence may also be a suitable transcription terminator sequence, a sequence recognized by a host cell to terminate transcription. The terminator sequence is operably linked to the 3' terminus of the nucleic acid sequence encoding the polypeptide. Any terminator which is functional in the host cell of choice may be used in the present disclosure. Exemplary terminators for yeast host cells can be obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992, *supra*.

The control sequence may also be a suitable leader sequence, a nontranslated region of an mRNA that is important for translation by the host cell. The leader sequence is operably linked to the 5' terminus of the nucleic acid sequence encoding the polypeptide. Any leader sequence that is functional in the host cell of choice may be used. Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3' terminus of the nucleic acid sequence and which, when transcribed, is recognized by the host cell as a signal to add polyadenosine residues to transcribed mRNA. Any polyadenylation sequence which is functional in the host cell of choice may be used in the present disclosure. Exemplary polyadenylation sequences for filamentous fungal host cells can be from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase. Useful polyadenylation sequences for yeast host cells are described by Guo and Sherman, 1995, Mol Cell Bio 15:5983-5990. The control sequence may also be a signal peptide coding region that codes for an amino acid sequence linked to the amino terminus of a polypeptide and directs the encoded polypeptide into the cell's secretory pathway.

The 5' end of the coding sequence of the nucleic acid sequence may inherently contain a signal peptide coding region naturally linked in translation reading frame with the segment of the coding region that encodes the secreted polypeptide. Alternatively, the 5' end of the coding sequence may contain a signal peptide coding region that is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present disclosure.

Exemplary signal peptides for yeast host cells can be from the genes for *Saccharomyces cerevisiae* alpha-factor and *Saccharomyces cerevisiae* invertase. Other useful signal peptide coding regions are described by Romanos et al., 1992, *supra*. The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (aprE), *Bacillus subtilis* neutral protease (nprT), *Saccharomyces cerevisiae* alpha-factor, *Rhizomucor miehei* aspartic proteinase, and *Myceliophthora thermophila* lactase (WO 95/33836). Where both signal peptide and propeptide regions are present at the amino terminus of a polypeptide, the propeptide region is positioned next to the amino terminus of a polypeptide and the signal peptide region is positioned next to the amino terminus of the propeptide region.

The recombinant expression vector may be any vector (*e*.*g*., a plasmid or virus), which can be conveniently subjected to recombinant DNA procedures and can bring about the expression of the polynucleotide sequence. The choice of the vector will typically depend on the compatibility of the vector with the host cell into which the vector is to be introduced. The vectors may be linear or closed circular plasmids. The expression vector will typically include a selectable marker such as but not limited to antibiotic resistance such as ampicillin, kanamycin, chloramphenicol or tetracycline resistance. Suitable markers for yeast host cells are ADE2, HIS3, LEU2, LYS2, MET3, TRP1, and URA3. Examples of expression vectors which may be useful in the present disclosure are commercially available for example from Sigma-Aldrich Chemicals, St. Louis MO. and Stratagene, LaJolla CA, and plasmids which are derived from pBR322 (Gibco BRL), pUC (Gibco BRL), pREP4, pCEP4 (Invitrogen) or pPoly (Lathe et al., 1987, Gene 57:193-201). In one embodiment, the present disclosure provides an autonomous replicating plasmid for expression of heterologous genes in *Yarrowia* and particularly in *Y. lipolytica.* This plasmid vector (pCEN351; FIG. 1) was engineered with two antibiotic selection marker cassettes for resistance to hygromycin and phleomycin (Hyg B ^{R} or Ble ^{R}, respectively). In this embodiment, expression of each cassette is independently regulated by a strong, constitutive promoter isolated from *Y*. *lipolytica*: pTEF1 for Ble ^{R} expression and pRPS7 for HygB ^{R} expression. When this plasmid, pCEN351, was transformed into *Y. lipolytica,* it conferred resistance to both hygromycin and phleomycin, validating the functionality of each cassette. This plasmid and the two selection markers enable expression of heterologous genes useful for fatty alcohol production in yeast, *inter alia*, *Y*. *lipolytica*. The antibiotic resistance cassettes constructed above are also useful for gene disruptions in *Y. lipolytica.* In such embodiments, for example, the antibiotic resistance cassettes are used to perform knockouts of genes involved in degradation of free fatty acids and fatty acyl-CoA compounds. Such gene disruption can be performed by homologous recombination, an established method in *Y. lipolytica* (*see e.g.* EP 0 138 508 B1, US Patent No. 4,889,741 and US Patent No. 5,071,764,).

In some embodiments a vector according to the disclosure will comprise a polynucleotide sequence coding for a CAR as described herein. In other embodiments, a vector may include a polynucleotide coding for a PPTase as described herein, for example a PPTase having at least 80% sequence identity to the PPTase of SEQ ID NO: 8. In some embodiments the polynucleotide coding for the PPTase and the polynucleotide coding for the CAR are both on the same plasmid. In some preferred embodiments, the vector is a plasmid such as pCEN351 which can be adapted for over-expression of the fatty alcohol pathway genes identified herein, by replacing one of the selection markers with a gene(s) of interest. For example, the Ble ^{R} gene can be replaced with different genes encoding enzymes for reduction of fatty acids (e.g. TE).

Methods for the transformation of *Y. lipolytica* strain PO1g (Yeastern Biotech) are known in the art. In other embodiments, modified procedures for the transformation *Y*. *lipolytica* strains have been developed. In certain embodiments, the expression vectors of the present disclosure are integrated into the chromosome of the recombinant host strain and comprise one or more heterologous genes operably associated with a promoter useful for production of long chain fatty alcohols. In other embodiments, the expression vectors are extrachromosomal replicative DNA molecules, *e*.*g*. plasmids, that are found in low copy number (e.g. 1 - 10 copies per genome equivalent) or in high copy number (more than 10 copies per genome equivalent).

In certain aspects, the present disclosure is directed to expression vectors comprising heterologous genes useful for the production of fatty alcohols (e.g., C8, C10, C12, C14, C16, C18, C20, C22 and C24), wherein each heterologous gene is operably linked with a promoter that may be independently selected to provide a desired level of expression of the heterologous gene.

### 6.5 Culture Conditions And Long Chain Fatty Alcohol Recovery

In certain embodiments of the present disclosure, the recombinant host strain comprising at least one heterologous gene encoding a CAR is cultured in an aqueous nutrient medium comprising an assimilable source of carbon, whereby long chain fatty alcohols are produced. The individual components of such media are available from commercial sources, *e*.*g*., under the Difco™ and BBL™ trademarks.

In one non-limiting example, the aqueous nutrient medium is a "rich medium" comprising complex sources of nitrogen, salts, and carbon, such as YP medium, comprising 10 g/L of peptone and 10 g/L yeast extract of such a medium.

In other non-limiting embodiments, the aqueous nutrient medium comprises a mixture of Yeast Nitrogen Base (Difco) in combination supplemented with an appropriate mixture of amino acids, *e.g.* SC medium. In particular aspects of this embodiment, the amino acid mixture lacks one or more amino acids, thereby imposing selective pressure for maintenance of an expression vector within the recombinant host strain.

Fermentation of the recombinant host strain comprising at least one heterologous gene useful for production of long chain fatty alcohols is carried out under suitable conditions and for a time sufficient for production of long chain fatty alcohols. Many references are available for the culture and production of many cells, including cells of bacterial, yeast and fungal cells. Cell culture media in general are set forth in Atlas and Parks (eds.) The Handbook of Microbiological Media (1993) CRC Press, Boca Raton, FL. Additional information for cell culture is found in available commercial literature such as the Life Science Research Cell Culture Catalogue (1998) from Sigma- Aldrich, Inc (St Louis, MO) ("Sigma-LSRCCC") and, for example, The Plant Culture Catalogue and supplement (1997) also from Sigma-Aldrich, Inc (St Louis, MO) ("Sigma-PCCS").

In some embodiments, cells expressing the CAR and/or other recombinant enzymes of the disclosure are grown under batch or continuous fermentations conditions. Classical batch fermentation is a closed system, wherein the compositions of the medium is set at the beginning of the fermentation and is not subject to artificial alternations during the fermentation. A variation of the batch system is a fed-batch fermentation which also finds use in the present disclosure. In this variation, the substrate is added in increments as the fermentation progresses. Fed-batch systems are useful when catabolite repression is likely to inhibit the metabolism of the cells and where it is desirable to have limited amounts of substrate in the medium. Batch and fed-batch fermentations are common and well known in the art. Continuous fermentation is an open system where a defined fermentation medium is added continuously to a bioreactor and an equal amount of conditioned medium is removed simultaneously for processing. Continuous fermentation generally maintains the cultures at a constant high density where cells are primarily in log phase growth. Continuous fermentation systems strive to maintain steady sate growth conditions. Methods for modulating nutrients and growth factors for continuous fermentation processes as well as techniques for maximizing the rate of product formation are well known in the art of industrial microbiology.

In various aspects, such culturing or fermentations are carried out at a temperature within the range of from about 10°C to about 80°C, from about 15°C to about 75°C, from about 15°C to about 65°C, from about 20°C to about 60°C, from about 20°C to about 55°C, from about 20°C to about 50°C, and from about 25°C to about 40°C. In other embodiments, the fermentation is carried out for a period of time within the range of from about 8 hours to about 240 hours, from about 10 hours to about 192 hours, from about 20 hours to about 96 hours, or from about 24 to about 72 hours. In other embodiments, culturing is carried out at a pH range of 3.5 to 7.5 (such as pH 4.0 to 7.0; pH 4.5 to 7.0 and pH 5.0 to 7.0).

Carbon sources useful in the aqueous fermentation medium or broth of the disclosed process in which the recombinant microorganisms are grown are those assimilable by the recombinant host strain. Assimilable carbon sources are available in many forms and include renewable carbon sources and the cellulosic and starch feedstock substrates obtained there from. Such examples include for example monosaccharides, disaccharides, oligosaccharides, saturated and unsaturated fatty acids, succinate, acetate and mixtures thereof. Further carbon sources include, without limitation, glucose, galactose, sucrose, xylose, fructose, glycerol, arabinose, raffinose, lactose, maltose, and mixtures thereof. In one aspect of this embodiment, the fermentation is carried out with a mixture of glucose and galactose as the assimilable carbon source. In another aspect, fermentation is carried out with glucose alone to accumulate biomass, after which the glucose is substantially removed and replaced with an inducer, e.g., galactose for induction of expression of one or more heterologous genes involved in fatty alcohol production. In still another aspect, fermentation is carried out with an assimilable carbon source that does not mediate glucose repression, *e.g.,* raffinose, to accumulate biomass, after which the inducer, *e.g.,* galactose, is added to induce expression of one or more heterologous genes involved in fatty alcohol production. In some preferred embodiments, the assimilable carbon source is from cellulosic and starch feedstock derived from but not limited to, wood, wood pulp, paper pulp, grain, corn stover, corn fiber, rice, paper and pulp processing waste, woody or herbaceous plants, fruit or vegetable pulp, distillers grain, grasses, rice hulls, wheat straw, cotton, hemp, flax, sisal, corn cobs, sugar cane bagasse, switch grass and mixtures thereof.

In certain aspects, the disclosure relates to a process for the biologically-derived production of fatty alcohols which comprises culturing the recombinant microbial host cell said host cell including a polynucleotide encoding a heterologous CAR polypeptide and culturing the recombinant microorganism in an aqueous nutrient medium comprising an assimilable source of carbon under suitable culture conditions for a sufficient period of time to allow the production the fatty alcohols and further recovering the fatty alcohols. In certain embodiments the recombinant host cell is a yeast such as but not limited to a *Saccharomyces cerevisiae* or *Yarrowia lipolytica.* In other embodiments, the recombinant host cell is a bacterial cell, such as but not limited to cells of *E.coli* or *Bacillus sp.*

In some embodiments the disclosure relates to a process for the biologically-derived production of fatty alcohols in a yeast cell which comprises a) culturing a recombinant yeast cell comprising a polynucleotide which encodes a CAR as herein described in an aqueous nutrient medium comprising an assimilable carbon source derived from a cellulosic or starch feedstock under suitable culture conditions for a sufficient period of time to allow expression of the CAR; b) producing the fatty alcohol and c) recovering the fatty alcohol. In some embodiments, the yeast cell is a *Saccharomyces* strain (e.g., *S. cerevisiae)* or a *Yarrowia* strain (e.g., *Y. lipolytica*).

While the fatty alcohols produced by the process of the disclosure include both saturated and unsaturated fatty alcohols, including monounsaturated fatty alcohols, with one or more double bonds (e.g., Δ9-hexadecenol), some preferred fatty alcohols include octan-1-ol, decan-1-ol, dodecan-1-ol, tetradecan-1-ol, hexadecane-1-ol, octadecan-1-ol, and icosan-1-ol. In a most preferred embodiment, the produced fatty alcohols will include C14, C16 and C18 fatty alcohols such as tetradecan-1-ol, hexadecane-1-ol, and octadecan-1-ol.

In some embodiments of the process encompassed by the disclosure, the production of fatty alcohols (C8 - C24) from a recombinant host cell will be in the range of about 2 mg/L to 250 g/L; about 2 mg/L to 200 g/L; about 5 mg/L to 150 g/L; about 10 mg/L to 150 g/L; and about 50 mg/L to 100 g/L of fermentation media. In some embodiments, the amount of fatty alcohol produced is greater than 500 mg/L, greater than 1.0 g/L, greater than 5.0 g/L, greater than 10.0 g/L greater than 25 g/L greater than 50 g/L, greater than 75 g/L, greater than 100g/L, greater than 150 g/L and also greater than 175 g/L of media. For example, in some embodiments, the amount of fatty alcohol produced by a recombinant yeast cell according to the disclosure will be at least 2 mg/L, also at least 5 mg/L, also at least 10 mg/L and also at least 1g/L of media.

In some embodiments, the production of fatty alcohols by the process of the disclosure will be in the range of about 0.1 mg/g to 10 g/g dry cell weight (DCW); in the range of about 100 mg/g to 10 g/g DCW, in the range of 500 mg/g to 10g/g DCW and also in the range of 1 g/g to 5 g/g DCW.

In some embodiments the production of fatty alcohols having C8 to C20 carbons in length will comprise at least 85%, at least 90%, at least 93%, at least 95%, at least 97% and at least 98% of the total isolated fatty alcohols. In some embodiments, the production of fatty alcohols having C10 to C18 carbons in length will comprise at least 85%, at least 88%, at least 90%, at least 93%, and at least 95% of the total produced isolated fatty alcohols.

Recovering when used in reference to "recovering" or "isolating" the fatty alcohols produced by a recombinant microorganism according to the disclosure includes, but is not limited to, recovering the fatty alcohols from the recombinant cells or recovering the fatty alcohols from the extracellular environment such as the culture media. In some embodiments, the fatty alcohols may be produced and released (e.g., secreted) from the recombinant cells into the culture or fermentation media. In other embodiments the recombinant or host cell is lysed prior to separation of the produced fatty alcohols. In some embodiments, the recovered fatty alcohols are further purified. Purification does not require absolute purity but is a relative term and means that the recovered fatty alcohols may be further separated from other cellular components such as but not limited to other proteins, hydrocarbons and lipids.

In certain aspects of the disclosure, long chain fatty alcohols are isolated by solvent extraction of the fermentation medium with a suitable water-immiscible solvent. Phase separation followed by solvent removal provides the long chain fatty alcohol which may then be further purified and fractionated using methods and equipment known in the art. In other aspects of the disclosure, the long chain fatty alcohols coalesce to form a water-immiscible phase that can be directly separated from the nutrient aqueous medium either during the fermentation or after its completion, or precipitate from the aqueous medium and can be separated by filtration or solvent extraction. Reference is made to Can J. Biochem Physiol., 1959, 37:911-7, J. Biol. Chem., 1957, 226, 497 -509 and examples herein below.

In some embodiments the fatty alcohols will be further reduced to the corresponding alkanes. Means for reducing fatty alcohols are well known in the art. In one example, the fatty alcohol may be dehydrated to a corresponding alkene and then the alkene is hydrogenated to the corresponding alkane.

In another embodiment, the disclosure relates to a method of catalytically reducing a fatty acid substrate to a corresponding C8 - C24 carbon containing fatty aldehyde comprising mixing an effective amount of a CAR polypeptide according to the disclosure with a fatty acid substrate and cofactors selected from the group of ATP and NADPH and incubating the mixture for a period of time and under conditions suitable to achieve reduction of the substrate to the corresponding fatty aldehyde. In some embodiments the fatty aldehyde is reduced to the corresponding fatty alcohol.

In some embodiments the fatty alcohol may be further converted to a fatty ester by either chemical or enzymatic means (such as by the use of lipases). Methods of conversion to fatty esters are well known in the art.

### 6.6 Post production and compositions

The fatty alcohols produced by the process described herein can either be used directly or further processed for example but not limited to use in the production of fuels, chemicals, lubricants, cosmetics and fuel blends. Fuels include gasoline, diesel, and jet fuels and particularly diesel and jet fuels. In addition, the fatty alcohols or derivatives produced there from can be combined with other fuels or fuel additives to produce fuels having desired properties. Such other fuels may include traditional fuels, such as alcohols and petroleum based fuels. Fuel additives may include but are not limited to cloud point lowering additives and surfactants. In some embodiments, the fuel composition comprising a fatty alcohol produced according to the disclosure and derivatives thereof having C8 to C24 will include at least about 5%, 10%, 15%, 20%, 25%, 30%, 35%, 40%, 45%, 50%, 55%, 60%, 65%, 70%, 75%, and 80% of the fatty alcohol or derivative thereof. In some embodiments, the percent of fatty alcohols or derivatives thereof will be between 5% and 50%. In some embodiments the percent will be greater than 5% but less than 60%.

In some embodiments, the term "biofuel" composition is used to distinguish a fuel composition comprising a fatty alcohol or derivative thereof made by the biological process as disclosed herein which includes production and/or secretion of a fatty alcohol from a recombinant microorganism which is grown on a carbon source from renewable feedstock as opposed to a fatty alcohol or derivative thereof made from a petroleum based carbon source.

### 6. EXAMPLES

Various features and embodiments of the disclosure are provided in the following representative examples, which are intended to be illustrative and not limiting.

### Example 1

### Gene acquisition

Wild-type *Nocardia* NRRL5646, *Mycobacteria sp.* JLS, and *Streptomyces griseus* carboxylic acid reductases (CARs) and *Nocardia* phosphopantetheine transferase (PPTase) genes were designed for expression in *E. coli, S. cerevisiae,* and *Y. lipolytica* based on the reported amino acid sequences *(*Nocardia CAR: Appl Environ Microbiol (2004) v 70 p1874, *S. griseus* CAR: J. Bacteriol. 190 (11), 4050-4060 (2008), *Mycobacteria* CAR: GenBank accession number YP_001070587, Nocardia PPTase: Biol. Chem. 282 (1), 478-485 (2007). Codon optimization was performed using an algorithm as described in Example 1 of WO2008042876. The genes were synthesized by Genscript (Piscataway, NJ) with flanking restriction sites for cloning into *E.coli* vector pCK 110900 described in US Pat. Appln. Pub. 2006 0195947. Nucleotide sequences for *Sfi*I sites were added to the 5' end and 3' end of the gene as well as the t7g10 RBS in front of the ATG start codon. The genes were provided in the vector pUC57 by Genscript (Piscataway, NJ) and the sequences verified by DNA sequencing. The sequences of the codon optimized genes correspond to SEQ ID NO: 1, SEQ ID NO: 3, SEQ ID NO: 5 and SEQ ID NO: 7. The corresponding expressed polypeptide sequences are designated SEQ ID NOs: 2 and 9; SEQ ID NO: 4; SEQ ID NOs: 6 and 10; and SEQ ID NOs: 8 and 11. Reference is made to Figures 3, 4, 5, and 6.

### Example 2

### Expression and activity of CARs and PPTase in E. coli

### (a) Construction of vectors to express CARs and PPTase in E. coli.

The genes were cloned into the vector pCK11900-I (depicted as Fig. 3 in US Pat. Appln. Pub. 2006 0195947) under the control of a *lac* promoter using the *Sfi* I restriction sites. The PPTase gene as well as the t7g10 RBS was added upstream of the ATG start codon for each of the CAR genes by restriction free cloning (J Biochemical Biophysical Methods, 2006, 67-74. The expression vector also contained the P15a origin of replication and the chloramphenicol resistance gene. The resulting plasmids were introduced into *E. coli* BW25113 (Δ*fadE*) (Nature 418(6896):387-9, (2002)) by routine transformation methods. (b) *In vivo* activity of CARs in recombinant *E. coli.*

Recombinant *E. coli* strains comprising a plasmid containing a heterologous gene encoding either the *Nocardia* NRRL5646, the *Mycobacteria sp. JLS,* or the *Streptomyces griseus* carboxylic acid reductase, were grown in Luria Bertani Broth (LB) medium supplemented with 1% glucose and 30 µg/mL chloramphenicol (CAM), for approximately 16-18 hours (overnight) at 30°C, in a shaker incubator at 200 rpm. A 5% inoculum was used to initiate fresh 50 mL Luria Bertani Broth (LB) culture supplemented with 30 µg/mLCAM. The culture was incubated for 2.5 hours 30°C, 200 rpm to an OD₆₀₀ of about 0.6 to about 0.8, at which point expression of the heterologous carboxylic acid was induced with isopropyl-β-D-thiogalactoside (IPTG) (1 mM final concentration). Incubation was continued for about 16 hours (overnight) under the same conditions. Cells were collected by centrifugation for 10 minutes at 6000 rpm in F15B-8x50C rotor. Aliquots of 40 OD₆₀₀ units of each culture were centrifuged and the cell pellets were resuspended in 0.5 mL of 6.7% Na₂SO₄ and then extracted with isopropanol:hexane (0.8:1.2) for 20 minutes. The extract was centrifuged and a 400 µL sample taken of the top organic layer. The solvent in the sample was evaporated under a nitrogen stream and the residue derivatized with 100 µL *N,O*-Bis(trimethylsilyl)trifluoroacetamide) (BSTFA) at 37°C for 1 hour, and then diluted with 100 µL of heptanes before analysis by GC-FID and GC-MS. In addition, 0.5 mL of the culture medium (after removal of cells by centrifugation) was combined with 0.5 mL methanol:chloroform (1:1) and extracted for 20 minutes. The lower organic phase was collected, solvent evaporated and the residue derivatized with BSTFA as above. A 1µL sample was analyzed by GC-MS or GC-FID with the split ratio 1:10. GC parameters: initial oven temperature 80°C and held at 80°C for 3 minutes. The oven temperature was increased to 200°C at a rate of 50°C/minute followed by rate of 10°C/minute to 270°C, and then 20°C/minute to 300°C, and then held at 300°C for five minutes. Under the conditions tested, expression of both the *Nocardia* NRRL5646 and *Mycobacteria sp. JLS* carboxylic acid reductase in *E. coli* resulted in the intracellular production of long chain fatty alcohols (see Table 2). In both cases PPTase improved the activity of the CAR enzyme from 1 to 2 times. Secreted fatty alcohols were not detected. Identification of individual fatty alcohol was done by comparison to commercial standards (Sigma Chemical Company, 6050 Spruce St. Louis, MO 63103).

**Table 2: Fatty alcohol profile exhibited by recombinant E. coli host cells over-expressing heterologous CAR genes.**

| | **Cellular fatty alcohol composition^{a}** | | | | | | **E Estimated productivity^{b}** |
|---|---|---|---|---|---|---|---|
| **Enzyme** | **C C12:0** | **C 4 C14:0** | **C16:1** | **C16:0** | **C18:1** | **C18:0** | (µg/OD600) |
| *Noc Nocardia CAR* | < 10 | <10 | 20-40 | 2 20-40 | 20-40 | ND | 0.1-0.5 |
| *Mycob Mycobacterium* CAR | < 10 | 10 >40 | 20-40 | 10-20 | ND | ND | <0.1 |

| | | | | | | | |
|---|---|---|---|---|---|---|---|
| ^{a}:The relative amounts of each fatty alcohol component are expressed as a % of the total fatty alcohols detected using TMS derivative *via* GC/FID or GC/MS. Endogenous fatty alcohols include: C12:0 (1-dodecanol), no C12:1 (1-dodecenol) was detected, C14:0 (1-tetradecanol), no C14:1 (1-tetradecenol) was detected, C16:1 *(cis* Δ⁹-1-hexadecenol), C16:0 (1-hexahecanol), C18:1 (*cis* Δ¹¹-1-octadecenol), 18:0 (1-octadecanol). ND: not detected. ^{b}: Enzyme productivity was estimated using external standard (1 OD600 unit corresponds to approximately 0.3 mg of cells). | | | | | | | |

### (c) In vitro activity of CARs in recombinant E. coli.

Preparation of cell pellets containing CARs and PPTase in 96-well plates:
The recombinant *E. coli* strains comprising a plasmid containing a heterologous gene encoding either the *Nocardia* NRRL5646, the *Mycobacteria sp. JLS,* or the *Streptomyces griseus* carboxylic acid reductase and the *Nocardia* PPTase, were grown in a 96-well shallow plate containing 180 µL Luria Bertani Broth (LB), supplemented with 1% glucose and 30 µg/mL chloramphenicol (CAM), for approximately 16 hours (overnight) at 30°C, 200 rpm at 85% humidity. A 20 µL of each seed culture was transferred into 390 µL Terrific Broth (TB) supplemented with 30 µg/mL CAM, in an individual well of a 96-deep well plate. The latter plate was incubated for 2.5 hours 30°C, 200 rpm at 85% humidity to an OD₆₀₀ of about 0.6 to about 0.8, at which point expression of the heterologous carboxylic acid was induced with isopropyl-β-D-thiogalactoside (IPTG) (1 mM final concentration). Incubation was continued for about 16 hours (overnight) under the same conditions. Cells were collected by centrifugation for 10 minutes at 4000 rpm.

Preparation of crude lysate of CAR enzymes and PPTase in 96-well plates: To each well of the 96-deep well plate containing the pelleted cells prepared above, 0.4 mL of lysis buffer (100 mM KH2PO4, pH 7.5, 1 mg/mL lysozyme, 0.5 mg/mL polymixin B sulfate (PMBS) was added. Cells were lysed for 2 h at room temperature with shaking on a bench-top shaker. Each plate was then centrifuged for 10 minutes at 4000 rpm at 4°C. The clear supernatant recovered after the centrifugation was recovered and used for the biochemical assays.

*In vitro* activity of CARs in 96-well plates using spectroscopic method: An aliquot of the supernatant obtained above was added to the assay mixture comprising 100 mM phosphate buffer (pH 7.5), 0.2 mg/mL NADPH, 1mM ATP, 1mM CoA and 5 mM substrate (e.g., benzoic acid, octanoic acid, and decanoic acid). The reaction was monitored by measuring the decrease of fluorescent emission of NADPH at 440 nm as a function of time. The results were plotted as relative fluorescent units (RFU) of NADPH versus time. The slope of the plot (RFU/min) was used to determine the rate of reaction.

*In vitro* activity of CARs in 96-well plates using GC method:
An aliquot (20 µL) of the CAR/PPTase supernatant obtained above was added to the assay mixture comprising 100 mM phosphate buffer (pH 7.5), 0.2 mg/mL NADPH, 2 mM ATP, 1 mM CoA, 4 mM hexadecanoic acid and 3% isopropyl alcohol (IPA). An engineered ketoreductase enzyme (2 mg/mL) (SEQ ID NO. 77 in WO2008103248A1) was added to regenerate NADPH in the reaction by converting IPA to acetone. After overnight incubation at room temperature on a bench top shaker, the reaction mixture was extracted by 600 µL MTBE containing methyl hexadecanoate as an internal standard. A 1µL sample was analyzed by GC-MS or GC-FID with conditions similar to those as described above (example 2b). Under the conditions tested approximately 50% conversion of substrate was detected by both *Mycobacteria* and *Nocardia* CARs. The data obtained indicated that both the enzymes (coupled with an apparent background *E. coli* alcohol dehydrogenase/ketoreductase activity) converted hexadecanoic acid to hexadecanol. Under the conditions tested, the *Streptomyces griseus* CAR did not display significant activity.

### (d) In vitro screening of Mycobacterium CAR variants in recombinant E. coli.

Random and targeted mutagenesis of *Mycobacterium* CAR was used to generate variants that were screened (growth, lysis, and assay) as described in examples 2C I, II and IV. A number of variants with 0.7 to 3.4-fold activity relative to wt *Mycobacterium* CAR (SEQ ID NO: 4) were identified (Table 3). Combinations of these mutations is expected to further improve the relative activity compared to wt *Mycobacterium* CAR.

**Table 3: Amino acid substitutions and relative activity of Mycobacterium CAR variants. The variants are aligned and compared to the CAR sequence of SEQ ID NO: 4.**

| **Sequence changes (Compare to WT *Mycobacterium* CAR)** | **elative activity (compare to WT *Mycobacterium* CAR)** |
|---|---|
| 1W | 0.8 |
| 5F | 1.8 |
| 1G | 0.7 |
| 6G | 1.9 |
| 4G | 1.1 |
| 4L; A369T; L380Y | 2.1 |
| 4L; V358H; E845A | 2.5 |
| 4M; T282K | 2.3 |
| 4N | 1.6 |
| 4Q; T282Y | 1.2 |
| 4S; A715T | 0.9 |
| 4V | 1.8 |
| 4W; L380F | 3.4 |
| 4W; L380G; A477T | 2.6 |
| 4W; T282E; L380V | 2.5 |
| 4W; T282Q | 3.3 |
| 4W; V358R | 3.4 |
| 7S | 1.2 |
| 4R | 1.0 |
| 3W | 1.1 |
| C; K274I | 2.9 |

### Example 3

### Expression and activity of CARs in S. cerevisiae

### (a) Construction of vectors to express CARs and PPTase in S. cerevisiae.

The *Nocardia* PPTase gene was PCR amplified and cloned downstream of the GAL10 promoter using NotI and SpeI sites into vector pESC-LEU (Stratagene, La Jolla, CA) to construct pCEN0314. The CAR genes were PCR amplified and cloned using BamHI and SalI sites into vector pCEN0314 under the control of the GAL1 promoter. These vectors contain a 2 micron yeast origin and LEU2 gene for selection in *S. cerevisiae* YPH499 (Stratagene, La Jolla, CA).

### (b) In vivo activity of CARs in recombinant S. cerevisiae host strains using shake flasks.

The recombinant *S. cerevisiae* strains comprising a plasmid containing a heterologous gene encoding either the *Nocardia NRRL5646*, the *Mycobacteria sp. JLS,* or the *Streptomyces griseus* carboxylic acid reductase gene, were inoculated in 5 ml of YNB (Yeast Nitrogen Base) -Leu containing 2% glucose (SD media) and grown at 30°C for overnight (OD ∼3). Approximately 2.5 ml were subcultured into 50 ml (20x dilution, OD ∼0.15) of SD media and grown at 30°C for 8 hours to OD ∼ 1. Cell cultures were centrifuged at ∼3000-4000 rpm (F15B-8X50C rotor) for 10 minutes, the supernatant was discarded. The residual medium was removed with the pipette or the cells were washed with SG medium (YNB -Leu containing 2% galactose). The pellets were resuspended in 250 mL SG media (5x dilution, starting culture ∼ OD 0.2,) and grown overnight at 30°C before harvesting.

For extraction and identification of intra-cellular fatty alcohols, 30-50 OD₆₀₀ units of cells were centrifuged and pellets were washed with 20 ml of 50 mM Tris-HCl pH7.5. Cells were resuspended in 0.5 ml of 6.7% Na₂SO₄, and transferred into 2 ml tubes. 0.4 ml of isopropanol and 0.6 ml of hexane were added and the mixture was vortexed for ∼30 minutes and centrifuged for 2 minutes at 14,000 rpm using a bench top centrifuge (eppendrof F45-25-11). The upper organic phase was collected and evaporated under a nitrogen stream. The remaining residue was derivatized with 100 µL BSTFA at 37-60 C for 1 hour, left at room temperature for another 3 to 12 hours and diluted with 100 µL heptane before analysis by GC-FID or GC-MS.

For extraction and identification of extra-cellular fatty alcohols, 1 ml of 1:1 (vol:vol) chloroform:methanol was added to 0.5 ml of culture supernatant, vortexed for ∼30 min, and centrifuged for 2 minutes at 14,000 rpm using a bench top centrifuge (eppendrof F45-25-11). The upper phase was discarded and the ∼ 1 ml of the lower phase was transferred to a 2 ml autosampler vial. The extracts were dried under a nitrogen stream and the residue was derivatized with 100 ul BSTFA at 37-60°C for 1 hour and 3 to 12 hours at room temperature. The mixture was diluted with 100 µl heptane before analysis by GC-FID or GC-MS. GC conditions were similar to those provided in example 2b. Under the conditions tested, expression of both the *Nocardia* NRRL5646, the *Mycobacteria sp. JLS* carboxylic acid reductase in *S. Cerevisiae* YPH499 resulted in the intracellular production of long chain fatty alcohols (see Table 4). Secreted fatty alcohols were not detected.

**Table 4: Fatty Alcohol Profile Exhibited by Recombinant S. cerevisiae Cells Over-Expressing the Heterologous Enzyme Genes**

| | **Cellular fatty alcohol composition^{a}** | | | | | | **Estimated productivity** |
|---|---|---|---|---|---|---|---|
| **Enzyme** | **C12:0** | **C14:0** | **C16:0** | **C16:1** | **C18:0** | **C18:0** | (mg/g DCW) |
| *Nocardia* CAR | 12 | 10 | 33 | 38 | trace | 6 | 0.3 |
| *A. Mycobacterium* CAR | 10 | 10 | 38 | 27 | 7 | 8 | 0.4 |

| | | | | | | | |
|---|---|---|---|---|---|---|---|
| ^{a}The relative amounts of each fatty alcohol component are expressed as a percent of the total fatty alcohols detected using TMS derivative *via* GC/FID or GC/MS. Endogenous fatty alcohols include C12:0 (1-dodecanol), C14:0 (1-tetradecanol), C16:0(1-hexadecanol), C18:1 (*cis* Δ⁹-1-octadecenol), and 18:0 (1-octadecanol). DCW = dry cell weight. | | | | | | | |

### Example 4

### Expression and activity of CAR enzymes in Yarrowia lipolytica

An autonomous replicating plasmid for expression of genes in *Y. lipolytica* was engineered with two antibiotic selection marker cassettes for resistance to hygromycin and phleomycin (HygB(R) or Ble(R), respectively) (plasmid pCEN351, Figure 1). Expression of each cassette is independently regulated by a strong, constitutive promoter isolated from *Y*. *lipolytica:* pTEF1 for Ble(R) expression and pRPS7 for HygB(R) expression. Plasmid pCEN351 was used to assemble *Y. lipolytica* expression plasmids. Using "restriction free cloning" methodology, the *Mycobacterium* CAR in *Y. lipolytica* gene was inserted into pCEN351 to provided plasmid pCEN364 (Figure 2). In pCEN364, heterologous gene expression is under control of the constitutive TEF1 promoter. The HygB ^{R} gene allows for selection in media containing hygromycin. Ars18 is an autonomous replicating sequence isolated from *Y. lipolytica* genomic DNA. The resulting plasmid (pCEN364) was transformed by standard procedures into *Y. lipolytica* 1345 which was obtained from the German Resource Centre for Biological Material (DSMZ). (a) *In vivo* of CAR activity in recombinant *Y. lipolytica.*

The recombinant *Y. lipolytica* strains comprising plasmid containing a heterologous gene encoding either the *Nocardia* NRRL5646, the *Mycobacteria sp. JLS,* or the *Streptomyces griseus* carboxylic acid reductase, were inoculated in 200 mL YPD media containing 500 µg/mL hygromycin. The cultures were grown at 30°C to an OD600 of 4-7. Cells were then harvested by centrifugation and washed with 20 ml of 50 mM Tris-HCl pH7.5. Extraction and identification of intra-cellular fatty alcohols were performed as described in Example3b. Under the conditions tested trace amount of 1-hexadecanol and 1-octadecanol were detected by *Nocardia* CAR. Secreted fatty alcohols were not detected. (b) *In vitro* of CAR activity in recombinant *Y. lipolytica.*

The recombinant *Y. lipolytica* strains comprising a plasmid containing a heterologous gene encoding either the *Nocardia* NRRL5646, the *Mycobacteria sp. JLS,* or the *Streptomyces griseus* carboxylic acid reductase, were inoculated in 200 mL YPD media containing 500 µg/mL hygromycin. The cultures were grown at 30° C to an OD600 of 4-7. Cells were then harvested by centrifugation, washed and stored at -80° C. For lysis, cell pellets were resuspended in 15 mL of 100 mM sodium phosphate pH 7.0. The cell suspension was supplemented with protease inhibitor tablets (Calbiochem #539137), then placed into a stainless steel bead beater (15 mL capacity) loaded with glass beads. The bead beater was submerged into an ice bath, and cells were lysed using ten cycles of bead beating for 30 seconds followed by cooling for 90 seconds. The lysate was centrifuged at 15,000 rpm in JA25.50 rotor for 20 minutes. The total protein concentration was estimated to be 9-16 mg/ml. *E. coli* lysate containing the *Nocardia* PPTase was prepared as described in Examples land 2. An aliquot (4-6 mL) of the *Y. lipolytica* CAR lysate obtained above was pre-incubated for - 1 hr with 1.5 mL of the *E. coli Nocardia* PPTase lysate. 110 µL of this mixture was then added to the assay mixture comprising 100 mM phosphate buffer (pH 7.5), 0.2 mg/mL NADPH, 2 mM ATP, 1 mM CoA, 1mM hexadecanoic acid and 3% IPA and 2 mg/mL ketoreductase (SEQ ID NO. 77 in WO2008103248A1) to regenerate NADPH. After 4 hrs (for *Nocardia* CAR) and 19 hrs (for *Mycobacterium,* CAR) incubation at room temperature on bench top shaker, the reaction mixture was extracted by 600 µL MTBE containing methyl hexadecanoate as internal standard. A 1µL sample was analyzed by GC-MS or GC-FID with the conditions described above. Under the conditions tested approximately 90% conversion of hexadanoic acid to 1-hexadecanol was detected by both *Mycobacteriaum* and *Nocardia* CARs. PPTase was observed to improve CAR activity for both *Nocardia* CAR and *Mycobacterium,* CAR by 3-5 times and by 9 - 20 times respectively activity The inventors believe the conversion of the hexadecanyl aldehyde to the corresponding alcohol occurs by endogenous ketoreductase activity in *Y. lipolytica.*

## Claims

1. A process for the biologically-derived production of fatty alcohols which comprise at least 8 carbon atoms comprising:
(a) culturing a recombinant microorganism comprising a nucleic acid sequence encoding a heterologous carboxylic acid reductase and a gene encoding a phosphopantetheinyl transferase polypeptide (PPTase) capable of attaching a phosphopantetheine moiety to the carboxylic acid reductase, in an aqueous nutrient medium comprising an assimilable source of carbon under suitable culture conditions for a sufficient period of time to allow the production the fatty alcohols, wherein the recombinant microorganism is capable of producing at least 2 mg/L of fatty alcohols having C8 to C24 carbons in length, and wherein the PPTase is:
(i) heterologous to the recombinant microorganism; or
(ii) derived from the recombinant microorganism and overexpressed by genetic manipulation of the PPTase, and
(b) isolating the produced fatty alcohols.

2. The process of claim 1 wherein the recombinant microorganism is capable of producing fatty alcohols having C10 to C20 carbons in length.

3. The process of claim 1 or claim 2, wherein the carboxylic acid reductase is selected from the group consisting of a *Mycobacterium* carboxylic acid reductase, a *Nocardia* carboxylic acid reductase, and a *Streptomyces griseus* carboxylic acid reductase, optionally wherein the carboxylic acid reductase has:
(i) at least 90%, for example at least 95%, sequence identity to SEQ ID NO:4;
(ii) at least 90%, for example at least 95%, sequence identity to SEQ ID NO:2; or
(iii) at least 90%, for example at least 95%, sequence identity to SEQ ID NO:6.

4. The process of claim 3, wherein the carboxylic acid reductase variant comprises at least 90% sequence identity to SEQ ID NO:4 and an amino acid substitution at one or more of the following positions R270, A271, K274, A275, P467, Q584, E626, and/or D701 when aligned with SEQ ID NO:4.

5. The process of claim 4 wherein the CAR variant comprises at least 93%, at least 95%, at least 96%, at least 97%, at least 98% or at least 99% sequence identity to SEQ ID NO:4.

6. The process of claim 4 or claim 5 wherein the CAR variant comprises an amino acid substitution of R270W, A271W, K274(G/ N/V/I/W/L/M/Q/S), A275F, P467S, Q584R, E626G, D701G, K274L/A369T/L380Y, K274L/V358H/E845A, K274M/T282K, K274Q/T282Y, K274S/A715T, K274W/L380G/ A477T, K274W/T282E/L380V, K274W/T282Q, K274W/V358R and/or R43C/K2741 in SEQ ID NO:4.

7. The process of any preceding claim, wherein the recombinant microorganism is a bacterial strain, a filamentous fungal strain, a yeast strain or an algal strain.

8. The process of claim 7, wherein the recombinant microorganism is *Escherichia coli.*

9. The process of any preceding claim, wherein the amount of fatty alcohol produced is at least 5 mg/L.

10. The process according to any preceding claim, wherein the culturing is carried out at a temperature within the range of from about 10°C to about 80°C and for a period of from about 8 hours to about 240 hours.

11. The process according to any preceding claim, wherein the amount of biologically produced fatty alcohol is in the range of 2 mg/L to 200 g/L.

12. The process according to any preceding claim, wherein the production of fatty alcohols having C10 to C20 carbons in length comprises at least 80% of the total isolated fatty alcohols.

13. The process according to any preceding claim, further comprising reducing the fatty alcohols to corresponding alkanes.

14. A process according to any one of claims 10-13 which comprises:
(a) culturing a recombinant microorganism, which comprises
(i) a polynucleotide coding for a heterologous carboxylic acid reductase (CAR) comprising an amino acid sequence having at least 90% sequence identity to SEQ ID NOs:4, 2 or 6, and
(ii) a polynucleotide coding for a heterologous phosphopantetheinyl transferase (PPTase) having at least 80% sequence identity to SEQ ID NO:8, wherein said PPTase is capable of attaching a phosphopantetheine moiety to the CAR under suitable culture conditions to allow the expression of the CAR and PPTase and production of the fatty alcohols, and
(b) recovering the produced fatty alcohol.

15. The process according to claim 14, wherein:
(i) the recombinant microorganism is a bacterial strain, a yeast strain, a filamentous fungal strain or an algal strain; or
(ii) the CAR and the PPTase are derived from the same organism.

## Patentansprüche

1. Verfahren für die biologisch hergeleitete Produktion von Fettalkoholen, die mindestens 8 Kohlenstoffatome umfassen, das Folgendes umfasst:
(a) Kultivieren eines rekombinanten Mikroorganismus umfassend eine Nukleinsäure, die für eine heterologe Carbonsäurereduktase codiert, und ein Gen, das für ein Phosphopantetheinyltransferase-Polypeptid (PPTase), das fähig ist, einen Phosphopantetheinrest auf die Carbonsäurereduktase zu übertragen, codiert, in einem wässrigen Nährmedium umfassend eine assimilierbare Kohlenstoffquelle unter geeigneten Kulturbedingungen über einen Zeitraum, der ausreicht, um die Produktion der Fettalkohole zu gestatten, wobei der rekombinante Mikroorganismus fähig ist, mindestens 2 mg/l Fettalkohole mit einer Länge von C8 bis C24 Kohlenstoffen zu produzieren, und wobei die PPTase:
(i) zu dem rekombinanten Mikroorganismus heterolog ist; oder
(ii) von dem rekombinanten Mikroorganismus abstammt und durch genetische Manipulation der PPTase überexprimiert wird, und
(b) Isolieren der produzierten Fettalkohole.

2. Verfahren nach Anspruch 1, wobei der rekombinante Mikroorganismus fähig ist, Fettalkohole mit einer Länge von C10 bis C20 Kohlenstoffen zu produzieren.

3. Verfahren nach Anspruch 1 oder Anspruch 2, wobei die Carbonsäurereduktase aus der Gruppe bestehend aus einer *Mycobacterium*-Carbonsäurereduktase, einer *Nocardia*-Carbonsäurereduktase und einer *Streptomyces-griseus*-Carbonsäurereduktase ausgewählt ist, wobei die Carbonsäurereduktase gegebenenfalls
(i) mindestens 90%, zum Beispiel mindestens 95%, Sequenzidentität zu SEQ ID NO: 4 aufweist;
(ii) mindestens 90%, zum Beispiel mindestens 95%, Sequenzidentität zu SEQ ID NO: 2 aufweist; oder
(iii) mindestens 90%, zum Beispiel mindestens 95%, Sequenzidentität zu SEQ ID NO: 6 aufweist.

4. Verfahren nach Anspruch 3, wobei die Carbonsäure-reduktasevariante mindestens 90% Sequenzidentität zu SEQ ID NO: 4 und bei Durchführung eines Alignments mit SEQ ID NO: 4 eine Aminosäuresubstitution an einer oder mehreren der folgenden Positionen R270, A271, K274, A275, P467, Q584, E626 und/oder D701 umfasst.

5. Verfahren nach Anspruch 4, wobei die CAR-Variante mindestens 93%, mindestens 95%, mindestens 96%, mindestens 97%, mindestens 98% oder mindestens 99% Sequenzidentität zu SEQ ID NO: 4 umfasst.

6. Verfahren nach Anspruch 4 oder Anspruch 5, wobei die CAR-Variante eine Aminosäuresubstitution von R270W, A271W, K274(G/N/V/I/W/L/M/Q/S), A275F, P467S, Q584R, E626G, D701G, K274L/A369T/L380Y, K274L/V358H/E845A, K274M/T282K, K274Q/T282Y, K274S/A715T, K274W/L380G/A477I, K274W/T282E/L380V, K274W/T282Q, K274W/V358R und/oder R43C/K274I in SEQ ID NO: 4 umfasst.

7. Verfahren nach einem vorhergehenden Anspruch, wobei es sich bei dem rekombinanten Mikroorganismus um einen Bakterienstamm, einen Fadenpilzstamm, einen Hefestamm oder einen Algenstamm handelt.

8. Verfahren nach Anspruch 7, wobei es sich bei dem rekombinanten Mikroorganismus um *Escherichia coli* handelt.

9. Verfahren nach einem vorhergehenden Anspruch, wobei die Menge an produziertem Fettalkohol mindestens 5 mg/l beträgt.

10. Verfahren nach einem vorhergehenden Anspruch, wobei die Kultivierung bei einer Temperatur im Bereich von ungefähr 10°C bis ungefähr 80°C und über einen Zeitraum von ungefähr 8 Stunden bis ungefähr 240 Stunden erfolgt.

11. Verfahren nach einem vorhergehenden Anspruch, wobei die Menge an biologisch produziertem Fettalkohol im Bereich von 2 mg/l bis 200 g/l liegt.

12. Verfahren nach einem vorhergehenden Anspruch, wobei die Produktion an Fettalkoholen mit einer Länge von C10 bis C20 Kohlenstoffen mindestens 80% der insgesamt isolierten Fettalkohole umfasst.

13. Verfahren nach einem vorhergehenden Anspruch, das weiterhin das Reduzieren der Fettalkohole zu entsprechenden Alkanen umfasst.

14. Verfahren nach einem der Ansprüche 10-13, das Folgendes umfasst:
(a) Kultivieren eines rekombinanten Mikroorganismus, der
(i) ein Polynukleotid, das für eine heterologe Carbonsäurereduktase (CAR) umfassend eine Aminosäuresequenz mit mindestens 90% Sequenzidentität zu SEQ ID NO: 4, 2 oder 6 codiert, und
(ii) ein Polynukleotid, das für eine heterologe Phosphopantetheinyltransferase (PPTase) mit mindestens 80% Sequenzidentität zu SEQ ID NO: 8 codiert, wobei die PPTase fähig ist, unter geeigneten Kulturbedingungen einen Phosphopantetheinrest auf die CAR zu übertragen, um die Expression der CAR und der PPTase und die Produktion der Fettalkohole zu gestatten, umfasst, und
(b) Gewinnen des produzierten Fettalkohols.

15. Verfahren nach Anspruch 14, wobei
(i) es sich bei dem rekombinanten Mikroorganismus um einen Bakterienstamm, einen Hefestamm, einen Fadenpilzstamm oder einen Algenstamm handelt; oder
(ii) die CAR und die PPTase von demselben Organismus abstammen.

## Revendications

1. Procédé de production biologiquement dérivée d'alcools gras qui comprennent au moins 8 atomes de carbone comprenant :
(a) la culture d'un micro-organisme recombinant comprenant une séquence d'acide nucléique codant pour une acide carboxylique réductase hétérologue et un gène codant pour un polypeptide de phosphopantéthéinyle transférase (PPTase) capable de lier un fragment phosphopantéthéine à l'acide carboxylique réductase, dans un milieu nutritif aqueux comprenant une source de carbone assimilable dans des conditions de culture adaptées pendant une durée suffisante pour permettre la production des alcools gras, dans lequel le micro-organisme recombinant est capable de produire au moins 2 mg/l d'alcools gras ayant C8 à C24 carbones de longueur, et dans lequel la PPTase est :
(i) hétérologue au micro-organisme recombinant ; ou
(ii) dérivée du micro-organisme recombinant et surexprimée par manipulation génétique de la PPTase, et
(b) l'isolement des alcools gras produits.

2. Procédé de la revendication 1 dans lequel le micro-organisme recombinant est capable de produire des alcools gras ayant C10 à C20 carbones de longueur.

3. Procédé de la revendication 1 ou la revendication 2, dans lequel l'acide carboxylique réductase est choisie dans le groupe constitué d'une acide carboxylique réductase de *Mycobacterium*, une acide carboxylique réductase de *Nocardia*, et une acide carboxylique réductase de *Streptomyces griseus*, facultativement dans lequel l'acide carboxylique réductase a :
(i) au moins 90 %, par exemple au moins 95 %, d'identité de séquence avec SEQ ID NO: 4 ;
(ii) au moins 90 %, par exemple au moins 95 %, d'identité de séquence avec SEQ ID NO: 2 ; ou
(iii) au moins 90 %, par exemple au moins 95 %, d'identité de séquence avec SEQ ID NO: 6.

4. Procédé de la revendication 3, dans lequel le variant d'acide carboxylique réductase comprend au moins 90 % d'identité de séquence avec SEQ ID NO: 4 et une substitution d'acide aminé à une ou plusieurs des positions suivantes R270, A271, K274, A275, P467, Q584, E626, et/ou D701 lorsqu'il est aligné avec SEQ ID NO: 4.

5. Procédé de la revendication 4 dans lequel le variant de CAR comprend au moins 93 %, au moins 95 %, au moins 96 %, au moins 97 %, au moins 98 % ou au moins 99 % d'identité de séquence avec SEQ ID NO: 4.

6. Procédé de la revendication 4 ou la revendication 5 dans lequel le variant de CAR comprend une substitution d'acide aminé de R270W, A271W, K274 (G/ N/V/I/W/L/M/Q/S), A275F, P467S, Q584R, E626G, D701G, K274L/A369T/L380Y, K274L/V358H/E845A, K274M/T282K, K274Q/T282Y, K274S/A715T, K274W/L380G/A477T, K274W/T282E/L380V, K274W/T282Q, K274W/V358R et/ou R43C/K274I dans SEQ ID NO: 4.

7. Procédé de l'une quelconque des revendications précédentes, dans lequel le micro-organisme recombinant est une souche bactérienne, une souche fongique filamenteuse, une souche de levure ou une souche d'algue.

8. Procédé de la revendication 7, dans lequel le micro-organisme recombinant est *Escherichia coli.*

9. Procédé de l'une quelconque des revendications précédentes, dans lequel la quantité d'alcool gras produit est au moins 5 mg/l.

10. Procédé selon l'une quelconque des revendications précédentes, dans lequel la culture est conduite à une température dans la plage d'environ 10 °C à environ 80 °C et pendant une durée d'environ 8 heures à environ 240 heures.

11. Procédé selon l'une quelconque des revendications précédentes, dans lequel la quantité d'alcool gras biologiquement produit est dans la plage de 2 mg/l à 200 g/l.

12. Procédé selon l'une quelconque des revendications précédentes, dans lequel la production d'alcools gras ayant C10 à C20 carbones de longueur comprend au moins 80 % des alcools gras isolés totaux.

13. Procédé selon l'une quelconque des revendications précédentes, comprenant en outre la réduction des alcools gras en alcanes correspondants.

14. Procédé selon l'une quelconque des revendications 10 à 13 qui comprend :
(a) la culture d'un micro-organisme recombinant, qui comprend
(i) un polynucléotide codant pour une acide carboxylique réductase hétérologue (CAR) comprenant une séquence d'acides aminés ayant au moins 90 % d'identité de séquence avec SEQ ID NO: 4, 2 ou 6, et
(ii) un polynucléotide codant pour une phosphopantéthéinyle transférase (PPTase) hétérologue ayant au moins 80 % d'identité de séquence avec SEQ ID NO: 8, ladite PPTase étant capable de lier un fragment phosphopantéthéine au CAR dans des conditions de culture adaptées pour permettre l'expression des CAR et PPTase et la production des alcools gras, et
(b) la récupération de l'alcool gras produit.

15. Procédé selon la revendication 14, dans lequel :
(i) le micro-organisme recombinant est une souche bactérienne, une souche de levure, une souche fongique filamenteuse ou une souche d'algue ; ou
(ii) la CAR et la PPTase sont dérivées du même organisme.


### SEQUENCE LISTING

<110> Codexis, Inc. Behrouzian, Behnaz McDaniel, Robert Zhang, Xiyun Clark, Louis
<120> Engineered Biosynthesis of Fatty Alcohols
<130> CX3-005WO1
<150> US 61/180,534
   <151> 2009-05-22
<160> 11
<170> PatentIn version 3.3
<210> 1
   <211> 3595
   <212> DNA
   <213> Artificial
<220>
   <223> Synthetic DNA sequence including optimized codon sequence coding for Nocardia NRRL5646 CAR peptide.
<400> 1
   acaatctaga ggccagcctg gccataagga gatatacata tggccgttga ctcccctgac      60
   gaacgtcttc agcgtcgtat tgcacaactg tttgccgagg atgaacaagt caaagccgca     120
   cgtcctcttg aggcagtctc cgccgctgtc tccgcaccag gtatgcgtct tgcccaaatc     180
   gcagcaactg tcatggccgg ttatgcagac cgtcctgctg ccggtcaacg tgcattcgaa     240
   cttaatactg acgacgctac gggccgtact tctcttcgtt tgttgcctcg tttcgagact     300
   atcacctacc gtgagctgtg gcagcgtgtg ggtgaagttg ctgccgcatg gcatcatgac     360
   ccggagaatc ctctgcgtgc gggcgacttc gttgcgcttt tgggctttac cagcattgat     420
   tacgctaccc tggaccttgc cgatattcac ttgggagcgg tgacagtccc ccttcaagca     480
   tctgccgccg tcagccaatt gatcgctatt ttgacggaga ccagcccgcg tcttctggcc     540
   agcacaccag aacatctgga tgccgcggtc gaatgtcttc tggccggtac aacccctgag     600
   cgtttggttg tttttgacta ccatccagag gatgacgacc agcgtgccgc tttcgagtcc     660
   gcgcgtcgtc gtttggccga cgccggttcc ctggtcatcg ttgagaccct tgatgccgtg     720
   cgtgctcgtg gccgtgactt gcctgccgct ccattgtttg tgcccgacac ggatgatgat     780
   cctttggcgc tgcttattta tactagcggt agcaccggta cacctaaggg tgcgatgtac     840
   accaaccgtc ttgcggccac gatgtggcag ggaaattcca tgttgcaggg aaactctcaa     900
   cgtgtcggta ttaacttgaa ctacatgcca atgagccata ttgccggtcg tatttccctg     960
   ttcggtgtgc ttgcccgtgg aggcactgct tactttgccg cgaaatccga tatgtccacg    1020
   ttgtttgagg acattggctt ggttcgtccg acggaaattt tctttgtccc tcgtgtctgt    1080
   gatatggttt tccagcgtta tcagtctgag ctggatcgtc gttctgtcgc cggtgcagat 1140
   ttggatacgc tggaccgtga agtgaaggcg gacttgcgtc agaactacct gggtggtcgt 1200
   ttcttggttg ccgtggtggg cagcgctccc cttgccgcag aaatgaagac ctttatggag 1260
   tctgtccttg atctgccctt gcacgacggt tacggttcca cggaagcagg cgcttccgtt 1320
   ctgctggata accaaattca acgtccaccc gtgctggatt acaagctggt ggatgttcct 1380
   gagctgggat actttcgtac tgatcgtcct cacccacgtg gtgaactgtt gctgaaggcc 1440
   gaaacgacga ttcctggcta ctacaagcgt cctgaggtca ccgcggaaat tttcgatgag 1500
   gacggttttt ataaaacagg cgacattgtc gccgaattgg agcatgatcg tcttgtctat 1560
   gttgaccgtc gtaacaacgt cttgaagttg tcccagggtg agttcgttac agttgcgcac 1620
   ttggaagcag tctttgcctc ctctcctctt atccgtcaaa ttttcatcta cggctcctct 1680
   gagcgttcct atcttcttgc tgttattgtc ccaactgacg atgcgctgcg tggacgtgac 1740
   accgccacgc tgaaatccgc actggcggaa tccatccaac gtatcgcaaa ggacgccaat 1800
   ctgcaaccct acgaaattcc ccgtgatttt ctgatcgaaa ccgagccgtt tacaattgct 1860
   aacggtcttt tgtctggtat tgccaaactg cttcgtccca acctgaagga gcgttacggc 1920
   gctcaacttg agcaaatgta cacggacctg gccacaggcc aagccgatga gttgcttgcc 1980
   ctgcgtcgtg aagccgctga tttgcctgtg cttgaaacag tgagccgtgc ggccaaagcc 2040
   atgttgggtg tcgcatctgc ggacatgcgt cctgacgccc acttcaccga tttgggcggt 2100
   gactccctta gcgccttgtc cttttccaat cttcttcatg aaatttttgg cgttgaagtt 2160
   ccggtcggag tggtcgtgag cccggcaaac gagttgcgtg acttggctaa ctatattgaa 2220
   gccgaacgta actctggtgc caagcgtcct acttttactt ctgttcacgg tggaggttcc 2280
   gaaattcgtg ccgccgattt gactttggac aagtttatcg acgcccgtac gttggccgca 2340
   gccgacagca tcccccatgc tccagtcccg gcccaaaccg ttcttctgac cggtgccaac 2400
   ggttacctgg gtcgtttcct gtgtcttgag tggcttgagc gtctggataa aaccggcgga 2460
   acactgattt gcgttgttcg tggttccgac gccgctgccg cccgtaaacg tttggatagc 2520
   gcgttcgact ccggtgatcc tggtcttctg gagcactacc aacagttggc tgcccgtacg 2580
   ctggaggttt tggctggaga catcggtgat ccgaatttgg gccttgatga tgctacgtgg 2640
   caacgtctgg cggaaacggt ggatttgatc gttcatcctg ccgctttggt caaccatgtt 2700
   ttgccatata cccagctgtt tggcccaaac gttgttggta ctgctgaaat cgttcgtctt 2760
   gcgattaccg ctcgtcgtaa gccagtcacc tacttgtcca ccgtgggtgt cgcggaccaa 2820
   gttgatcccg ccgagtacca agaggattcc gatgttcgtg agatgtccgc tgttcgtgtt 2880
   gtccgtgagt cctatgccaa cggttacgga aactctaaat gggccggtga ggtgctgctg 2940
   cgtgaagcac acgatctttg tggtctgcca gttgcggtgt tccgttccga catgatcttg 3000
   gcccattctc gttatgctgg ccagcttaat gtccaagatg ttttcactcg tctgatcctg 3060
   tccctggtgg ctactggtat cgccccttat tccttctatc gtactgacgc tgatggtaac   3120
   cgtcagcgtg cacattacga tggtttgcca gctgacttca cggcggctgc tatcaccgct   3180
   ttgggtattc aggccacaga aggattccgt acgtacgatg tgttgaatcc ttacgatgat   3240
   ggtatcagcc tggacgagtt cgtggactgg cttgtggaat ccggacatcc gatccaacgt   3300
   atcacggatt actccgattg gtttcatcgt ttcgagactg cgattcgtgc acttcctgaa   3360
   aagcaacgtc aggcctccgt gttgccgctt ttggacgctt accgtaaccc ttgcccagcg   3420
   gtgcgtggcg ccattctgcc tgccaaagag tttcaggctg cggttcaaac cgcgaagatt   3480
   ggcccagaac aggacattcc tcacctttct gctcccctga ttgacaagta cgtgtctgat   3540
   ctggagcttt tgcaattgtt gtaatgaggc caaactggcc accatcacca tcacc         3595
<210> 2
   <211> 1174
   <212> PRT
   <213> Artificial
<220>
   <223> Synthetic peptide sequence from optimized codon sequence coding for Nocardia NRRL5646 CAR peptide.
<400> 2
   Met Ala Val Asp Ser Pro Asp Glu Arg Leu Gln Arg Arg Ile Ala Gln
   1               5                   10                  15
   Leu Phe Ala Glu Asp Glu Gln Val Lys Ala Ala Arg Pro Leu Glu Ala
               20                  25                  30
   Val Ser Ala Ala Val Ser Ala Pro Gly Met Arg Leu Ala Gln Ile Ala
           35                  40                  45
   Ala Thr Val Met Ala Gly Tyr Ala Asp Arg Pro Ala Ala Gly Gln Arg
       50                  55                  60
   Ala Phe Glu Leu Asn Thr Asp Asp Ala Thr Gly Arg Thr Ser Leu Arg
   65                  70                  75                  80
   Leu Leu Pro Arg Phe Glu Thr Ile Thr Tyr Arg Glu Leu Trp Gln Arg
                   85                  90                  95
   Val Gly Glu Val Ala Ala Ala Trp His His Asp Pro Glu Asn Pro Leu
               100                 105                 110
   Arg Ala Gly Asp Phe Val Ala Leu Leu Gly Phe Thr Ser Ile Asp Tyr
           115                 120                 125
   Ala Thr Leu Asp Leu Ala Asp Ile His Leu Gly Ala Val Thr Val Pro
       130                 135                 140
   Leu Gln Ala Ser Ala Ala Val Ser Gln Leu Ile Ala Ile Leu Thr Glu
   145 150 155 160
   Thr Ser Pro Arg Leu Leu Ala Ser Thr Pro Glu His Leu Asp Ala Ala
   165 170 175
   Val Glu Cys Leu Leu Ala Gly Thr Thr Pro Glu Arg Leu Val Val Phe
   180 185 190
   Asp Tyr His Pro Glu Asp Asp Asp Gln Arg Ala Ala Phe Glu Ser Ala
   195 200 205
   Arg Arg Arg Leu Ala Asp Ala Gly Ser Leu Val Ile Val Glu Thr Leu
   210 215 220
   Asp Ala Val Arg Ala Arg Gly Arg Asp Leu Pro Ala Ala Pro Leu Phe
   225 230 235 240
   Val Pro Asp Thr Asp Asp Asp Pro Leu Ala Leu Leu Ile Tyr Thr Ser
   245 250 255
   Gly Ser Thr Gly Thr Pro Lys Gly Ala Met Tyr Thr Asn Arg Leu Ala
   260 265 270
   Ala Thr Met Trp Gln Gly Asn Ser Met Leu Gln Gly Asn Ser Gln Arg
   275 280 285
   Val Gly Ile Asn Leu Asn Tyr Met Pro Met Ser His Ile Ala Gly Arg
   290 295 300
   Ile Ser Leu Phe Gly Val Leu Ala Arg Gly Gly Thr Ala Tyr Phe Ala
   305 310 315 320
   Ala Lys Ser Asp Met Ser Thr Leu Phe Glu Asp Ile Gly Leu Val Arg
   325 330 335
   Pro Thr Glu Ile Phe Phe Val Pro Arg Val Cys Asp Met Val Phe Gln
   340 345 350
   Arg Tyr Gln Ser Glu Leu Asp Arg Arg Ser Val Ala Gly Ala Asp Leu
   355 360 365
   Asp Thr Leu Asp Arg Glu Val Lys Ala Asp Leu Arg Gln Asn Tyr Leu
   370 375 380
   Gly Gly Arg Phe Leu Val Ala Val Val Gly Ser Ala Pro Leu Ala Ala
   385 390 395 400
   Glu Met Lys Thr Phe Met Glu Ser Val Leu Asp Leu Pro Leu His Asp
   405 410 415
   Gly Tyr Gly Ser Thr Glu Ala Gly Ala Ser Val Leu Leu Asp Asn Gln
   420 425 430
   Ile Gln Arg Pro Pro Val Leu Asp Tyr Lys Leu Val Asp Val Pro Glu
   435 440 445
   Leu Gly Tyr Phe Arg Thr Asp Arg Pro His Pro Arg Gly Glu Leu Leu
   450 455 460
   Leu Lys Ala Glu Thr Thr Ile Pro Gly Tyr Tyr Lys Arg Pro Glu Val
   465 470 475 480
   Thr Ala Glu Ile Phe Asp Glu Asp Gly Phe Tyr Lys Thr Gly Asp Ile
   485 490 495
   Val Ala Glu Leu Glu His Asp Arg Leu Val Tyr Val Asp Arg Arg Asn
   500 505 510
   Asn Val Leu Lys Leu Ser Gln Gly Glu Phe Val Thr Val Ala His Leu
   515 520 525
   Glu Ala Val Phe Ala Ser Ser Pro Leu Ile Arg Gln Ile Phe Ile Tyr
   530 535 540
   Gly Ser Ser Glu Arg Ser Tyr Leu Leu Ala Val Ile Val Pro Thr Asp
   545 550 555 560
   Asp Ala Leu Arg Gly Arg Asp Thr Ala Thr Leu Lys Ser Ala Leu Ala
   565 570 575
   Glu Ser Ile Gln Arg Ile Ala Lys Asp Ala Asn Leu Gln Pro Tyr Glu
   580 585 590
   Ile Pro Arg Asp Phe Leu Ile Glu Thr Glu Pro Phe Thr Ile Ala Asn
   595 600 605
   Gly Leu Leu Ser Gly Ile Ala Lys Leu Leu Arg Pro Asn Leu Lys Glu
   610 615 620
   Arg Tyr Gly Ala Gln Leu Glu Gln Met Tyr Thr Asp Leu Ala Thr Gly
   625 630 635 640
   Gln Ala Asp Glu Leu Leu Ala Leu Arg Arg Glu Ala Ala Asp Leu Pro
   645 650 655
   Val Leu Glu Thr Val Ser Arg Ala Ala Lys Ala Met Leu Gly Val Ala
   660 665 670
   Ser Ala Asp Met Arg Pro Asp Ala His Phe Thr Asp Leu Gly Gly Asp
   675 680 685
   Ser Leu Ser Ala Leu Ser Phe Ser Asn Leu Leu His Glu Ile Phe Gly
   690 695 700
   Val Glu Val Pro Val Gly Val Val Val Ser Pro Ala Asn Glu Leu Arg
   705 710 715 720
   Asp Leu Ala Asn Tyr Ile Glu Ala Glu Arg Asn Ser Gly Ala Lys Arg
   725 730 735
   Pro Thr Phe Thr Ser Val His Gly Gly Gly Ser Glu Ile Arg Ala Ala
   740 745 750
   Asp Leu Thr Leu Asp Lys Phe Ile Asp Ala Arg Thr Leu Ala Ala Ala
   755 760 765
   Asp Ser Ile Pro His Ala Pro Val Pro Ala Gln Thr Val Leu Leu Thr
   770 775 780
   Gly Ala Asn Gly Tyr Leu Gly Arg Phe Leu Cys Leu Glu Trp Leu Glu
   785 790 795 800
   Arg Leu Asp Lys Thr Gly Gly Thr Leu Ile Cys Val Val Arg Gly Ser
   805 810 815
   Asp Ala Ala Ala Ala Arg Lys Arg Leu Asp Ser Ala Phe Asp Ser Gly
   820 825 830
   Asp Pro Gly Leu Leu Glu His Tyr Gln Gln Leu Ala Ala Arg Thr Leu
   835 840 845
   Glu Val Leu Ala Gly Asp Ile Gly Asp Pro Asn Leu Gly Leu Asp Asp
   850 855 860
   Ala Thr Trp Gln Arg Leu Ala Glu Thr Val Asp Leu Ile Val His Pro
   865 870 875 880
   Ala Ala Leu Val Asn His Val Leu Pro Tyr Thr Gln Leu Phe Gly Pro
   885 890 895
   Asn Val Val Gly Thr Ala Glu Ile Val Arg Leu Ala Ile Thr Ala Arg
   900 905 910
   Arg Lys Pro Val Thr Tyr Leu Ser Thr Val Gly Val Ala Asp Gln Val
   915 920 925
   Asp Pro Ala Glu Tyr Gln Glu Asp Ser Asp Val Arg Glu Met Ser Ala
   930 935 940
   Val Arg Val Val Arg Glu Ser Tyr Ala Asn Gly Tyr Gly Asn Ser Lys
   945 950 955 960
   Trp Ala Gly Glu Val Leu Leu Arg Glu Ala His Asp Leu Cys Gly Leu
   965 970 975
   Pro Val Ala Val Phe Arg Ser Asp Met Ile Leu Ala His Ser Arg Tyr
   980 985 990
   Ala Gly Gln Leu Asn Val Gln Asp Val Phe Thr Arg Leu Ile Leu Ser
   995 1000 1005
   Leu Val Ala Thr Gly Ile Ala Pro Tyr Ser Phe Tyr Arg Thr Asp
   1010 1015 1020
   Ala Asp Gly Asn Arg Gln Arg Ala His Tyr Asp Gly Leu Pro Ala
   1025 1030 1035
   Asp Phe Thr Ala Ala Ala Ile Thr Ala Leu Gly Ile Gln Ala Thr
   1040 1045 1050
   Glu Gly Phe Arg Thr Tyr Asp Val Leu Asn Pro Tyr Asp Asp Gly
   1055 1060 1065
   Ile Ser Leu Asp Glu Phe Val Asp Trp Leu Val Glu Ser Gly His
   1070 1075 1080
   Pro Ile Gln Arg Ile Thr Asp Tyr Ser Asp Trp Phe His Arg Phe
   1085 1090 1095
   Glu Thr Ala Ile Arg Ala Leu Pro Glu Lys Gln Arg Gln Ala Ser
   1100 1105 1110
   Val Leu Pro Leu Leu Asp Ala Tyr Arg Asn Pro Cys Pro Ala Val
   1115 1120 1125
   Arg Gly Ala Ile Leu Pro Ala Lys Glu Phe Gln Ala Ala Val Gln
   1130 1135 1140
   Thr Ala Lys Ile Gly Pro Glu Gln Asp Ile Pro His Leu Ser Ala
   1145 1150 1155
   Pro Leu Ile Asp Lys Tyr Val Ser Asp Leu Glu Leu Leu Gln Leu
   1160 1165 1170
   Leu
<210> 3
   <211> 3525
   <212> DNA
   <213> Artificial
<220>
   <223> Synthetic DNA sequence including optimized codon sequence coding for Mycobacterium sp. (strain JLS) CAR peptide.
<400> 3
   atgtccactg agacccgtga agcccgtttg cagcaacgta ttgctcactt gtttgccacc     60
   gacccccaat ttgccgccgc ccgtcccgac cctcgtattt ctgacgccgt tgatcgtgac    120
   gacgcacgtt tgaccgccat tgtgtctgct gtgatgtctg gctatgcaga tcgtcctgct    180
   cttggtcaac gtgcagcaga gttcgctact gacccccaga ctggtcgtac tacgatggaa    240
   ctgttgcctc gttttgacac gattacctac cgtgagttgc tggatcgtgt gcgtgccctt    300
   accaacgcct ggcatgctga cggtgttcgt cctggagacc gtgttgcgat tttgggcttt    360
   accggtattg attacactgt tgttgacctt gccttgattc agttgggtgc agtcgcagtc    420
   ccattgcaaa ccagcgccgc cgttgaagcc cttcgtccca ttgttgctga aacagaaccc    480
   atgttgattg ccaccggagt tgatcatgtt gatgccgccg ccgagcttgc tcttacaggt    540
   caccgtccga gccaggttgt tgtgtttgac catcgtgaac aagttgatga cgaacgtgac    600
   gctgtgcgtg ccgctaccgc acgtttgggt gacgcagttc cggtggagac tttggcagaa    660
   gtcttgcgtc gtggtgccca tctgcccgct gtcgcacccc acgtctttga cgaggccgat    720
   cctttgcgtt tgctgattta cacctctggc tctaccggcg ctccgaaggg tgcgatgtac    780
   ccagagagca aagtcgcagg catgtggcgt gcaagcgcaa aggctgcctg gaacaatgat    840
   cagacggcaa ttccgtctat cacgcttaac ttcttgccga tgtctcacgt catgggccgt    900
   ggattgctgt gtggtactct ttctactggc ggtactgcgt attttgccgc acgttccgac    960
   ctgtccacac tgcttgagga cttgcgtctt gtccgtccca cccaattgtc cttcgttccg   1020
   cgtatttggg acatgctttt tcaggagttt gtcggtgaag tcgatcgtcg tgttaacgac   1080
   ggtgcggacc gtcccactgc tgaggctgat gtcttggccg agttgcgtca ggaactgttg   1140
   ggaggtcgtt ttgtcaccgc gatgaccggt agcgccccta tttcccctga gatgaaaacg   1200
   tgggtggaaa cgcttcttga catgcacctt gttgaaggct atggttctac ggaggctggc   1260
   gctgtgttcg tggacggtca cattcaacgt cccccggttt tggattacaa acttgttgac   1320
   gttccagacc tgggttactt tagcacagac cgtccacatc cgcgtggtga gctgcttgtt   1380
   cgttctacgc agttgtttcc aggatactac aaacgtccag acgtgaccgc cgaggttttc   1440
   gatgatgatg gcttctaccg tactggagat attgtggctg aattgggtcc tgaccagttg   1500
   cagtacctgg accgtcgtaa taatgtcctg aaattggcgc agggcgagtt cgtcactatc 1560
   agcaaactgg aagctgtgtt tgccggttcc gccctggtcc gtcagatctt tgtttatggc 1620
   aactccgccc gttcttactt gttggccgtc gttgttccca ctgatgacgc cgttgcacgt 1680
   cacgatcctg cctccctgaa gacagctatt agcgcttctt tgcagcaggc tgcgaaaaca 1740
   gcaggcttgc agagctatga attgcctcgt gactttttgg tggagacaca accttttaca 1800
   ctggaaaacg gactgctgac gggtattcgt aagttggcac gtcctaagtt gaaagcgcgt 1860
   tacggtgacc gtctggaggc cttgtatgtt gagcttgcag aaggccaggc aggtgaactg 1920
   cgtactctgc gtcgtgatgg tgcaaaacgt cctgtcgccg agacagttgg ccgtgccgcc 1980
   gccgccttgt tgggtgcagc tgcggcggat gtgcgtccag atgcccattt caccgatctt 2040
   ggcggcgact ctctgtccgc cctgactttt ggtaatttgt tgcaggaaat cttcggtgtt 2100
   gacgttcccg tcggcgtcat tgtctccccc gctgctgact tggcctccat cgctgcgtat 2160
   attgaaacag agcaggcttc cacgggtaaa cgtccaactt atgcctccgt tcatggtcgt 2220
   gatgctgagc aagtccgtgc ccgtgacttg acccttgata aattcatcga cgcagagacg 2280
   ttgtctgcgg cgacagagtt gccagtgcca atcggtgaag tgcgtaccgt gctgcttaca 2340
   ggcgctactg gctttctggg tcgttacctg gccctggatt ggctggaacg tatggcactg 2400
   gttgatggca aagtgatctg cttggtccgt gcaaaagacg acgcagctgc gcgtaagcgt 2460
   ctggatgaca ccttcgattc cggtgaccct aaattgttgg ctcattaccg taagttggcc 2520
   gctgatcacc tggaggtctt ggcgggcgac aagggcgaag ccgatttggg tctgccacac 2580
   caggtgtggc aacgtttggc tgacaccgtc gatcttatcg tggaccccgc tgcgttggtc 2640
   aatcatgtgc tgccgtactc tcaacttttc ggacccaacg ccctgggaac ggcagagttg 2700
   atccgtcttg ccttgacgac ccgtatcaaa cctttcacct acgtttccac cattggtgtt 2760
   ggcgcgggta ttgagccggg tcgtttcaca gaagacgacg acattcgtgt tattagccct 2820
   actcgtgccg ttgatacggg ttacgctaac ggatatggta actctaagtg ggcaggtgag 2880
   gttcttcttc gtgaggccca cgatctttgt ggtctgccag ttgcagtttt tcgttgtgat 2940
   atgattctgg ccgatacaac gtatgccggt caactgaacc tgccagatat gtttacccgt 3000
   atgatggtct ctttggtgac aaccggcatt gccccgaagt cttttcatcc acttgatgcg 3060
   aagggccacc gtcagcgtgc ccattatgac ggtttgccag tggaatttgt cgctgaaagc 3120
   atctctgccc tgggtgccca ggctgtggac gaggctggca ctggtttcgc cacataccat 3180
   gttatgaacc ctcatgatga cggcattggc cttgatgaat ttgttgattg gttggttgaa 3240
   gcgggttatc gtatcgaccg tattgacgac tatgcagcct ggcttcaacg ttttgaaacc 3300
   gctctgcgtg cactgcctga gcgtacacgt cagtactcct tgctgccgtt gcttcataat 3360
   taccagcgtc ccgctcatcc aatcaacggt gctatggccc ccacggaccg tttccgtgcg 3420
   gcagttcagg aggctaagtt gggtcctgac aaagacattc cgcatgttac tcctggtgtc 3480
   atcgttaagt acgccacaga tttggaattg cttggcctga tttaa 3525
<210> 4
   <211> 1174
   <212> PRT
   <213> Artificial
<220>
   <223> Synthetic peptide sequence from optimized codon sequence coding for Mycobacterium sp. (strain JLS) CAR peptide.
<400> 4
   Met Ser Thr Glu Thr Arg Glu Ala Arg Leu Gln Gln Arg Ile Ala His
   1               5                   10                  15
   Leu Phe Ala Thr Asp Pro Gln Phe Ala Ala Ala Arg Pro Asp Pro Arg
               20                  25                  30
   Ile Ser Asp Ala Val Asp Arg Asp Asp Ala Arg Leu Thr Ala Ile Val
           35                  40                  45
   Ser Ala Val Met Ser Gly Tyr Ala Asp Arg Pro Ala Leu Gly Gln Arg
       50                  55                  60
   Ala Ala Glu Phe Ala Thr Asp Pro Gln Thr Gly Arg Thr Thr Met Glu
   65                  70                  75                  80
   Leu Leu Pro Arg Phe Asp Thr Ile Thr Tyr Arg Glu Leu Leu Asp Arg
                   85                  90                  95
   Val Arg Ala Leu Thr Asn Ala Trp His Ala Asp Gly Val Arg Pro Gly
               100                 105                 110
   Asp Arg Val Ala Ile Leu Gly Phe Thr Gly Ile Asp Tyr Thr Val Val
           115                 120                 125
   Asp Leu Ala Leu Ile Gln Leu Gly Ala Val Ala Val Pro Leu Gln Thr
       130                 135                 140
   Ser Ala Ala Val Glu Ala Leu Arg Pro Ile Val Ala Glu Thr Glu Pro
   145                 150                 155                 160
   Met Leu Ile Ala Thr Gly Val Asp His Val Asp Ala Ala Ala Glu Leu
                   165                 170                 175
   Ala Leu Thr Gly His Arg Pro Ser Gln Val Val Val Phe Asp His Arg
               180                 185                 190
   Glu Gln Val Asp Asp Glu Arg Asp Ala Val Arg Ala Ala Thr Ala Arg
   195 200 205
   Leu Gly Asp Ala Val Pro Val Glu Thr Leu Ala Glu Val Leu Arg Arg
   210 215 220
   Gly Ala His Leu Pro Ala Val Ala Pro His Val Phe Asp Glu Ala Asp
   225 230 235 240
   Pro Leu Arg Leu Leu Ile Tyr Thr Ser Gly Ser Thr Gly Ala Pro Lys
   245 250 255
   Gly Ala Met Tyr Pro Glu Ser Lys Val Ala Gly Met Trp Arg Ala Ser
   260 265 270
   Ala Lys Ala Ala Trp Asn Asn Asp Gln Thr Ala Ile Pro Ser Ile Thr
   275 280 285
   Leu Asn Phe Leu Pro Met Ser His Val Met Gly Arg Gly Leu Leu Cys
   290 295 300
   Gly Thr Leu Ser Thr Gly Gly Thr Ala Tyr Phe Ala Ala Arg Ser Asp
   305 310 315 320
   Leu Ser Thr Leu Leu Glu Asp Leu Arg Leu Val Arg Pro Thr Gln Leu
   325 330 335
   Ser Phe Val Pro Arg Ile Trp Asp Met Leu Phe Gln Glu Phe Val Gly
   340 345 350
   Glu Val Asp Arg Arg Val Asn Asp Gly Ala Asp Arg Pro Thr Ala Glu
   355 360 365
   Ala Asp Val Leu Ala Glu Leu Arg Gln Glu Leu Leu Gly Gly Arg Phe
   370 375 380
   Val Thr Ala Met Thr Gly Ser Ala Pro Ile Ser Pro Glu Met Lys Thr
   385 390 395 400
   Trp Val Glu Thr Leu Leu Asp Met His Leu Val Glu Gly Tyr Gly Ser
   405 410 415
   Thr Glu Ala Gly Ala Val Phe Val Asp Gly His Ile Gln Arg Pro Pro
   420 425 430
   Val Leu Asp Tyr Lys Leu Val Asp Val Pro Asp Leu Gly Tyr Phe Ser
   435 440 445
   Thr Asp Arg Pro His Pro Arg Gly Glu Leu Leu Val Arg Ser Thr Gln
   450 455 460
   Leu Phe Pro Gly Tyr Tyr Lys Arg Pro Asp Val Thr Ala Glu Val Phe
   465 470 475 480
   Asp Asp Asp Gly Phe Tyr Arg Thr Gly Asp Ile Val Ala Glu Leu Gly
   485 490 495
   Pro Asp Gln Leu Gln Tyr Leu Asp Arg Arg Asn Asn Val Leu Lys Leu
   500 505 510
   Ala Gln Gly Glu Phe Val Thr Ile Ser Lys Leu Glu Ala Val Phe Ala
   515 520 525
   Gly Ser Ala Leu Val Arg Gln Ile Phe Val Tyr Gly Asn Ser Ala Arg
   530 535 540
   Ser Tyr Leu Leu Ala Val Val Val Pro Thr Asp Asp Ala Val Ala Arg
   545 550 555 560
   His Asp Pro Ala Ser Leu Lys Thr Ala Ile Ser Ala Ser Leu Gln Gln
   565 570 575
   Ala Ala Lys Thr Ala Gly Leu Gln Ser Tyr Glu Leu Pro Arg Asp Phe
   580 585 590
   Leu Val Glu Thr Gln Pro Phe Thr Leu Glu Asn Gly Leu Leu Thr Gly
   595 600 605
   Ile Arg Lys Leu Ala Arg Pro Lys Leu Lys Ala Arg Tyr Gly Asp Arg
   610 615 620
   Leu Glu Ala Leu Tyr Val Glu Leu Ala Glu Gly Gln Ala Gly Glu Leu
   625 630 635 640
   Arg Thr Leu Arg Arg Asp Gly Ala Lys Arg Pro Val Ala Glu Thr Val
   645 650 655
   Gly Arg Ala Ala Ala Ala Leu Leu Gly Ala Ala Ala Ala Asp Val Arg
   660 665 670
   Pro Asp Ala His Phe Thr Asp Leu Gly Gly Asp Ser Leu Ser Ala Leu
   675 680 685
   Thr Phe Gly Asn Leu Leu Gln Glu Ile Phe Gly Val Asp Val Pro Val
   690 695 700
   Gly Val Ile Val Ser Pro Ala Ala Asp Leu Ala Ser Ile Ala Ala Tyr
   705 710 715 720
   Ile Glu Thr Glu Gln Ala Ser Thr Gly Lys Arg Pro Thr Tyr Ala Ser
   725 730 735
   Val His Gly Arg Asp Ala Glu Gln Val Arg Ala Arg Asp Leu Thr Leu
   740 745 750
   Asp Lys Phe Ile Asp Ala Glu Thr Leu Ser Ala Ala Thr Glu Leu Pro
   755 760 765
   Val Pro Ile Gly Glu Val Arg Thr Val Leu Leu Thr Gly Ala Thr Gly
   770 775 780
   Phe Leu Gly Arg Tyr Leu Ala Leu Asp Trp Leu Glu Arg Met Ala Leu
   785 790 795 800
   Val Asp Gly Lys Val Ile Cys Leu Val Arg Ala Lys Asp Asp Ala Ala
   805 810 815
   Ala Arg Lys Arg Leu Asp Asp Thr Phe Asp Ser Gly Asp Pro Lys Leu
   820 825 830
   Leu Ala His Tyr Arg Lys Leu Ala Ala Asp His Leu Glu Val Leu Ala
   835 840 845
   Gly Asp Lys Gly Glu Ala Asp Leu Gly Leu Pro His Gln Val Trp Gln
   850 855 860
   Arg Leu Ala Asp Thr Val Asp Leu Ile Val Asp Pro Ala Ala Leu Val
   865 870 875 880
   Asn His Val Leu Pro Tyr Ser Gln Leu Phe Gly Pro Asn Ala Leu Gly
   885 890 895
   Thr Ala Glu Leu Ile Arg Leu Ala Leu Thr Thr Arg Ile Lys Pro Phe
   900 905 910
   Thr Tyr Val Ser Thr Ile Gly Val Gly Ala Gly Ile Glu Pro Gly Arg
   915 920 925
   Phe Thr Glu Asp Asp Asp Ile Arg Val Ile Ser Pro Thr Arg Ala Val
   930 935 940
   Asp Thr Gly Tyr Ala Asn Gly Tyr Gly Asn Ser Lys Trp Ala Gly Glu
   945 950 955 960
   Val Leu Leu Arg Glu Ala His Asp Leu Cys Gly Leu Pro Val Ala Val
   965 970 975
   Phe Arg Cys Asp Met Ile Leu Ala Asp Thr Thr Tyr Ala Gly Gln Leu
   980 985 990
   Asn Leu Pro Asp Met Phe Thr Arg Met Met Val Ser Leu Val Thr Thr
   995 1000 1005
   Gly Ile Ala Pro Lys Ser Phe His Pro Leu Asp Ala Lys Gly His
   1010 1015 1020
   Arg Gln Arg Ala His Tyr Asp Gly Leu Pro Val Glu Phe Val Ala
   1025 1030 1035
   Glu Ser Ile Ser Ala Leu Gly Ala Gln Ala Val Asp Glu Ala Gly
   1040 1045 1050
   Thr Gly Phe Ala Thr Tyr His Val Met Asn Pro His Asp Asp Gly
   1055 1060 1065
   Ile Gly Leu Asp Glu Phe Val Asp Trp Leu Val Glu Ala Gly Tyr
   1070 1075 1080
   Arg Ile Asp Arg Ile Asp Asp Tyr Ala Ala Trp Leu Gln Arg Phe
   1085 1090 1095
   Glu Thr Ala Leu Arg Ala Leu Pro Glu Arg Thr Arg Gln Tyr Ser
   1100 1105 1110
   Leu Leu Pro Leu Leu His Asn Tyr Gln Arg Pro Ala His Pro Ile
   1115 1120 1125
   Asn Gly Ala Met Ala Pro Thr Asp Arg Phe Arg Ala Ala Val Gln
   1130 1135 1140
   Glu Ala Lys Leu Gly Pro Asp Lys Asp Ile Pro His Val Thr Pro
   1145 1150 1155
   Gly Val Ile Val Lys Tyr Ala Thr Asp Leu Glu Leu Leu Gly Leu
   1160 1165 1170
   Ile
<210> 5
   <211> 3517
   <212> DNA
   <213> Artificial
<220>
   <223> Synthetic DNA sequence including optimized codon sequence coding for Streptomyces griseus CAR peptide.
<400> 5
   acaatctaga ggccagcctg gccataagga gatatacata tggctgaacc ccttgatgcc 60
   gcaaccgcct ccgcacacga ccctggacaa ggtttggcag aagcccttgc cgccgtggaa 120
   cctggtcgtg cccttgctga agttatggct tccgttttgg aaggtcacgg tgatcgtccg 180
   gctttgggcg agcgtgctcg tgaacccgaa actggacgtt tgttgcctca ttttgatacg 240
   atctcctatc gtgagctttg gtctcgtgtg cgtgctttgg ccggtcgttg gcatcatgac 300
   cctgagtacc ctctgggtcc cggagaccgt atctgcaccc tgggctttac cagcacagat 360
   tatgccaccc tggatcttgc ttgcatccac ctgggtgctg ttccagttcc attgccatcc 420
   aacgctccat tgccccgttt ggcgccggtg gttgaggagt ccggcccaac cgttcttgct 480
   gcatccgttg atcgtttgga tactgcgatt gatgttgtcc tggccagctc taccatccgt 540
   cgtctgttgg tcttcgatga tggacctggt gccacccgtc caggaggtgc ccttgccgca 600
   gcccgtcaac gtctgtccgg ttccccggtc accgtggaca ctctggccgg tcttatcgac 660
   cgtggccgtg accttccccc cccacccctt tatattcctg atcctggcga ggaccctctg 720
   gctctgctga tttacacgtc cggatctaca ggcgcaccaa aaggcgcaat gtacactcaa 780
   cgtctgctgg gtacagcatg gtacggtttc agctacggcg ccgccgatac ccctgccatt 840
   tccgttctgt atctgccaca gtcccacctg gctggtcgtt acgctgtcat gggtagcttg 900
   gtgaaaggtg gtactggata ctttacggca gcggatgact tgtccaccct tttcgaagac 960
   attgcgcttg tccgtcccac cgaattgacg atggttcctc gtctgtgtga catgcttctg 1020
   caacactatc gttctgagcg tgatcgtcgt gcggacgagc ccggtgatat tgaagctgcc 1080
   gtcactaaag ccgttcgtga ggacttcctg ggtggacgtg tggcgaaggc tttcgttggt 1140
   actgcccctc tttccgccga gttgacggct ttcgtcgaat ccgtgttggg ttttcacctg 1200
   tacactggct atggaagcac ggaagccggt ggtgtcttgt tggatactgt tgttcagcgt 1260
   cctcccgtca cagactacaa actggtcgat gtgcctgagc tgggatatta tgctaccgat 1320
   ctgccgcatc ctcgtggaga gttgcttttg aaatcccaca ccttgattcc tggttattac 1380
   cgtcgtcccg acctgaccgc cgccatcttt gacgccgacg gttactaccg taccggcgat 1440
   gtttttgctg aaaccggtcc ggatcgtctt gtctatgttg accgtactaa agacacgttg 1500
   aagctgtctc agggtgagtt cgttgccgtg tcccgtttgg aaacagtctt gttggactct 1560
   cctcttgttc aacacttgta cctttatggc aactctgagc gtgcatattt gcttgcggtc 1620
   gtcgttccta cgccagatgc gttggctggt tgtggaggcg acacggaagc cctgcgtccg 1680
   ttgctgatgg agtccctgcg ttctgtcgca cgtcgtgccg gtttgaacgc ttacgaaatc 1740
   cctcgtggta tcttggtcga accggaacct tttagcccgg agaacggtct gttcaccgag 1800
   tctcataagt tgctgcgtcc acgtcttaaa gaacgttatg gtcctgcttt ggagttgctg 1860
   tacgatcgtc ttgccgacgg tcaggatcgt cgtttgcgtg agcttcgtcg tactggtgcc 1920
   gaccgtcctg tccaggagac cgtgctgcgt gccgctcaag ccttgttggg ctccccaggc 1980
   tctgacttgc gtcccggcgc tcactttacg gatcttggtg gcgactcttt gtccgcagtc 2040
   agcttttccg agttgatgaa ggaaattttt catgtcgatg ttcctgttgg cgccattatt 2100
   ggtccagccg ctgacctggc cgaagttgcg cgttacatca ctgctgctcg tcgtcctgcg 2160
   ggagcgcccc gtccaacgcc agcctccgtt catggcgaac atcgtactga ggtccgtgcc 2220
   ggtgatctgg ccccagagaa gtttttggat gcgccgactc tggcagcagc ccccgctttg 2280
   ccacgtccag acggtgacgt tcgtactgtg ttgctgaccg gtgccactgg ctacttgggt 2340
   cgttttctgt gtttggaatg gctggagcgt ttggcgccaa gcggtggtcg tcttgtttgt 2400
   ttggttcgtg gtagcgacgc aacggtcgcg gcccgtcgtc tggaggcggc gtttgactct 2460
   ggcgacacgg cacttttgcg tcgttatcgt aaggccgcag gaaaaacgtt ggatgttgtc 2520
   gccggtgaca ttggcgagcc tttgctgggt ctggccgagg agacctggcg tgagcttgct 2580
   ggtgccgtcg atttgattgt gcaccctgcc gctcttgtca accacttgtt gccctacggt 2640
   gagctgttcg gtccaaatgt cgtgggcacc gctgaggcga tccgtctggc cctgaccacc 2700
   cgtttgaagc ctgttaatca cgtttccacc gtggcggtgt gcctgggtac gcccgccgag 2760
   accgccgacg aaaacgctga tattcgtgcc gctgttccgg tgcgtacaac aggtcaaggc 2820
   tatgccgacg gttacgcgac ctctaaatgg gctggcgagg tccttcttcg tgaggcacat 2880
   gaacgttacg gtttgccagt ggctgtcttt cgttctgaca tggttttggc acaccgtact 2940
   tacactggcc aagttaacgt cccagatgtt ttgacacgtt tgttgcttag ccttgtggcg 3000
   actggcatcg ccccaggttc tttttaccgt accgatacac gtgcccacta tgacggcctg 3060
   ccagtggact ttaccgccga ggctgttgtg gcactgggag cccccattac tgagggtcac 3120
   cgtacgttca acgttctgaa cccccacgat gacggtgtga gccttgatac ttttgtggat 3180
   tggttgatcg aggcaggtca tcctattcgt cgtatcgacg atcatggtgc ttggttgact 3240
   cgtttcaccg ccgccttgcg tgcgctgcct gagaagcaac gtcaacactc cttgttgccg 3300
   cttatcggtg cctgggcgga gcccggcgaa ggtgcccccg gtccccttct gccagcacgt 3360
   cgttttcatg cagcggtccg tgcagccggt gtgggtcctg aacgtgatat tccgcgtgtc 3420
   tcccctgatt tgattcgtaa gtacgtgacc gatttgcgtg ccctgggttt gttggcaggt 3480
   ccgtaatgag gccaaactgg ccaccatcac catcacc 3517
<210> 6
   <211> 1148
   <212> PRT
   <213> Artificial
<220>
   <223> Synthetic peptide sequence from optimized codon sequence coding for Streptomyces griseus CAR peptide.
<400> 6
   Met Ala Glu Pro Leu Asp Ala Ala Thr Ala Ser Ala His Asp Pro Gly
   1               5                   10                  15
   Gln Gly Leu Ala Glu Ala Leu Ala Ala Val Glu Pro Gly Arg Ala Leu
               20                  25                  30
   Ala Glu Val Met Ala Ser Val Leu Glu Gly His Gly Asp Arg Pro Ala
           35                  40                  45
   Leu Gly Glu Arg Ala Arg Glu Pro Glu Thr Gly Arg Leu Leu Pro His
       50                  55                  60
   Phe Asp Thr Ile Ser Tyr Arg Glu Leu Trp Ser Arg Val Arg Ala Leu
   65                  70                  75                  80
   Ala Gly Arg Trp His His Asp Pro Glu Tyr Pro Leu Gly Pro Gly Asp
                   85                  90                  95
   Arg Ile Cys Thr Leu Gly Phe Thr Ser Thr Asp Tyr Ala Thr Leu Asp
               100                 105                 110
   Leu Ala Cys Ile His Leu Gly Ala Val Pro Val Pro Leu Pro Ser Asn
           115                 120                 125
   Ala Pro Leu Pro Arg Leu Ala Pro Val Val Glu Glu Ser Gly Pro Thr
       130                 135                 140
   Val Leu Ala Ala Ser Val Asp Arg Leu Asp Thr Ala Ile Asp Val Val
   145                 150                 155                 160
   Leu Ala Ser Ser Thr Ile Arg Arg Leu Leu Val Phe Asp Asp Gly Pro
                   165                 170                 175
   Gly Ala Thr Arg Pro Gly Gly Ala Leu Ala Ala Ala Arg Gln Arg Leu
               180                 185                 190
   Ser Gly Ser Pro Val Thr Val Asp Thr Leu Ala Gly Leu Ile Asp Arg
           195                 200                 205
   Gly Arg Asp Leu Pro Pro Pro Pro Leu Tyr Ile Pro Asp Pro Gly Glu
       210                 215                 220
   Asp Pro Leu Ala Leu Leu Ile Tyr Thr Ser Gly Ser Thr Gly Ala Pro
   225                 230                 235                 240
   Lys Gly Ala Met Tyr Thr Gln Arg Leu Leu Gly Thr Ala Trp Tyr Gly
                   245                 250                 255
   Phe Ser Tyr Gly Ala Ala Asp Thr Pro Ala Ile Ser Val Leu Tyr Leu
   260 265 270
   Pro Gln Ser His Leu Ala Gly Arg Tyr Ala Val Met Gly Ser Leu Val
   275 280 285
   Lys Gly Gly Thr Gly Tyr Phe Thr Ala Ala Asp Asp Leu Ser Thr Leu
   290 295 300
   Phe Glu Asp Ile Ala Leu Val Arg Pro Thr Glu Leu Thr Met Val Pro
   305 310 315 320
   Arg Leu Cys Asp Met Leu Leu Gln His Tyr Arg Ser Glu Arg Asp Arg
   325 330 335
   Arg Ala Asp Glu Pro Gly Asp Ile Glu Ala Ala Val Thr Lys Ala Val
   340 345 350
   Arg Glu Asp Phe Leu Gly Gly Arg Val Ala Lys Ala Phe Val Gly Thr
   355 360 365
   Ala Pro Leu Ser Ala Glu Leu Thr Ala Phe Val Glu Ser Val Leu Gly
   370 375 380
   Phe His Leu Tyr Thr Gly Tyr Gly Ser Thr Glu Ala Gly Gly Val Leu
   385 390 395 400
   Leu Asp Thr Val Val Gln Arg Pro Pro Val Thr Asp Tyr Lys Leu Val
   405 410 415
   Asp Val Pro Glu Leu Gly Tyr Tyr Ala Thr Asp Leu Pro His Pro Arg
   420 425 430
   Gly Glu Leu Leu Leu Lys Ser His Thr Leu Ile Pro Gly Tyr Tyr Arg
   435 440 445
   Arg Pro Asp Leu Thr Ala Ala Ile Phe Asp Ala Asp Gly Tyr Tyr Arg
   450 455 460
   Thr Gly Asp Val Phe Ala Glu Thr Gly Pro Asp Arg Leu Val Tyr Val
   465 470 475 480
   Asp Arg Thr Lys Asp Thr Leu Lys Leu Ser Gln Gly Glu Phe Val Ala
   485 490 495
   Val Ser Arg Leu Glu Thr Val Leu Leu Asp Ser Pro Leu Val Gln His
   500 505 510
   Leu Tyr Leu Tyr Gly Asn Ser Glu Arg Ala Tyr Leu Leu Ala Val Val
   515 520 525
   Val Pro Thr Pro Asp Ala Leu Ala Gly Cys Gly Gly Asp Thr Glu Ala
   530 535 540
   Leu Arg Pro Leu Leu Met Glu Ser Leu Arg Ser Val Ala Arg Arg Ala
   545 550 555 560
   Gly Leu Asn Ala Tyr Glu Ile Pro Arg Gly Ile Leu Val Glu Pro Glu
   565 570 575
   Pro Phe Ser Pro Glu Asn Gly Leu Phe Thr Glu Ser His Lys Leu Leu
   580 585 590
   Arg Pro Arg Leu Lys Glu Arg Tyr Gly Pro Ala Leu Glu Leu Leu Tyr
   595 600 605
   Asp Arg Leu Ala Asp Gly Gln Asp Arg Arg Leu Arg Glu Leu Arg Arg
   610 615 620
   Thr Gly Ala Asp Arg Pro Val Gln Glu Thr Val Leu Arg Ala Ala Gln
   625 630 635 640
   Ala Leu Leu Gly Ser Pro Gly Ser Asp Leu Arg Pro Gly Ala His Phe
   645 650 655
   Thr Asp Leu Gly Gly Asp Ser Leu Ser Ala Val Ser Phe Ser Glu Leu
   660 665 670
   Met Lys Glu Ile Phe His Val Asp Val Pro Val Gly Ala Ile Ile Gly
   675 680 685
   Pro Ala Ala Asp Leu Ala Glu Val Ala Arg Tyr Ile Thr Ala Ala Arg
   690 695 700
   Arg Pro Ala Gly Ala Pro Arg Pro Thr Pro Ala Ser Val His Gly Glu
   705 710 715 720
   His Arg Thr Glu Val Arg Ala Gly Asp Leu Ala Pro Glu Lys Phe Leu
   725 730 735
   Asp Ala Pro Thr Leu Ala Ala Ala Pro Ala Leu Pro Arg Pro Asp Gly
   740 745 750
   Asp Val Arg Thr Val Leu Leu Thr Gly Ala Thr Gly Tyr Leu Gly Arg
   755 760 765
   Phe Leu Cys Leu Glu Trp Leu Glu Arg Leu Ala Pro Ser Gly Gly Arg
   770 775 780
   Leu Val Cys Leu Val Arg Gly Ser Asp Ala Thr Val Ala Ala Arg Arg
   785 790 795 800
   Leu Glu Ala Ala Phe Asp Ser Gly Asp Thr Ala Leu Leu Arg Arg Tyr
   805 810 815
   Arg Lys Ala Ala Gly Lys Thr Leu Asp Val Val Ala Gly Asp Ile Gly
   820 825 830
   Glu Pro Leu Leu Gly Leu Ala Glu Glu Thr Trp Arg Glu Leu Ala Gly
   835 840 845
   Ala Val Asp Leu Ile Val His Pro Ala Ala Leu Val Asn His Leu Leu
   850 855 860
   Pro Tyr Gly Glu Leu Phe Gly Pro Asn Val Val Gly Thr Ala Glu Ala
   865 870 875 880
   Ile Arg Leu Ala Leu Thr Thr Arg Leu Lys Pro Val Asn His Val Ser
   885 890 895
   Thr Val Ala Val Cys Leu Gly Thr Pro Ala Glu Thr Ala Asp Glu Asn
   900 905 910
   Ala Asp Ile Arg Ala Ala Val Pro Val Arg Thr Thr Gly Gln Gly Tyr
   915 920 925
   Ala Asp Gly Tyr Ala Thr Ser Lys Trp Ala Gly Glu Val Leu Leu Arg
   930 935 940
   Glu Ala His Glu Arg Tyr Gly Leu Pro Val Ala Val Phe Arg Ser Asp
   945 950 955 960
   Met Val Leu Ala His Arg Thr Tyr Thr Gly Gln Val Asn Val Pro Asp
   965 970 975
   Val Leu Thr Arg Leu Leu Leu Ser Leu Val Ala Thr Gly Ile Ala Pro
   980 985 990
   Gly Ser Phe Tyr Arg Thr Asp Thr Arg Ala His Tyr Asp Gly Leu Pro
   995 1000 1005
   Val Asp Phe Thr Ala Glu Ala Val Val Ala Leu Gly Ala Pro Ile
   1010 1015 1020
   Thr Glu Gly His Arg Thr Phe Asn Val Leu Asn Pro His Asp Asp
   1025 1030 1035
   Gly Val  Ser Leu Asp Thr Phe  Val Asp Trp Leu Ile  Glu Ala Gly
       1040                 1045                 1050
   His Pro  Ile Arg Arg Ile Asp  Asp His Gly Ala Trp  Leu Thr Arg
       1055                 1060                 1065
   Phe Thr  Ala Ala Leu Arg Ala  Leu Pro Glu Lys Gln  Arg Gln His
       1070                 1075                 1080
   Ser Leu  Leu Pro Leu Ile Gly  Ala Trp Ala Glu Pro  Gly Glu Gly
       1085                 1090                 1095
   Ala Pro  Gly Pro Leu Leu Pro  Ala Arg Arg Phe His  Ala Ala Val
       1100                 1105                 1110
   Arg Ala  Ala Gly Val Gly Pro  Glu Arg Asp Ile Pro  Arg Val Ser
       1115                 1120                 1125
   Pro Asp  Leu Ile Arg Lys Tyr  Val Thr Asp Leu Arg  Ala Leu Gly
       1130                 1135                 1140
   Leu Leu  Ala Gly Pro
       1145
<210> 7
   <211> 739
   <212> DNA
   <213> Artificial
<220>
   <223> Synthetic DNA sequence including optimized codon sequence coding for Nocardia NRRL5646 PPTase peptide.
<400> 7
   acaatctaga ggccagcctg gccataagga gatatacata tgatcgaaac tattttgccc     60
   gcaggtgttg aatctgccga gcttttggaa tatcctgagg accttaaagc ccaccctgct    120
   gaagaacatc ttattgccaa atccgtcgaa aaacgtcgtc gtgatttcat tggtgcccgt    180
   cattgcgcgc gtctggccct ggccgagttg ggcgagccac ccgtcgcaat tggcaaaggt    240
   gaacgtggtg cccctatttg gccgcgtggc gttgtcggct ctcttaccca ctgcgacggc    300
   taccgtgccg ccgcagtcgc ccataagatg cgtttccgtt ctattggcat tgacgccgaa    360
   ccgcacgcca cccttcctga aggagtcctg gactctgttt ctcttccacc tgaacgtgag    420
   tggttgaaga ccactgattc tgctttgcat cttgatcgtt tgttgttctg cgcgaaggaa    480
   gcaacttata aggcttggtg gccattgacc gctcgttggc ttggctttga ggaagcacat    540
   attacttttg agattgagga tggtagcgcc gatagcggca atggtacttt tcatagcgaa    600
   ctgttggttc ctggtcagac gaatgacggt ggtactcctc ttcttagctt tgatggacgt    660
   tggctgattg ccgatggttt tatcttgacc gcaattgcgt atgcgtaatg aggccaaact    720
   ggccaccatc accatcacc                                                 739
<210> 8
   <211> 222
   <212> PRT
   <213> Artificial
<220>
   <223> Synthetic peptide sequence from optimized codon sequence coding for Nocardia NRRL5646 PPTase peptide.
<400> 8
   Met Ile Glu Thr Ile Leu Pro Ala Gly Val Glu Ser Ala Glu Leu Leu
   1               5                   10                  15
   Glu Tyr Pro Glu Asp Leu Lys Ala His Pro Ala Glu Glu His Leu Ile
               20                  25                  30
   Ala Lys Ser Val Glu Lys Arg Arg Arg Asp Phe Ile Gly Ala Arg His
           35                  40                  45
   Cys Ala Arg Leu Ala Leu Ala Glu Leu Gly Glu Pro Pro Val Ala Ile
       50                  55                  60
   Gly Lys Gly Glu Arg Gly Ala Pro Ile Trp Pro Arg Gly Val Val Gly
   65                  70                  75                  80
   Ser Leu Thr His Cys Asp Gly Tyr Arg Ala Ala Ala Val Ala His Lys
                   85                  90                  95
   Met Arg Phe Arg Ser Ile Gly Ile Asp Ala Glu Pro His Ala Thr Leu
               100                 105                 110
   Pro Glu Gly Val Leu Asp Ser Val Ser Leu Pro Pro Glu Arg Glu Trp
           115                 120                 125
   Leu Lys Thr Thr Asp Ser Ala Leu His Leu Asp Arg Leu Leu Phe Cys
       130                 135                 140
   Ala Lys Glu Ala Thr Tyr Lys Ala Trp Trp Pro Leu Thr Ala Arg Trp
   145                 150                 155                 160
   Leu Gly Phe Glu Glu Ala His Ile Thr Phe Glu Ile Glu Asp Gly Ser
                   165                 170                 175
   Ala Asp Ser Gly Asn Gly Thr Phe His Ser Glu Leu Leu Val Pro Gly
               180                 185                 190
   Gln Thr Asn Asp Gly Gly Thr Pro Leu Leu Ser Phe Asp Gly Arg Trp
               195                 200                 205
   Leu Ile Ala Asp Gly Phe Ile Leu Thr Ala Ile Ala Tyr Ala
       210                 215                 220
<210> 9
   <211> 1178
   <212> PRT
   <213> Artificial
<220>
   <223> Synthetic peptide having GDIH at N-terminal of CAR peptide from Nocardia NRRL5646.
<400> 9
   Gly Asp Ile His Met Ala Val Asp Ser Pro Asp Glu Arg Leu Gln Arg
   1               5                   10                  15
   Arg Ile Ala Gln Leu Phe Ala Glu Asp Glu Gln Val Lys Ala Ala Arg
               20                  25                  30
   Pro Leu Glu Ala Val Ser Ala Ala Val Ser Ala Pro Gly Met Arg Leu
           35                  40                  45
   Ala Gln Ile Ala Ala Thr Val Met Ala Gly Tyr Ala Asp Arg Pro Ala
       50                  55                  60
   Ala Gly Gln Arg Ala Phe Glu Leu Asn Thr Asp Asp Ala Thr Gly Arg
   65                  70                  75                  80
   Thr Ser Leu Arg Leu Leu Pro Arg Phe Glu Thr Ile Thr Tyr Arg Glu
                   85                  90                  95
   Leu Trp Gln Arg Val Gly Glu Val Ala Ala Ala Trp His His Asp Pro
               100                 105                 110
   Glu Asn Pro Leu Arg Ala Gly Asp Phe Val Ala Leu Leu Gly Phe Thr
           115                 120                 125
   Ser Ile Asp Tyr Ala Thr Leu Asp Leu Ala Asp Ile His Leu Gly Ala
       130                 135                 140
   Val Thr Val Pro Leu Gln Ala Ser Ala Ala Val Ser Gln Leu Ile Ala
   145                 150                 155                 160
   Ile Leu Thr Glu Thr Ser Pro Arg Leu Leu Ala Ser Thr Pro Glu His
                   165                 170                 175
   Leu Asp Ala Ala Val Glu Cys Leu Leu Ala Gly Thr Thr Pro Glu Arg
               180                 185                 190
   Leu Val Val Phe Asp Tyr His Pro Glu Asp Asp Asp Gln Arg Ala Ala
           195                 200                 205
   Phe Glu Ser Ala Arg Arg Arg Leu Ala Asp Ala Gly Ser Leu Val Ile
       210                 215                 220
   Val Glu Thr Leu Asp Ala Val Arg Ala Arg Gly Arg Asp Leu Pro Ala
   225                 230                 235                 240
   Ala Pro Leu Phe Val Pro Asp Thr Asp Asp Asp Pro Leu Ala Leu Leu
                   245                 250                 255
   Ile Tyr Thr Ser Gly Ser Thr Gly Thr Pro Lys Gly Ala Met Tyr Thr
               260                 265                 270
   Asn Arg Leu Ala Ala Thr Met Trp Gln Gly Asn Ser Met Leu Gln Gly
           275                 280                 285
   Asn Ser Gln Arg Val Gly Ile Asn Leu Asn Tyr Met Pro Met Ser His
       290                 295                 300
   Ile Ala Gly Arg Ile Ser Leu Phe Gly Val Leu Ala Arg Gly Gly Thr
   305                 310                 315                 320
   Ala Tyr Phe Ala Ala Lys Ser Asp Met Ser Thr Leu Phe Glu Asp Ile
                   325                 330                 335
   Gly Leu Val Arg Pro Thr Glu Ile Phe Phe Val Pro Arg Val Cys Asp
               340                 345                 350
   Met Val Phe Gln Arg Tyr Gln Ser Glu Leu Asp Arg Arg Ser Val Ala
           355                 360                 365
   Gly Ala Asp Leu Asp Thr Leu Asp Arg Glu Val Lys Ala Asp Leu Arg
       370                 375                 380
   Gln Asn Tyr Leu Gly Gly Arg Phe Leu Val Ala Val Val Gly Ser Ala
   385                 390                 395                 400
   Pro Leu Ala Ala Glu Met Lys Thr Phe Met Glu Ser Val Leu Asp Leu
                   405                 410                 415
   Pro Leu His Asp Gly Tyr Gly Ser Thr Glu Ala Gly Ala Ser Val Leu
               420                 425                 430
   Leu Asp Asn Gln Ile Gln Arg Pro Pro Val Leu Asp Tyr Lys Leu Val
           435                 440                 445
   Asp Val Pro Glu Leu Gly Tyr Phe Arg Thr Asp Arg Pro His Pro Arg
   450 455 460
   Gly Glu Leu Leu Leu Lys Ala Glu Thr Thr Ile Pro Gly Tyr Tyr Lys
   465 470 475 480
   Arg Pro Glu Val Thr Ala Glu Ile Phe Asp Glu Asp Gly Phe Tyr Lys
   485 490 495
   Thr Gly Asp Ile Val Ala Glu Leu Glu His Asp Arg Leu Val Tyr Val
   500 505 510
   Asp Arg Arg Asn Asn Val Leu Lys Leu Ser Gln Gly Glu Phe Val Thr
   515 520 525
   Val Ala His Leu Glu Ala Val Phe Ala Ser Ser Pro Leu Ile Arg Gln
   530 535 540
   Ile Phe Ile Tyr Gly Ser Ser Glu Arg Ser Tyr Leu Leu Ala Val Ile
   545 550 555 560
   Val Pro Thr Asp Asp Ala Leu Arg Gly Arg Asp Thr Ala Thr Leu Lys
   565 570 575
   Ser Ala Leu Ala Glu Ser Ile Gln Arg Ile Ala Lys Asp Ala Asn Leu
   580 585 590
   Gln Pro Tyr Glu Ile Pro Arg Asp Phe Leu Ile Glu Thr Glu Pro Phe
   595 600 605
   Thr Ile Ala Asn Gly Leu Leu Ser Gly Ile Ala Lys Leu Leu Arg Pro
   610 615 620
   Asn Leu Lys Glu Arg Tyr Gly Ala Gln Leu Glu Gln Met Tyr Thr Asp
   625 630 635 640
   Leu Ala Thr Gly Gln Ala Asp Glu Leu Leu Ala Leu Arg Arg Glu Ala
   645 650 655
   Ala Asp Leu Pro Val Leu Glu Thr Val Ser Arg Ala Ala Lys Ala Met
   660 665 670
   Leu Gly Val Ala Ser Ala Asp Met Arg Pro Asp Ala His Phe Thr Asp
   675 680 685
   Leu Gly Gly Asp Ser Leu Ser Ala Leu Ser Phe Ser Asn Leu Leu His
   690 695 700
   Glu Ile Phe Gly Val Glu Val Pro Val Gly Val Val Val Ser Pro Ala
   705 710 715 720
   Asn Glu Leu Arg Asp Leu Ala Asn Tyr Ile Glu Ala Glu Arg Asn Ser
   725 730 735
   Gly Ala Lys Arg Pro Thr Phe Thr Ser Val His Gly Gly Gly Ser Glu
   740 745 750
   Ile Arg Ala Ala Asp Leu Thr Leu Asp Lys Phe Ile Asp Ala Arg Thr
   755 760 765
   Leu Ala Ala Ala Asp Ser Ile Pro His Ala Pro Val Pro Ala Gln Thr
   770 775 780
   Val Leu Leu Thr Gly Ala Asn Gly Tyr Leu Gly Arg Phe Leu Cys Leu
   785 790 795 800
   Glu Trp Leu Glu Arg Leu Asp Lys Thr Gly Gly Thr Leu Ile Cys Val
   805 810 815
   Val Arg Gly Ser Asp Ala Ala Ala Ala Arg Lys Arg Leu Asp Ser Ala
   820 825 830
   Phe Asp Ser Gly Asp Pro Gly Leu Leu Glu His Tyr Gln Gln Leu Ala
   835 840 845
   Ala Arg Thr Leu Glu Val Leu Ala Gly Asp Ile Gly Asp Pro Asn Leu
   850 855 860
   Gly Leu Asp Asp Ala Thr Trp Gln Arg Leu Ala Glu Thr Val Asp Leu
   865 870 875 880
   Ile Val His Pro Ala Ala Leu Val Asn His Val Leu Pro Tyr Thr Gln
   885 890 895
   Leu Phe Gly Pro Asn Val Val Gly Thr Ala Glu Ile Val Arg Leu Ala
   900 905 910
   Ile Thr Ala Arg Arg Lys Pro Val Thr Tyr Leu Ser Thr Val Gly Val
   915 920 925
   Ala Asp Gln Val Asp Pro Ala Glu Tyr Gln Glu Asp Ser Asp Val Arg
   930 935 940
   Glu Met Ser Ala Val Arg Val Val Arg Glu Ser Tyr Ala Asn Gly Tyr
   945 950 955 960
   Gly Asn Ser Lys Trp Ala Gly Glu Val Leu Leu Arg Glu Ala His Asp
   965 970 975
   Leu Cys Gly Leu Pro Val Ala Val Phe Arg Ser Asp Met Ile Leu Ala
   980 985 990
   His Ser Arg Tyr Ala Gly Gln Leu Asn Val Gln Asp Val Phe Thr Arg
   995 1000 1005
   Leu Ile Leu Ser Leu Val Ala Thr Gly Ile Ala Pro Tyr Ser Phe
   1010 1015 1020
   Tyr Arg Thr Asp Ala Asp Gly Asn Arg Gln Arg Ala His Tyr Asp
   1025 1030 1035
   Gly Leu Pro Ala Asp Phe Thr Ala Ala Ala Ile Thr Ala Leu Gly
   1040 1045 1050
   Ile Gln Ala Thr Glu Gly Phe Arg Thr Tyr Asp Val Leu Asn Pro
   1055 1060 1065
   Tyr Asp Asp Gly Ile Ser Leu Asp Glu Phe Val Asp Trp Leu Val
   1070 1075 1080
   Glu Ser Gly His Pro Ile Gln Arg Ile Thr Asp Tyr Ser Asp Trp
   1085 1090 1095
   Phe His Arg Phe Glu Thr Ala Ile Arg Ala Leu Pro Glu Lys Gln
   1100 1105 1110
   Arg Gln Ala Ser Val Leu Pro Leu Leu Asp Ala Tyr Arg Asn Pro
   1115 1120 1125
   Cys Pro Ala Val Arg Gly Ala Ile Leu Pro Ala Lys Glu Phe Gln
   1130 1135 1140
   Ala Ala Val Gln Thr Ala Lys Ile Gly Pro Glu Gln Asp Ile Pro
   1145 1150 1155
   His Leu Ser Ala Pro Leu Ile Asp Lys Tyr Val Ser Asp Leu Glu
   1160 1165 1170
   Leu Leu Gln Leu Leu
   1175
<210> 10
   <211> 1152
   <212> PRT
   <213> Artificial
<220>
   <223> Synthetic peptide having GDIH at N-terminal of CAR peptide from Streptomyces griseus.
<400> 10
   Gly Asp Ile His Met Ala Glu Pro Leu Asp Ala Ala Thr Ala Ser Ala
   1               5                   10                  15
   His Asp Pro Gly Gln Gly Leu Ala Glu Ala Leu Ala Ala Val Glu Pro
               20                  25                  30
   Gly Arg Ala Leu Ala Glu Val Met Ala Ser Val Leu Glu Gly His Gly
           35                  40                  45
   Asp Arg Pro Ala Leu Gly Glu Arg Ala Arg Glu Pro Glu Thr Gly Arg
       50                  55                  60
   Leu Leu Pro His Phe Asp Thr Ile Ser Tyr Arg Glu Leu Trp Ser Arg
   65                  70                  75                  80
   Val Arg Ala Leu Ala Gly Arg Trp His His Asp Pro Glu Tyr Pro Leu
                   85                  90                  95
   Gly Pro Gly Asp Arg Ile Cys Thr Leu Gly Phe Thr Ser Thr Asp Tyr
               100                 105                 110
   Ala Thr Leu Asp Leu Ala Cys Ile His Leu Gly Ala Val Pro Val Pro
           115                 120                 125
   Leu Pro Ser Asn Ala Pro Leu Pro Arg Leu Ala Pro Val Val Glu Glu
       130                 135                 140
   Ser Gly Pro Thr Val Leu Ala Ala Ser Val Asp Arg Leu Asp Thr Ala
   145                 150                 155                 160
   Ile Asp Val Val Leu Ala Ser Ser Thr Ile Arg Arg Leu Leu Val Phe
                   165                 170                 175
   Asp Asp Gly Pro Gly Ala Thr Arg Pro Gly Gly Ala Leu Ala Ala Ala
               180                 185                 190
   Arg Gln Arg Leu Ser Gly Ser Pro Val Thr Val Asp Thr Leu Ala Gly
           195                 200                 205
   Leu Ile Asp Arg Gly Arg Asp Leu Pro Pro Pro Pro Leu Tyr Ile Pro
       210                 215                 220
   Asp Pro Gly Glu Asp Pro Leu Ala Leu Leu Ile Tyr Thr Ser Gly Ser
   225                 230                 235                 240
   Thr Gly Ala Pro Lys Gly Ala Met Tyr Thr Gln Arg Leu Leu Gly Thr
   245 250 255
   Ala Trp Tyr Gly Phe Ser Tyr Gly Ala Ala Asp Thr Pro Ala Ile Ser
   260 265 270
   Val Leu Tyr Leu Pro Gln Ser His Leu Ala Gly Arg Tyr Ala Val Met
   275 280 285
   Gly Ser Leu Val Lys Gly Gly Thr Gly Tyr Phe Thr Ala Ala Asp Asp
   290 295 300
   Leu Ser Thr Leu Phe Glu Asp Ile Ala Leu Val Arg Pro Thr Glu Leu
   305 310 315 320
   Thr Met Val Pro Arg Leu Cys Asp Met Leu Leu Gln His Tyr Arg Ser
   325 330 335
   Glu Arg Asp Arg Arg Ala Asp Glu Pro Gly Asp Ile Glu Ala Ala Val
   340 345 350
   Thr Lys Ala Val Arg Glu Asp Phe Leu Gly Gly Arg Val Ala Lys Ala
   355 360 365
   Phe Val Gly Thr Ala Pro Leu Ser Ala Glu Leu Thr Ala Phe Val Glu
   370 375 380
   Ser Val Leu Gly Phe His Leu Tyr Thr Gly Tyr Gly Ser Thr Glu Ala
   385 390 395 400
   Gly Gly Val Leu Leu Asp Thr Val Val Gln Arg Pro Pro Val Thr Asp
   405 410 415
   Tyr Lys Leu Val Asp Val Pro Glu Leu Gly Tyr Tyr Ala Thr Asp Leu
   420 425 430
   Pro His Pro Arg Gly Glu Leu Leu Leu Lys Ser His Thr Leu Ile Pro
   435 440 445
   Gly Tyr Tyr Arg Arg Pro Asp Leu Thr Ala Ala Ile Phe Asp Ala Asp
   450 455 460
   Gly Tyr Tyr Arg Thr Gly Asp Val Phe Ala Glu Thr Gly Pro Asp Arg
   465 470 475 480
   Leu Val Tyr Val Asp Arg Thr Lys Asp Thr Leu Lys Leu Ser Gln Gly
   485 490 495
   Glu Phe Val Ala Val Ser Arg Leu Glu Thr Val Leu Leu Asp Ser Pro
   500 505 510
   Leu Val Gln His Leu Tyr Leu Tyr Gly Asn Ser Glu Arg Ala Tyr Leu
   515 520 525
   Leu Ala Val Val Val Pro Thr Pro Asp Ala Leu Ala Gly Cys Gly Gly
   530 535 540
   Asp Thr Glu Ala Leu Arg Pro Leu Leu Met Glu Ser Leu Arg Ser Val
   545 550 555 560
   Ala Arg Arg Ala Gly Leu Asn Ala Tyr Glu Ile Pro Arg Gly Ile Leu
   565 570 575
   Val Glu Pro Glu Pro Phe Ser Pro Glu Asn Gly Leu Phe Thr Glu Ser
   580 585 590
   His Lys Leu Leu Arg Pro Arg Leu Lys Glu Arg Tyr Gly Pro Ala Leu
   595 600 605
   Glu Leu Leu Tyr Asp Arg Leu Ala Asp Gly Gln Asp Arg Arg Leu Arg
   610 615 620
   Glu Leu Arg Arg Thr Gly Ala Asp Arg Pro Val Gln Glu Thr Val Leu
   625 630 635 640
   Arg Ala Ala Gln Ala Leu Leu Gly Ser Pro Gly Ser Asp Leu Arg Pro
   645 650 655
   Gly Ala His Phe Thr Asp Leu Gly Gly Asp Ser Leu Ser Ala Val Ser
   660 665 670
   Phe Ser Glu Leu Met Lys Glu Ile Phe His Val Asp Val Pro Val Gly
   675 680 685
   Ala Ile Ile Gly Pro Ala Ala Asp Leu Ala Glu Val Ala Arg Tyr Ile
   690 695 700
   Thr Ala Ala Arg Arg Pro Ala Gly Ala Pro Arg Pro Thr Pro Ala Ser
   705 710 715 720
   Val His Gly Glu His Arg Thr Glu Val Arg Ala Gly Asp Leu Ala Pro
   725 730 735
   Glu Lys Phe Leu Asp Ala Pro Thr Leu Ala Ala Ala Pro Ala Leu Pro
   740 745 750
   Arg Pro Asp Gly Asp Val Arg Thr Val Leu Leu Thr Gly Ala Thr Gly
   755 760 765
   Tyr Leu Gly Arg Phe Leu Cys Leu Glu Trp Leu Glu Arg Leu Ala Pro
       770                 775                 780
   Ser Gly Gly Arg Leu Val Cys Leu Val Arg Gly Ser Asp Ala Thr Val
   785                 790                 795                 800
   Ala Ala Arg Arg Leu Glu Ala Ala Phe Asp Ser Gly Asp Thr Ala Leu
                   805                 810                 815
   Leu Arg Arg Tyr Arg Lys Ala Ala Gly Lys Thr Leu Asp Val Val Ala
               820                 825                 830
   Gly Asp Ile Gly Glu Pro Leu Leu Gly Leu Ala Glu Glu Thr Trp Arg
           835                 840                 845
   Glu Leu Ala Gly Ala Val Asp Leu Ile Val His Pro Ala Ala Leu Val
       850                 855                 860
   Asn His Leu Leu Pro Tyr Gly Glu Leu Phe Gly Pro Asn Val Val Gly
   865                 870                 875                 880
   Thr Ala Glu Ala Ile Arg Leu Ala Leu Thr Thr Arg Leu Lys Pro Val
                   885                 890                 895
   Asn His Val Ser Thr Val Ala Val Cys Leu Gly Thr Pro Ala Glu Thr
               900                 905                 910
   Ala Asp Glu Asn Ala Asp Ile Arg Ala Ala Val Pro Val Arg Thr Thr
           915                 920                 925
   Gly Gln Gly Tyr Ala Asp Gly Tyr Ala Thr Ser Lys Trp Ala Gly Glu
       930                 935                 940
   Val Leu Leu Arg Glu Ala His Glu Arg Tyr Gly Leu Pro Val Ala Val
   945                 950                 955                 960
   Phe Arg Ser Asp Met Val Leu Ala His Arg Thr Tyr Thr Gly Gln Val
                   965                 970                 975
   Asn Val Pro Asp Val Leu Thr Arg Leu Leu Leu Ser Leu Val Ala Thr
               980                 985                 990
   Gly Ile Ala Pro Gly Ser Phe Tyr  Arg Thr Asp Thr Arg  Ala His Tyr
           995                 1000                 1005
   Asp Gly  Leu Pro Val Asp Phe  Thr Ala Glu Ala Val  Val Ala Leu
       1010                 1015                 1020
   Gly Ala Pro Ile Thr Glu Gly His Arg Thr Phe Asn Val Leu Asn
   1025 1030 1035
   Pro His Asp Asp Gly Val Ser Leu Asp Thr Phe Val Asp Trp Leu
   1040 1045 1050
   Ile Glu Ala Gly His Pro Ile Arg Arg Ile Asp Asp His Gly Ala
   1055 1060 1065
   Trp Leu Thr Arg Phe Thr Ala Ala Leu Arg Ala Leu Pro Glu Lys
   1070 1075 1080
   Gln Arg Gln His Ser Leu Leu Pro Leu Ile Gly Ala Trp Ala Glu
   1085 1090 1095
   Pro Gly Glu Gly Ala Pro Gly Pro Leu Leu Pro Ala Arg Arg Phe
   1100 1105 1110
   His Ala Ala Val Arg Ala Ala Gly Val Gly Pro Glu Arg Asp Ile
   1115 1120 1125
   Pro Arg Val Ser Pro Asp Leu Ile Arg Lys Tyr Val Thr Asp Leu
   1130 1135 1140
   Arg Ala Leu Gly Leu Leu Ala Gly Pro
   1145 1150
<210> 11
   <211> 226
   <212> PRT
   <213> Artificial
<220>
   <223> Synthetic peptide having GDIH at N-terminal of PPTase peptide from Nocardia NRRL5646.
<400> 11
   Gly Asp Ile His Met Ile Glu Thr Ile Leu Pro Ala Gly Val Glu Ser
   1               5                   10                  15
   Ala Glu Leu Leu Glu Tyr Pro Glu Asp Leu Lys Ala His Pro Ala Glu
               20                  25                  30
   Glu His Leu Ile Ala Lys Ser Val Glu Lys Arg Arg Arg Asp Phe Ile
           35                  40                  45
   Gly Ala Arg His Cys Ala Arg Leu Ala Leu Ala Glu Leu Gly Glu Pro
       50                  55                  60
   Pro Val Ala Ile Gly Lys Gly Glu Arg Gly Ala Pro Ile Trp Pro Arg
   65                  70                  75                  80
   Gly Val Val Gly Ser Leu Thr His Cys Asp Gly Tyr Arg Ala Ala Ala
                   85                  90                  95
   Val Ala His Lys Met Arg Phe Arg Ser Ile Gly Ile Asp Ala Glu Pro
               100                 105                 110
   His Ala Thr Leu Pro Glu Gly Val Leu Asp Ser Val Ser Leu Pro Pro
           115                 120                 125
   Glu Arg Glu Trp Leu Lys Thr Thr Asp Ser Ala Leu His Leu Asp Arg
       130                 135                 140
   Leu Leu Phe Cys Ala Lys Glu Ala Thr Tyr Lys Ala Trp Trp Pro Leu
   145                 150                 155                 160
   Thr Ala Arg Trp Leu Gly Phe Glu Glu Ala His Ile Thr Phe Glu Ile
                   165                 170                 175
   Glu Asp Gly Ser Ala Asp Ser Gly Asn Gly Thr Phe His Ser Glu Leu
               180                 185                 190
   Leu Val Pro Gly Gln Thr Asn Asp Gly Gly Thr Pro Leu Leu Ser Phe
           195                 200                 205
   Asp Gly Arg Trp Leu Ile Ala Asp Gly Phe Ile Leu Thr Ala Ile Ala
       210                 215                 220
   Tyr Ala
   225